(19) Europäisches Patentamt
European Patent Office
Office européen des brevets

(11) Veröffentlichungsnummer: **0 548 261 B1**

# (12) EUROPÄISCHE PATENTSCHRIFT

(45) Veröffentlichungstag der Patentschrift: **10.05.95**

(51) Int. Cl.$^6$: **C07J 9/00**, C07J 51/00, A61K 31/56

(21) Anmeldenummer: **91917941.6**

(22) Anmeldetag: **25.10.91**

(86) Internationale Anmeldenummer:
**PCT/CH91/00221**

(87) Internationale Veröffentlichungsnummer:
**WO 92/12989 (06.08.92 92/21)**

Die Akte enthält technische Angaben, die nach dem Eingang der Anmeldung eingereicht wurden und die nicht in dieser Patentschrift enthalten sind.

## (54) Spontan dispergierbares Konzentrat enthaltend einen Sterolester bzw. ein Sterolphosphatid.

(30) Priorität: **28.01.91 CH 257/91**

(43) Veröffentlichungstag der Anmeldung:
**30.06.93 Patentblatt 93/26**

(45) Bekanntmachung des Hinweises auf die Patenterteilung:
**10.05.95 Patentblatt 95/19**

(84) Benannte Vertragsstaaten:
**DE FR GB IT**

(56) Entgegenhaltungen:
**EP-A- 0 375 349**
**WO-A-91/05754**
**US-A- 3 852 311**
**US-A- 4 190 594**

(73) Patentinhaber: **MARIGEN S.A.**
**Eugster, Carl, Hackbergstrasse 40**
**CH-4125 Riehen (CH)**

(72) Erfinder: **EUGSTER, Carl**
**Hackbergstrasse 40**
**CH-4125 Riehen (CH)**
Erfinder: **EUGSTER, Conrad, Hans**
**Herrengütlistrasse 18**
**CH-8304 Wallisellen (CH)**
Erfinder: **HALDEMANN, Walter**
**Zeigerweg 23**
**CH-4102 Binningen (CH)**
Erfinder: **RIVARA, Giorgio Ospedale Maggiore**
**San Giovanni**
**Battista**
**Le Molinette**
**Corso Bramante, 88**
**I-10126 Torino (IT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

CHEMISTRY AND PHYSICS OF LIPIDS Bd. 20, Nr. 2, 1977, UTRECHT NL Seiten 131 -139; T. MURAMATSU ET AL: 'Synthesis of Phospholipids. III. Synthesis of 1,2-Dipalmitoyl-rac-Glyceryl-3 -Phosphoryl-3 Beta-Cholesterol and 1,2-Dipalmito yl-rac-Glyceryl-3-Phosphoryl-20-(3-Beta-Hydroxy Norpregn-5-ene)' siehe das ganze Dokument

CHEMISTRY AND PHYSICS OF LIPIDS Bd. 23, Nr. 1, 1979, UTRECHT NL Seiten 7 - 12;I. HARA ET AL: 'Immunochemical Properties of Phosphatidyl Cholesterol and itsHomologue' siehe das ganze Dokument

BIOCHIMICA ET BIOPHYSICA ACTA Bd. 775, Nr. 3, 5. September 1984, UTRECHT NLSeiten 426 - 434; MAHENDRA KUMAR JAIN ET AL: 'Lateral Interaction ofCholesterol in Diacylphosphatidylcholesterol Bilayers' siehe Seite 428

BIOCHIMICA ET BIOPHYSICA ACTA Bd. 691, Nr. 2, 7. Oktober 1982, UTRECHT NLSeiten 240 - 248; J. H. NOGGLE ET AL: 'Bilayers of Phosphatidyldiacylglyceroland Phosphatidylcholesterol Give 31P-NMR Spectra Characteristic for Hexagonaland Isotropic Phases' siehe Seite 247, Spalte 1 - Seite 247, Spalte 2

BIOCHIMICA ET BIOPHYSICA ACTA Bd. 600, Nr. 3, 14. August 1980, UTRECHT NLSeiten 678 - 688; MAHENDRA KUMAR JAIN ET AL: 'Phosphatidylcholesterol BilayersA Model for Phospholipid-Cholesterol Interaction' siehe Seite 679 - Seite 680

PHOSPHORUS AND SULFUR Bd. 17, Nr. 1, 1983, Seiten 67 - 71; F. RAMIREZ ET AL:'Covalent Models for Phospholipid-Sterol Interactions. Synthesis of Phosphatidyl-delta-5,7,9-Cholestatrien-3-beta-ol' siehe das ganze Dokument

CHEMICAL ABSTRACTS, vol. 97, no. 13, 27. September 1982, Columbus, Ohio, US;abstract no. 110220, A. K. SINGH: 'A Convenient and Improved Method for ThePreparation of Retinoates' Seite 634 ;Spalte 2 ; siehe Zusammenfassung &
SYNTH.COMMUN. Bd. 12, Nr. 6, 1982, SA 52323 030Seiten 447 - 451;

(74) Vertreter: **Haldemann, Walter, Dr.**
**c/o MARIGEN S.A.**
**40, Hackbergstrasse**
**CH-4125 Riehen (CH)**

SYNTHESIS. Nr. 10, Oktober 1977, STUTTGART DE Seiten 673 - 675; F. RAMIREZ ETAL: 'Synthesis of Phospholiposteroids. Phosphotidylcholesterol' Siehe das ganze-Artikel

CHEMICAL ABSTRACTS, vol. 100, no. 13, 26. März 1984, Columbus, Ohio, US;abstract no. 99762, M. M. GOMEZ ET AL: 'Effect of Sex Hormones on Junin VirusReplication' Seite 347 ;Spalte 1 ; siehe Zusammenfassung & COMUN. BIOL. Bd. 2,Nr. 2, 1983, SA 52323 030Seiten 161 - 166;

CHEMICAL ABSTRACTS, vol. 104, no. 9, 3. März 1986, Columbus, Ohio, US; abstractno. 62329, M. A. VAZOUEZ-ALCANTARA ET AL: 'Synthesis and Biological Assessmentof Long-Acting Estradiol Fatty Acid Esters in Overiectomized Rats' Seite 94 Spalte 1; siehe Zusammenfassung & J STEROID BIOCHEM. Bd. 23, Nr 5A, 1985, Seiten 599-602

CHEMICAL ABSTRACTS, vol. 112, no. 17, 23. April 1990, Columbus, Ohio, US;abstract no. 151977, M. A. VAZOUEZ-ALCANTARA ET AL: 'Long Acting EstrogenicResponses of Estradiol Fatty Acid Esters' Seite 93 ;Spalte 1 ; sieheZusammenfassung & J. STEROID BIOCHEM. SA 52323 030Bd. 33, Nr. 6, 1989, Seiten1111 - 1118;

A.KUKSIS et al. J.Org.Chem. 25, 1209 (1960)

Chem.Abstr. 111, 130382x (1989)

## Beschreibung

Die vorliegende Erfindung betrifft neue Sterolester, Sterolphosphatide und Sterolphosphonate, Verfahren zu ihrer Herstellung, spontan dispergierbare Mittel mit diesen Estern, Phosphatiden und Phosphonaten, sowie ihre Verwendung zur Behandlung von Tumoren.

In der CH-Patentschrift No. 678276-0 werden die aus den Extrakten der Samen der Sonnenblume (Helianthus annuus L.) und gewisser Kürbisarten (Cucurbita pepo L. und Cucurbita maxima Duch.) gewonnenen Sterole, deren Glucoside und deren Fettsäureester, wie auch die mit diesen Verbindungen hergestellten, spontan dispergierbaren Mittel und überdies ihre Verwendung zur Bekämpfung von Tumoren beschrieben.

Es wurde nun überraschenderweise gefunden, dass die neu synthetisierten Sterolester, Sterolphosphatide und Sterolphosphorsäure-diester ebenfalls eine überragende antitumorale Wirkung aufweisen, insbesondere dann, wenn diese Verbindungen in spontan dispergierbare Konzentrate eingearbeitet worden sind.

Die neuen Sterolester entsprechen den allgemeinen Formeln (I) bis (XV)

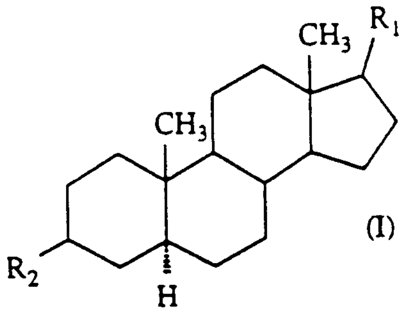

(I)

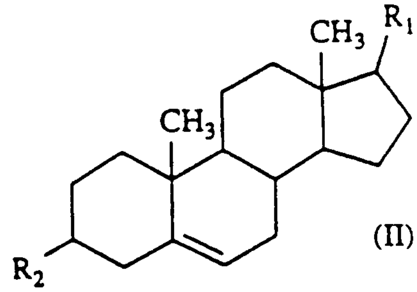

(II)

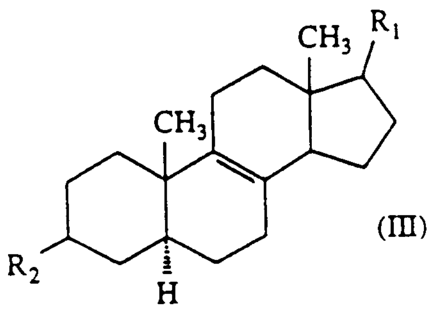

(III)

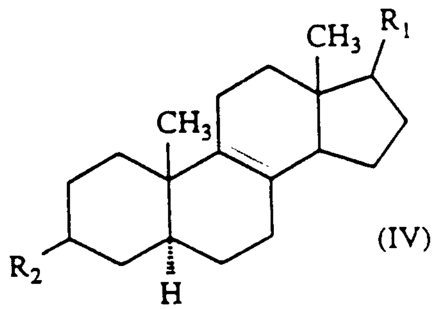

(IV)

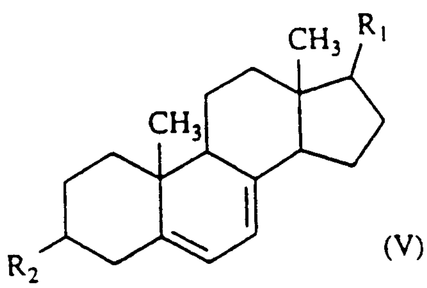

(V)

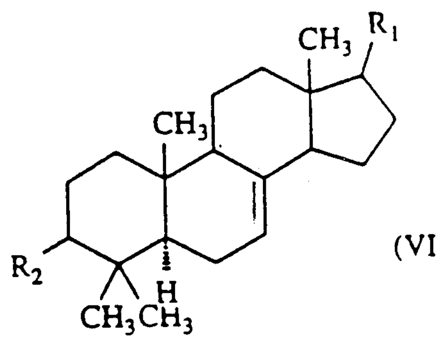

(VI)

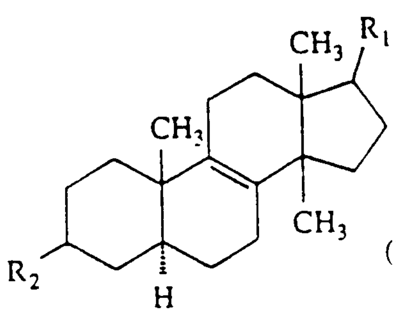

(VII)

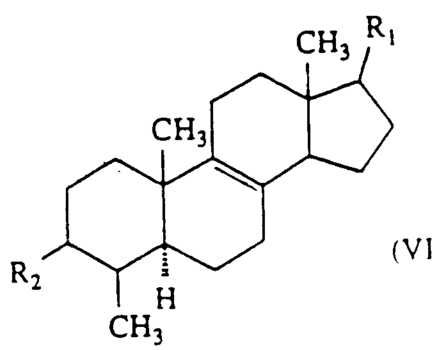

(VIII)

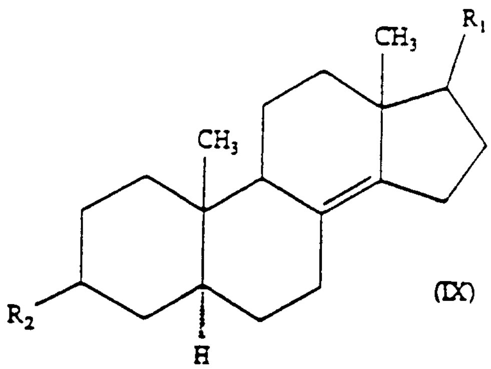

(IX)

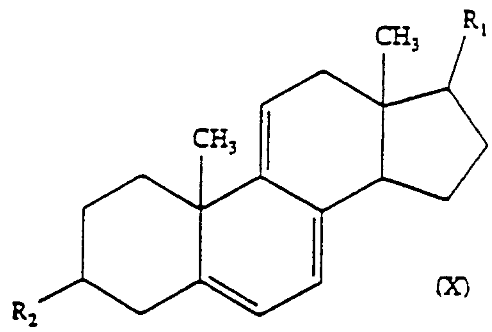

(X)

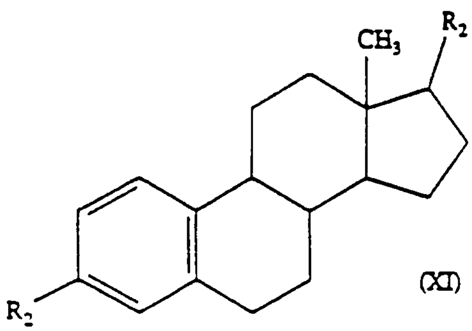

(XI)

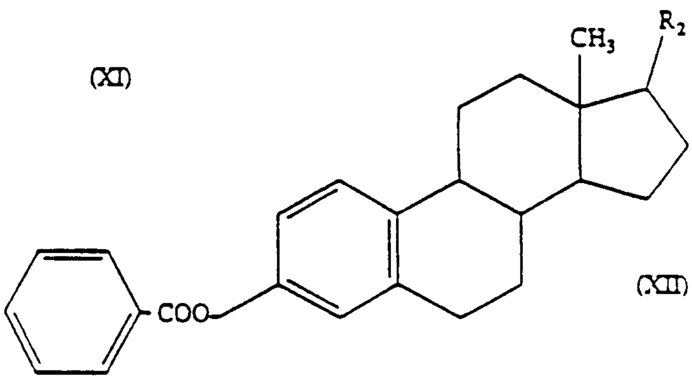

(XII)

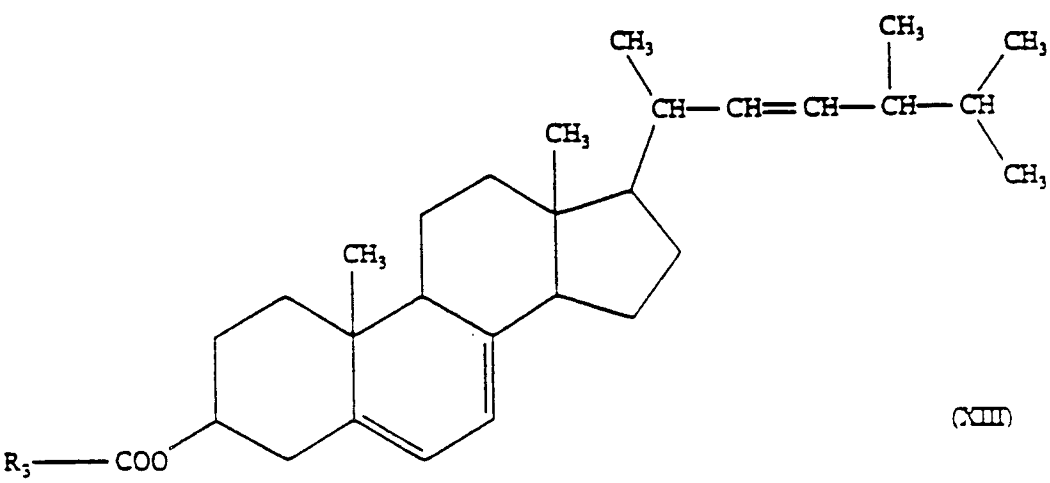

(XIII)

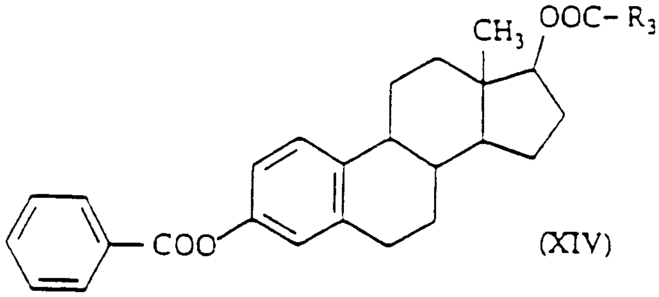

(XIV)

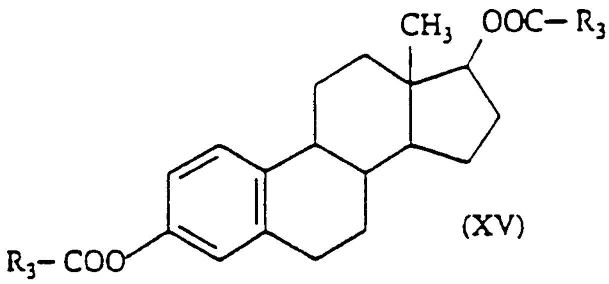

(XV)

wobei in den Formeln (I) bis (X) das Radikal $R_1$ eine $C_1$ bis $C_{10}$-Alkyl- oder eine $C_2$ bis $C_{10}$-Alkenylgruppe und das Radikal $R_2$ in den Formeln (I) bis (XII) eine Gruppe der Formeln (XVI) und (XVII) ist:

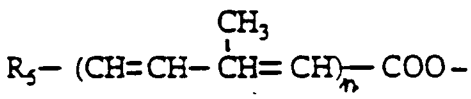

(XVI)

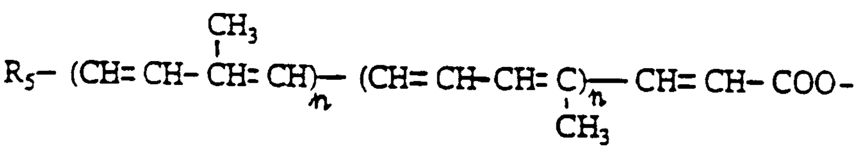

(XVII)

worin n die Zahlen 1,2,3,4 oder 5 bezeichnet und $R_5$ für die Radikale der Formeln

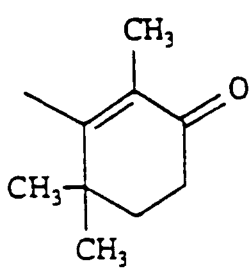 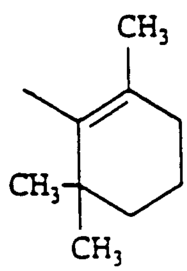 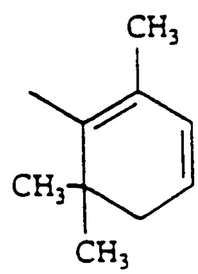

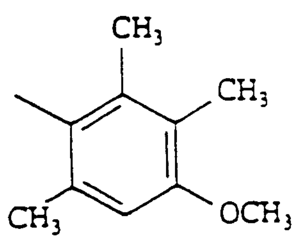 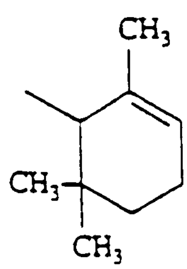 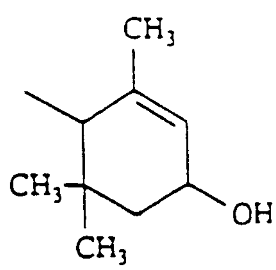

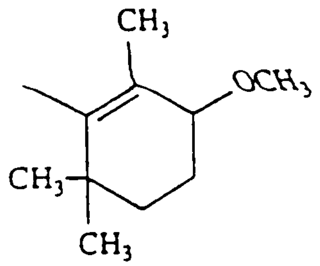

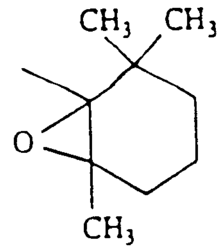

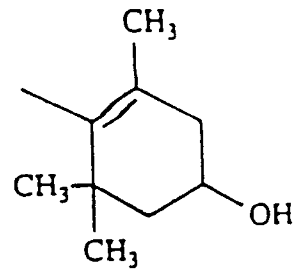

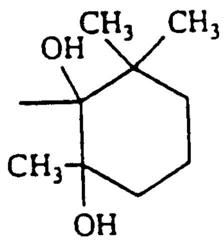

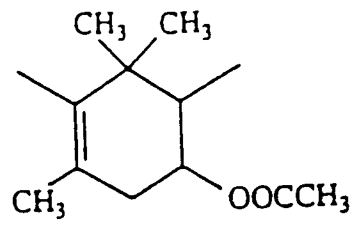

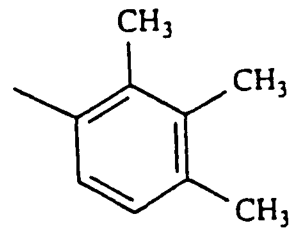

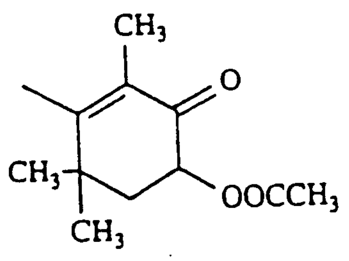

oder

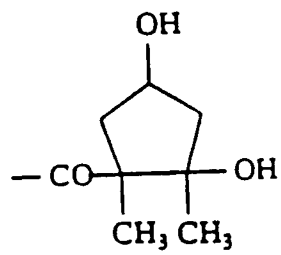

steht und wobei $R_3$ in den Formeln (XIII) bis (XV) für eine $C_4$ bis $C_{32}$-Alkylgruppe oder eine $C_4$ bis $C_{32}$-Alkenyl- bzw. Alkapolyengruppe (d.h. die entsprechenden Alkadiene, Alkatriene, Alkatetraene, Alkapentanene, Alkahexaene oder Alkaheptaene) steht;

mit der Massgabe, dass die Formel (II) nicht für Cholest-5-en-3-all trans-Retinat fungieren kann und dass die folgenden Estradioldiester der Formel (XV) ausgenommen sind: Estradioldiundecenoat, Estradioldipalmitat, Estradioldistearat und Estradioldioleat, und dass ferner im Falle der Verbindungen der Formel (XIII) die Ester der folgenden Säuren ausgeschlossen sind: Butter-, Isobutter-, Valerian-, Isovalerian-, Capron- Capryl-, Pelargon- Caprin- Laurin-, Tridecan-, Myristin-, Palmitin- Stearin-, Arachin- und Behensäure; ferner Palmitolein- Öl-, Linol- und Linolensäure.

Die Seitenketten bei $R_1$ und $R_3$ können geradkettig oder verzweigt sein. Bei $R_1$ haben die Alkyl- und die Alkenylgruppen vorzugsweise 8 bis 10 Kohlenstoffatome.

Beispiele solcher Alkyl- oder Alkenyl-Gruppen sind u.a.:

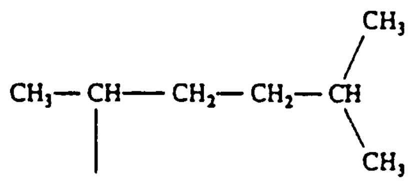

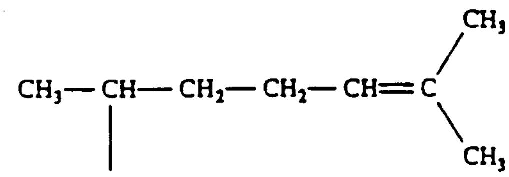

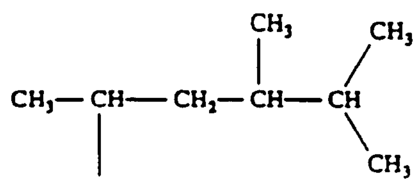

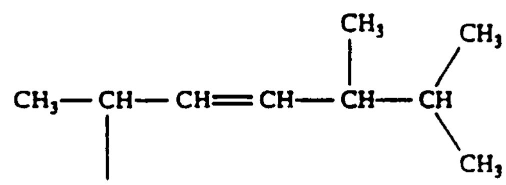

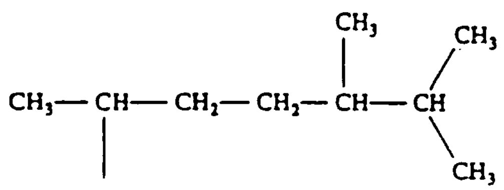

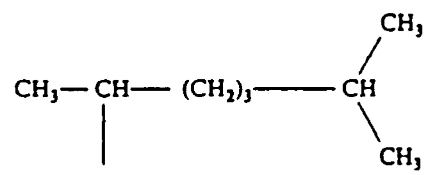

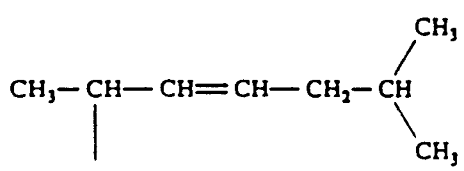

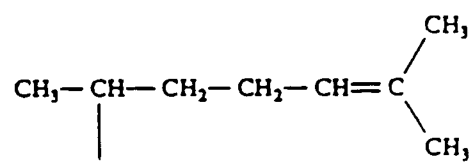

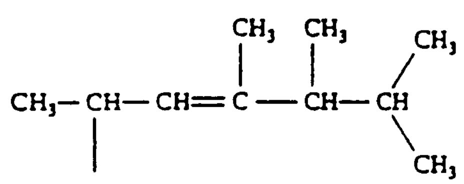

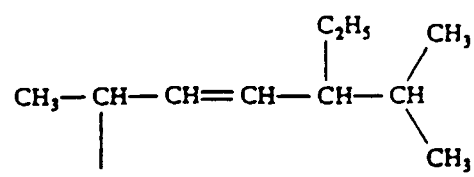

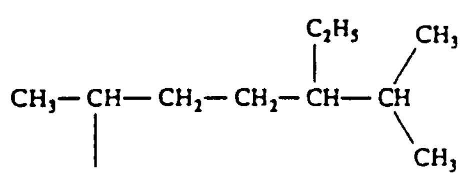

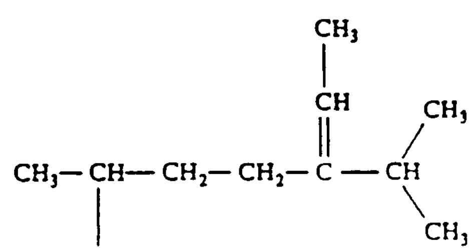

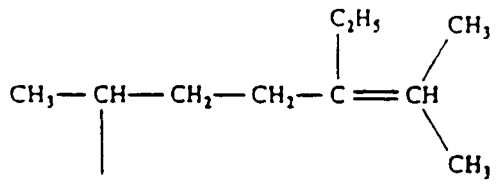

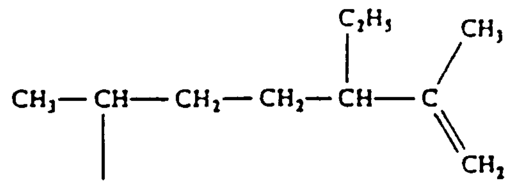

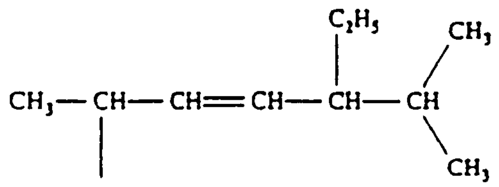

Bei $R_3$ haben die Alkyl- und Alkenyl-/Alkapolyengruppen (mit 1 - 7 Doppelbindungen) vorzugsweise 4 bis 22 Kohlenstoffatome. Besonders bevorzugt sind bei $R_3$ Alkyl- und Alkenyl-, bzw. Alkapolyengruppen mit 10 bis 20 Kohlenstoffatomen.

Wichtige Gruppen der Formeln (XVI) und (XVII) sind gekennzeichnet durch die Formeln (XX) oder (XXI):

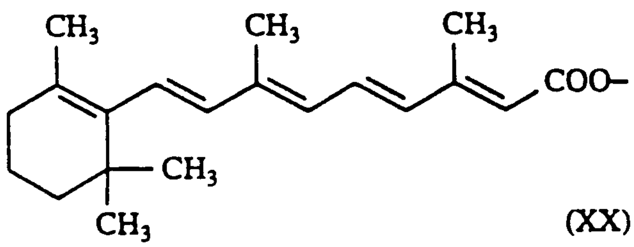

(XX)

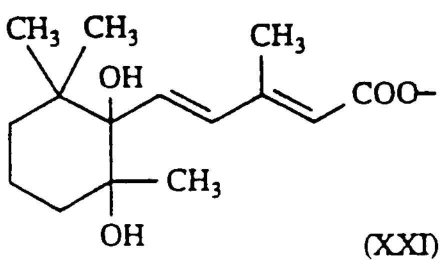

(XXI)

Die Gruppe der Formel (XX) liegt in verschiedenen stereoisomeren Formen, so z.B. als all-trans, als 9-cis oder als 13-cis-Form vor. Die wichtigste Gruppe der Formel (XVII) ist gekennzeichnet durch die Formel (XXII)

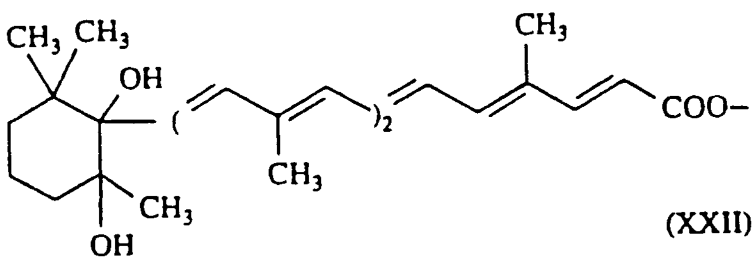

(XXII)

Von besonderer Bedeutung sind Verbindungen der Formeln (I) bis (XV), wobei ergänzend festgestellt sei, dass in den Formeln (I) bis (XII) das Radikal $R_1$ eine $C_1$ bis $C_{10}$-Alkyl- oder eine $C_2$ bis $C_{10}$-Alkenylgruppe

und das Radikal $R_2$ in den Formeln (I) bis (XII) eine Verbindung der Formel (XX) ist:

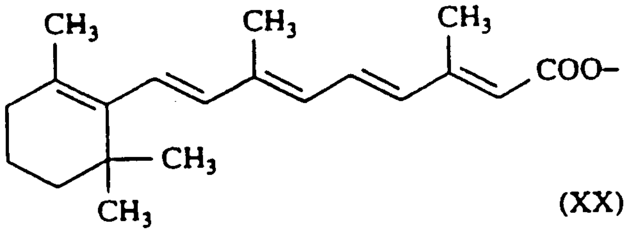

(XX)

oder die Verbindung (XXII) bezeichnet:

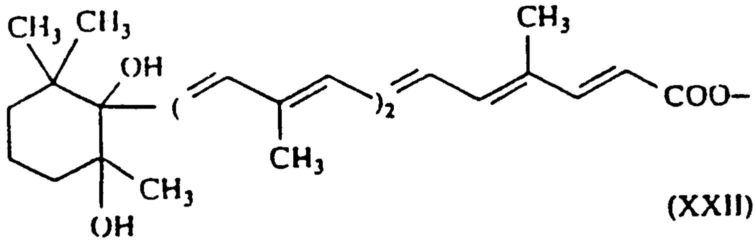

(XXII)

Beispiele von erfindungsgemässen neuen Sterolestern der Formeln (I) bis (XV) sind u.a.:

Ergosta-5,7,22-trien-3-ol-all-trans-retinat
Ergosta-5,7,22-trien-3-ol-13-cis-retinat
Stigmast-5-en-3-ol-all-trans-retinat
(β-Sitosterol-all-trans-retinat)
Stigmast-5-en-3-ol-13-cis-retinat
(β-Sitosterol-13-cis-retinat)
Stigmast-5-en-3-ol-azafrinat
Stigmasta-5,22-dien-3-ol-all-trans-retinat
(Stigmasterol-all-trans-retinat)
Stigmasta-5,22-dien-3-ol-13-cis-retinat
(Stigmasterol-13-cis-retinat)
Stigmasta-5,22-dien-3-ol-azafrinat
Ergosta-5,7,22-trien-3-ol-crotonat
Ergosta-5,7,22-trien-3-ol-undecenoat
Ergosta-5,7,22-trien-3-ol-dodecenoat
Ergosta-5,7,22-trien-3-ol-azafrinat
β-Oestradiol-3,17-diretinat
β-Oestradiol-3-benzoat-17-undecenoat
β-Oestradiol-3-benzoat-17-retinat

Die Sterolester, wie auch die Sterolphosphorverbindungen der Formeln (I) bis (XII) lassen sich allgemein nach folgenden, an sich bekannnten Verfahren herstellen:

a) Umsetzung einer Verbindung der Formel (XXIII) und (XXIV):

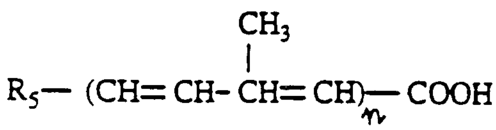

(XXIII)

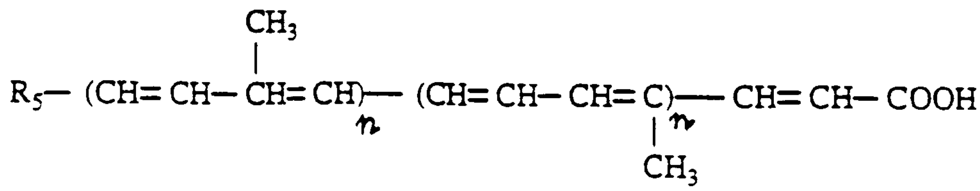

(XXIV)

worin n die Zahlen 1,2,3,4 oder 5 bedeutet und $R_5$ für die Radikale der folgenden Formeln:

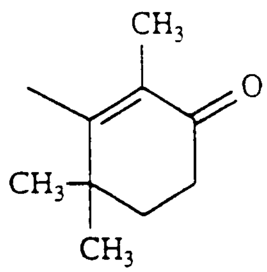
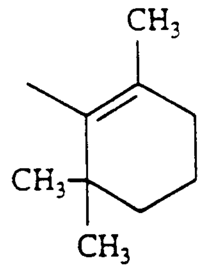
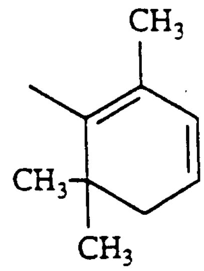

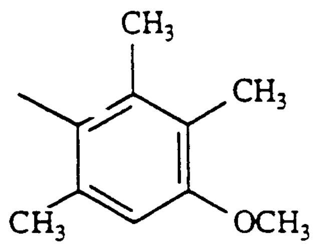
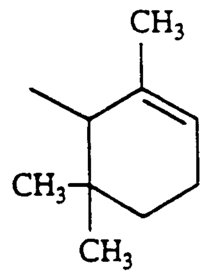
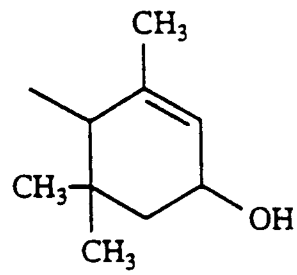

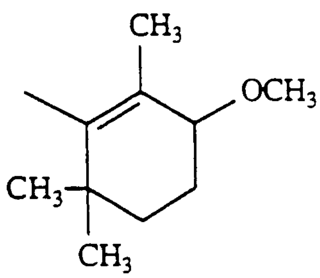
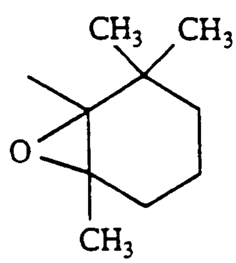
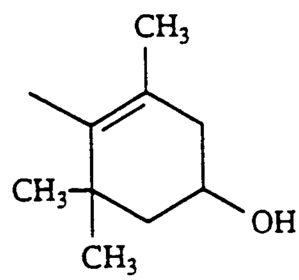

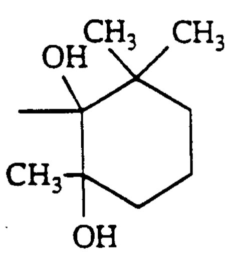
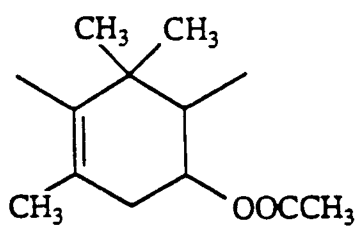
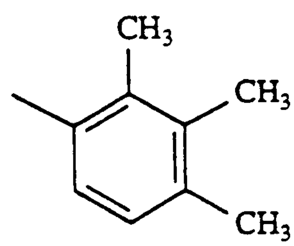

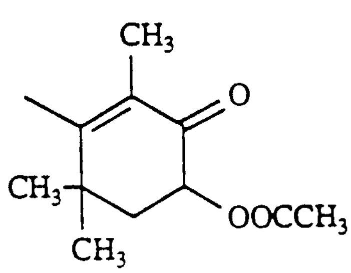

oder

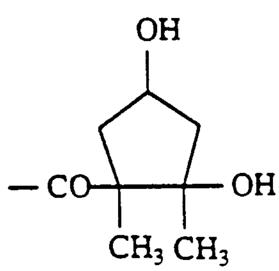

steht, mit N,N'-Carbonyl-diimidazol bei 25 bis 70 ° C unter Zusatz einer katalytischen Menge eines Alkoholates in einem indifferenten Lösungsmittel wie z.B. Tetrahydrofuran, Benzol, Chloroform oder Dimethylformamid und anschliessender Alkoholyse der gebildeten Imidazolide mit einem Sterol der Formeln (XXV) bis (XXXVI) :

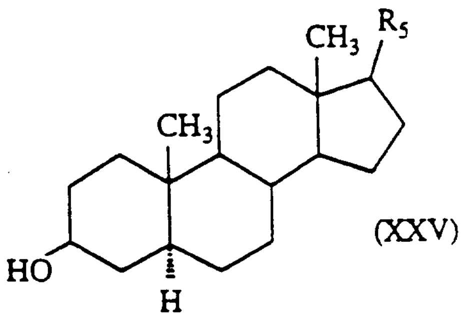

(XXV)

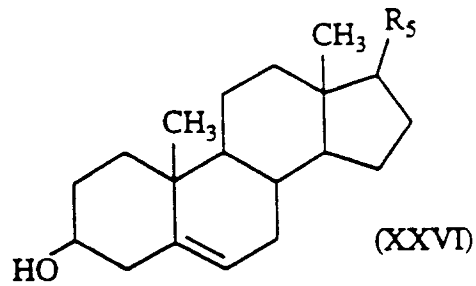

(XXVI)

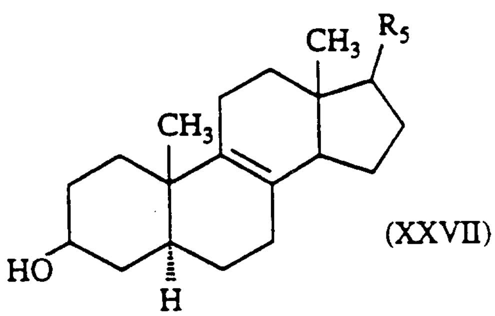

(XXVII)

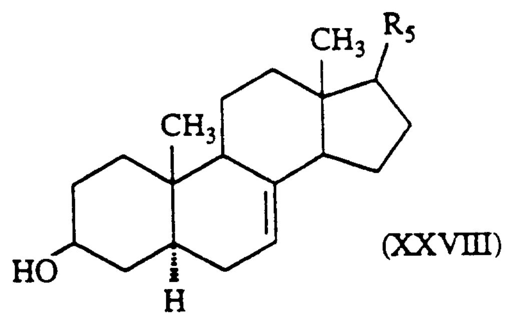

(XXVIII)

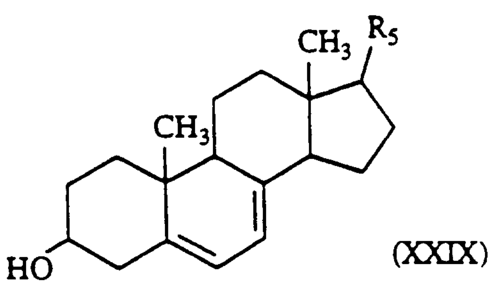

(XXIX)

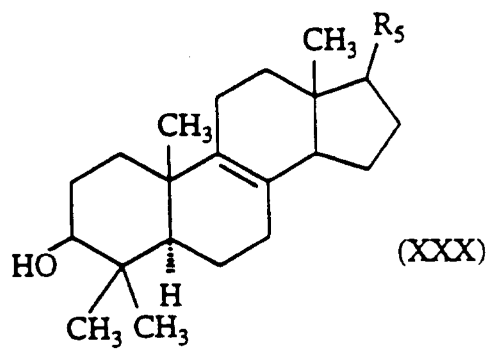

(XXX)

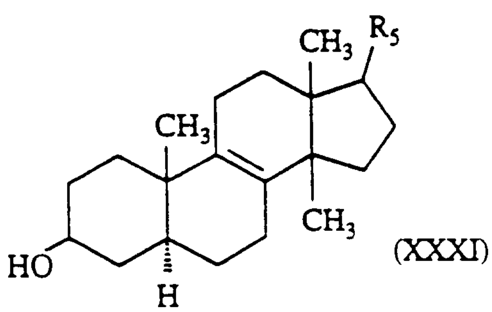

(XXXI)

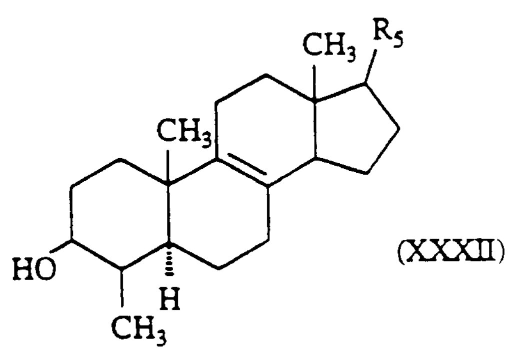

(XXXII)

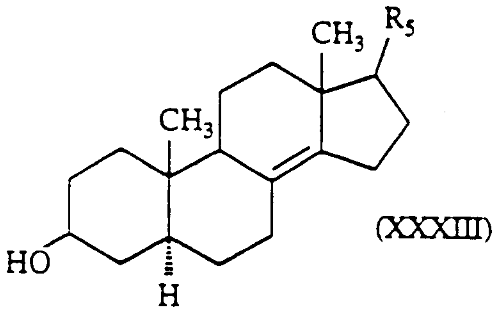

(XXXIII)

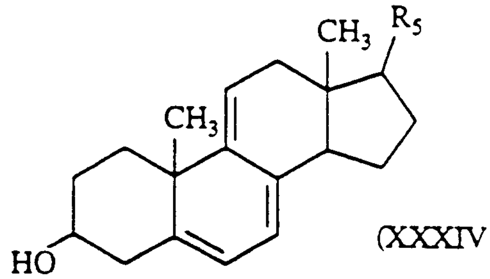

(XXXIV)

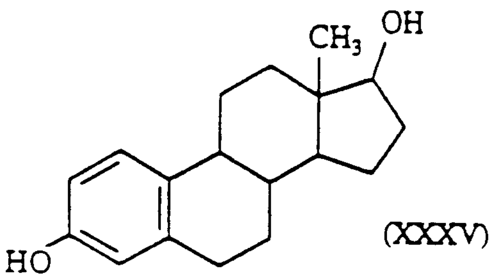

(XXXV)

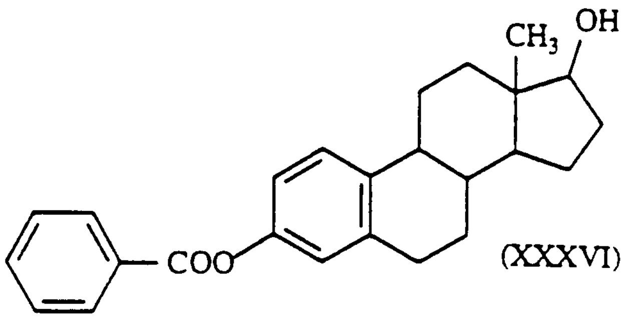

(XXXVI)

worin das Radikal $R_5$ für eine $C_1$ bis $C_{10}$-Alkyl- oder eine $C_2$ bis $C_{10}$-Alkenylgruppe steht.

b) Bildung des Chlorides, bzw. des Bromides einer Verbindung der Formeln (XXIII) oder (XXIV) :

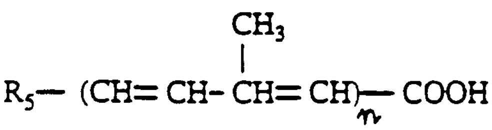

(XXIII)

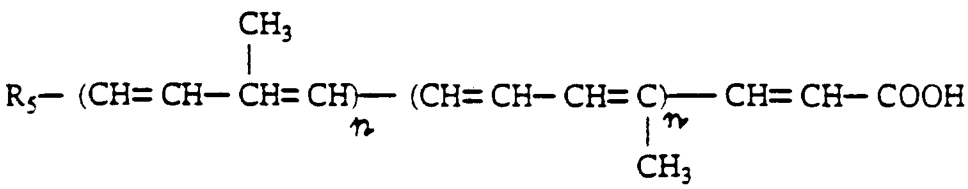

(XXIV)

worin n die Zahlen 1,2,3,4 oder 5 bedeutet und $R_5$ für die Radikale mit folgenden Formeln steht:

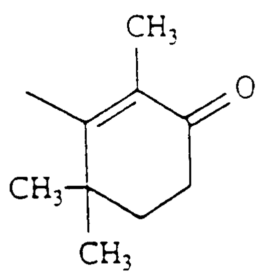
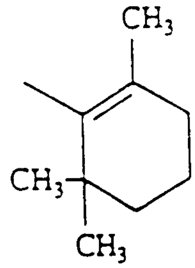
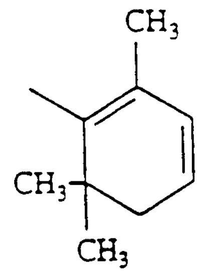

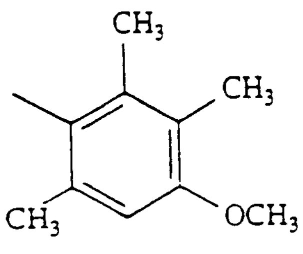
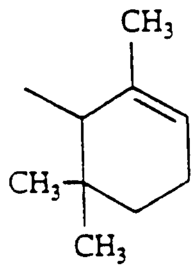
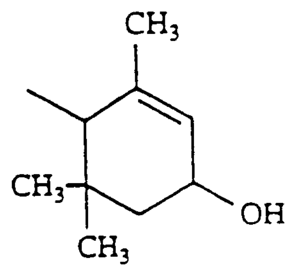

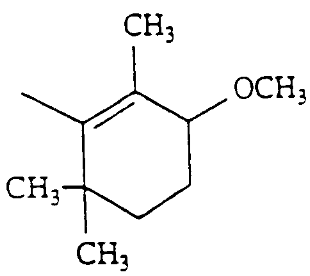
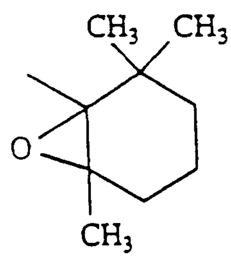
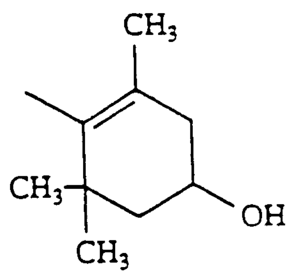

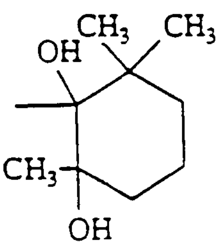
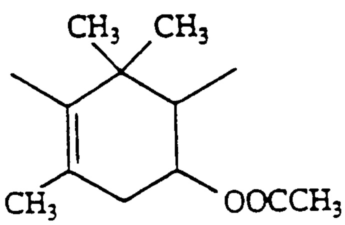
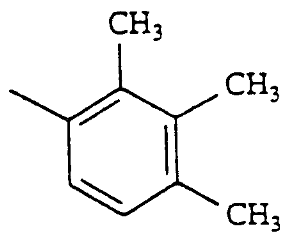

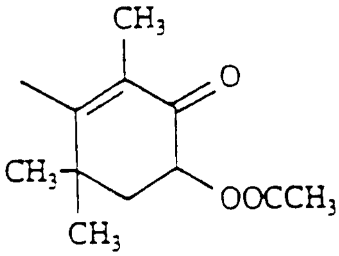

oder

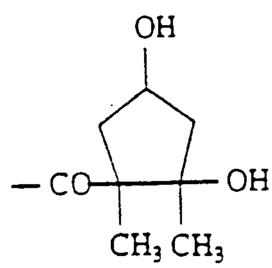

mit einem Chlorierungs- oder Bromierungsmittel, wie z.B. Thionylchlorid, Oxalychlorid oder Oxalylbromid, und anschliessender Umsetzung mit einem Sterol der Formeln (XXV) bis (XXXVII) [Vgl. Verfahren a)] bei einer Temperatur von 40 bis 120 °C in einem indifferenten Lösungsmittel wie z.B. Toluol oder Xylol und in Gegenwart eines Katalysatoren wie z.B. Dimethylformamid oder p-Dimethylaminopyridin.

c) Chlorierung oder Bromierung einer Verbindung der Formel (XXXVIII) :

$R_7$ - COOH (XXXVIII)

worin $R_7$ eine $C_4$ bis $C_{32}$-Alkyl- oder eine $C_4$ bis $C_{32}$-Alkenyl-, bzw. Alkapolyengruppe darstellt, mit einem Chlorierungs-, bzw. Bromierungsmittel wie z.B. Thionylchlorid, Oxalylchlorid oder Oxalylbromid und anschliessender Reaktion der entstandenen Chloride, bzw. Bromide mit einem Sterol der Formeln (XXXV) oder (XXXVII) :

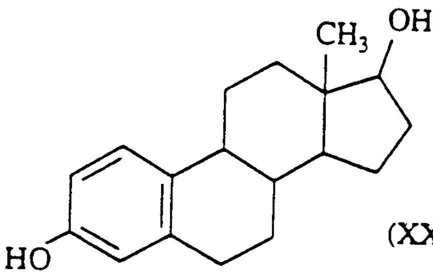

(XXXV)

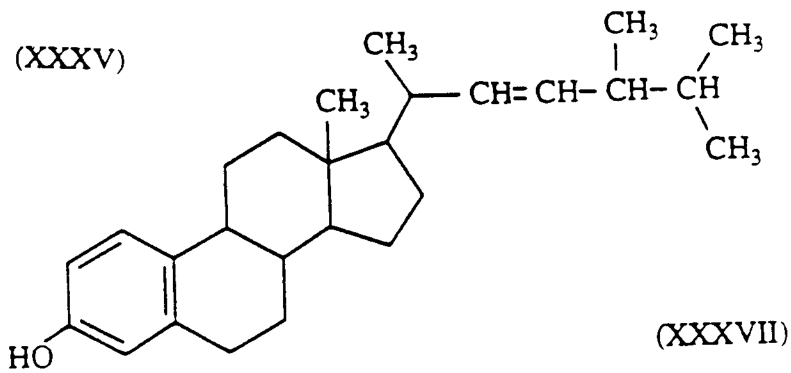

(XXXVII)

bei einer Temperatur zwischen 40 und 120 °C in einem indifferenten Lösungsmittel wie z.B. Tetrachlorkohlenstoff, Chloroform, Toluol oder Xylol in Gegenwart eines Katalysatoren wie z.B. Dimethylformamid oder p-Dimethyl-aminopyridin.

d) Veresterung einer Verbindung der Formel (XXXIX) :

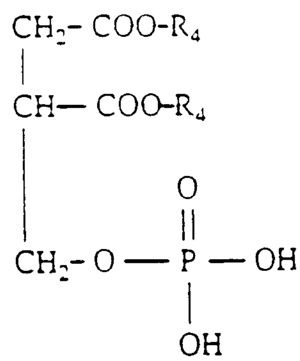

(XXXIX)

worin $R_4$ eine $C_1$ bis $C_{32}$-Alkyl- oder eine $C_2$ bis $C_{32}$-Alkenyl-, bzw. Alkapolyengruppe darstellt, in einem inerten Lösungsmittel wie z.B. Pyridin, Tetrahydrofuran oder Chloroform bei einer Temperatur von 20 °C mit Pivaloylchlorid und anschliessender Umsetzung des Reaktionsproduktes in der gleichen Lösung mit einem Sterol der Formeln (XXV) bis (XXXVII).

e) Bildung des Hydrogenphosphonates von einem Sterol der Formeln (XXV) bis (XXXVII) in einem inerten Lösungsmittel wie z.B. Acetonitril unter Zusatz von Phosphortrichlorid und Imidazol und anschliessender Umsetzung der gereinigten und getrockneten Rohprodukte in einem inerten Lösungsmittel wie z.B. Pyridin, Tetrahydrofuran oder Chloroform bei einer Temperatur von 20 °C unter Zusatz von Pivaloylchlorid mit einer Verbindung der Formeln (XL), (XLI) oder (XLII) :

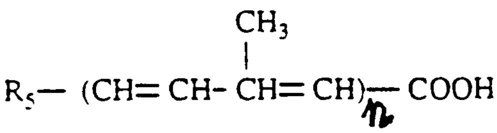

(XL)

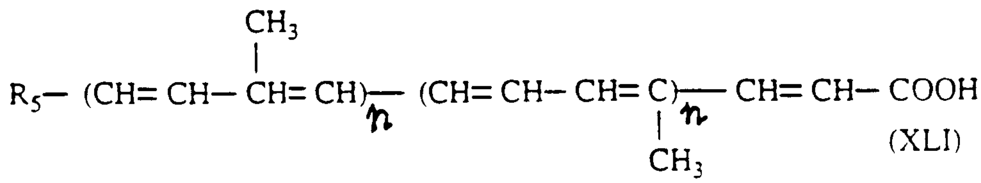

(XLI)

$R_6$ - OH (XLII)

worin n die Zahlen 1,2,3 oder 4 meint und $R_5$ für die Radikale mit folgenden Formeln:

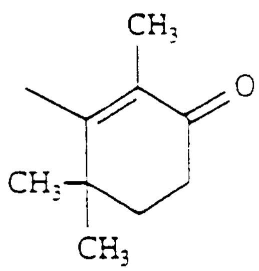

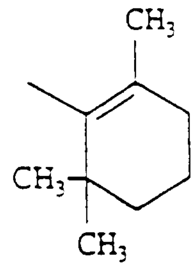

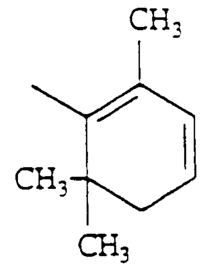

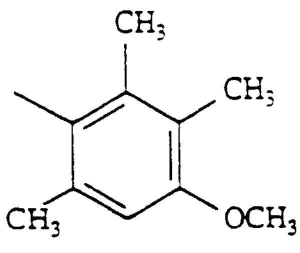

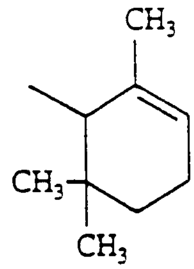

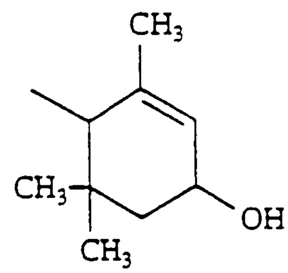

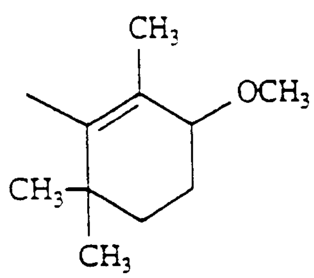

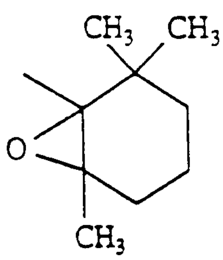

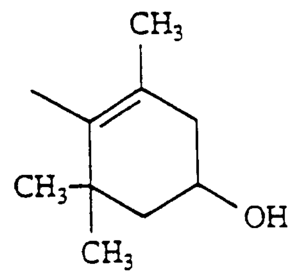

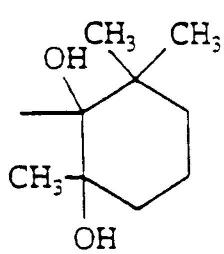

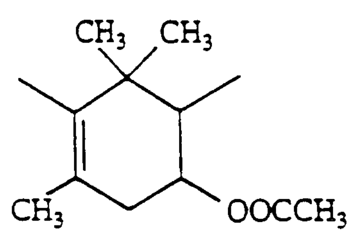

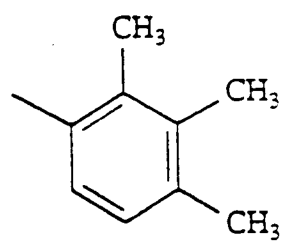

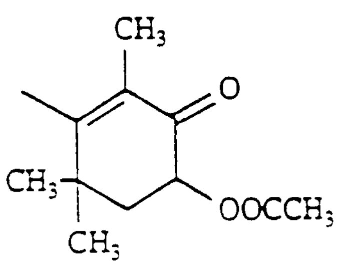

oder

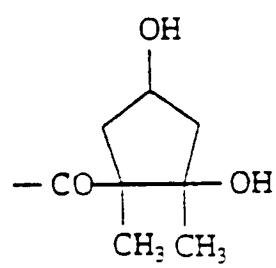

steht und $R_6$ eine $C_4$- bis $C_{32}$-Alkyl- oder eine $C_4$- bis $C_{32}$-Alkenyl-, bzw. Alkapolyengrupe darstellt.

Die entsprechenden Phosphothioatverbindungen der Sterole der Formeln (XXV) bis (XXXVII) werden aus den jeweils dazu passenden, getrockneten Hydrogenphosphonaten (vgl. oben) unter Zusatz von Pivaloylchlorid und Schwefel in einem inerten Lösungsmittel wie z.B. Pyridin oder Toluol hergestellt.

Die neuen Sterolester, Sterolphosphatide und Sterolphosphonatverbindungen haben überraschenderweise eine ausgezeichnete antitumorale Wirkung, vor allem dann, wenn sie in spontan dispergierbare Konzentrate eingearbeitet worden sind.

Gegenstand der vorliegenden Erfindung sind deshalb auch spontan dispergierbare Konzentrate mit diesen Verbindungen. Solche Konzentrate ergeben, mit Wasser versetzt, Mikroemulsionen von hervorragender Phasenstabilität und von überaus deutlich erhöhtem Permeations- und Spreitungsvermögen.

Die erfindungsgemässen spontan dispergierbaren Konzentrate enthalten :

7,5 bis 25 Gewichts-% eines Sterolesters, bzw. Sterolphosphatids der Formeln (I) bis (XV), bzw. einer Kombination solcher Komponenten

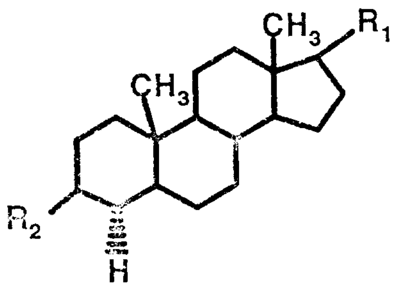

(I)

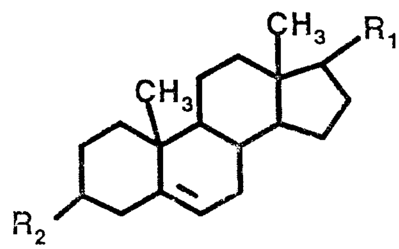

(II)

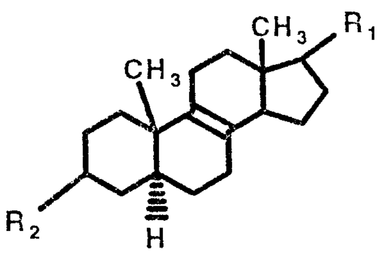

(III)

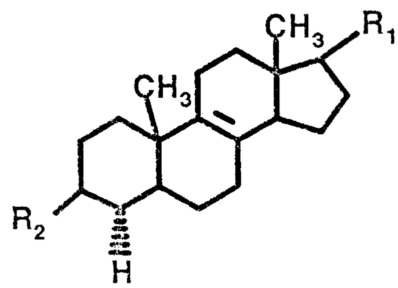

(IV)

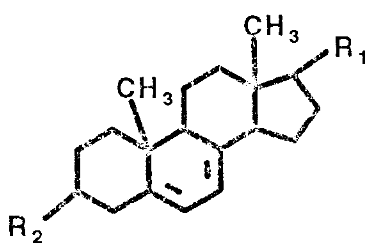

(V)

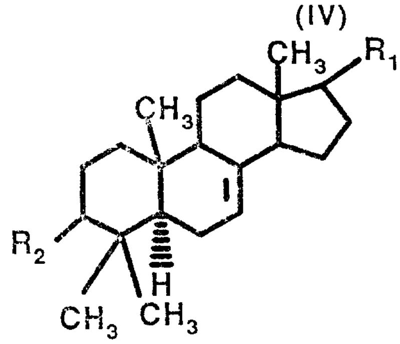

(VI)

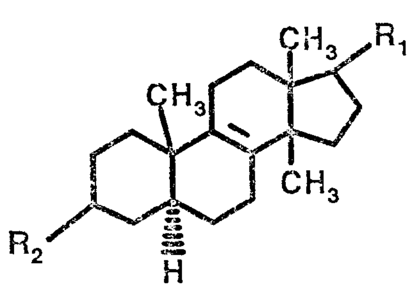

(VII)

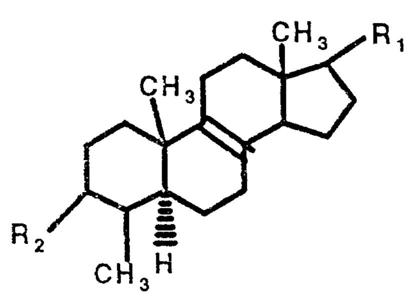

(VIII)

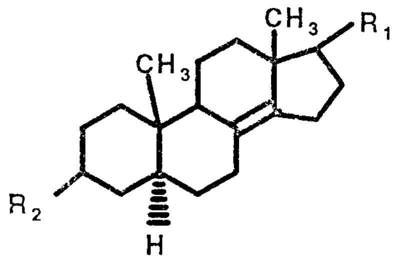

(IX)

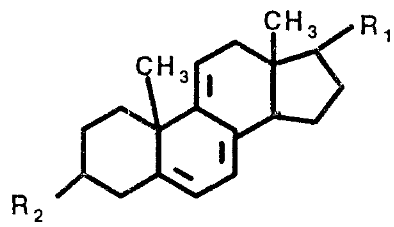

(X)

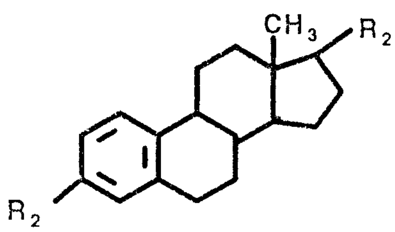

(XI)

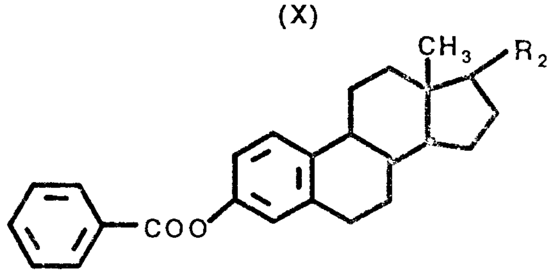

(XII)

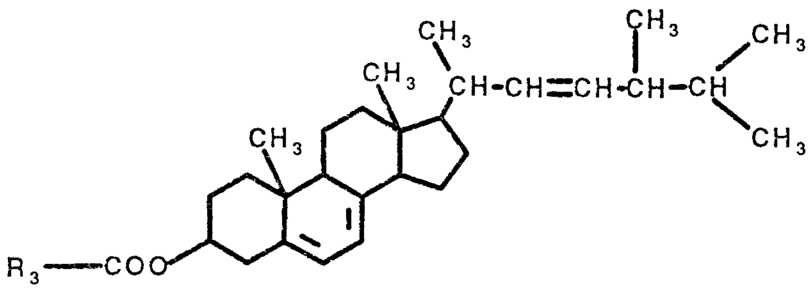

(XIII)

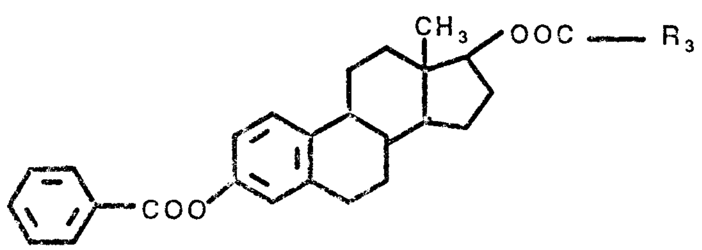

(XIV)

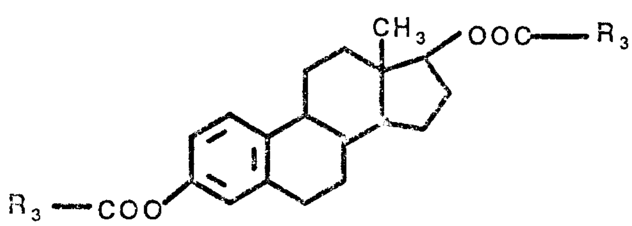

(XV)

wobei in den Formeln (I) bis (X) das Radikal $R_1$ eine $C_1$- bis $C_{10}$-Alkyl- oder eine $C_2$- bis $C_{10}$-Alkenylgruppe und das Radikal $R_2$ in den Formeln (I) bis (XII) eine Gruppe der Formeln (XVI) oder (XVII) ist

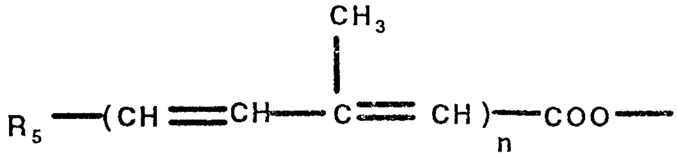

(XVI)

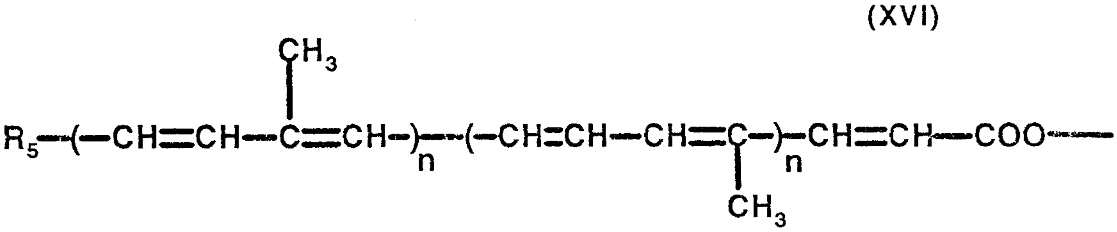

(XVII)

worin n die Zahlen 1, 2, 3, 4 oder 5 bezeichnet und $R_5$ für ein Radikal der Formeln:

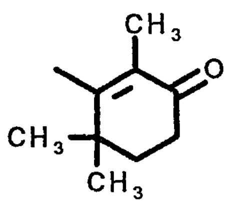

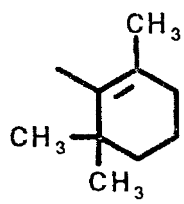

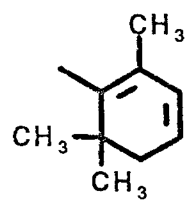

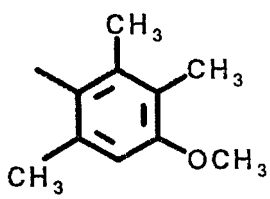

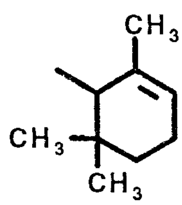

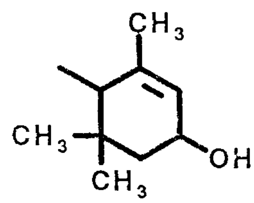

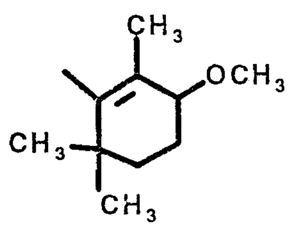

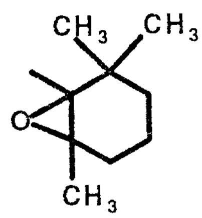

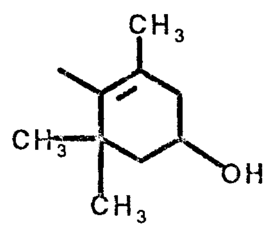

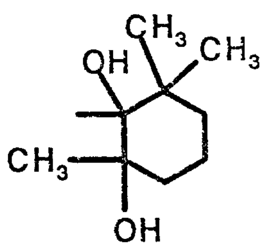

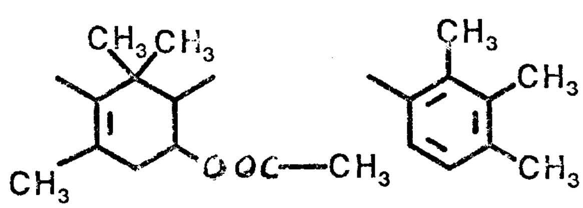

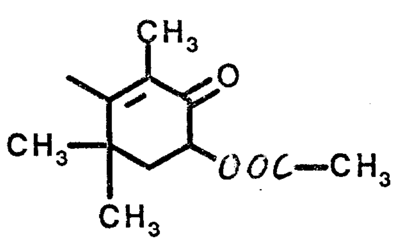

oder

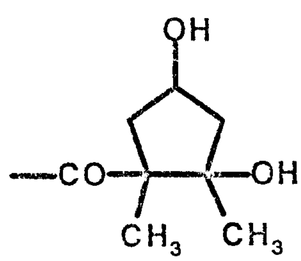

steht oder aber die Gruppe gemäss der Formel (XVIII) darstellt

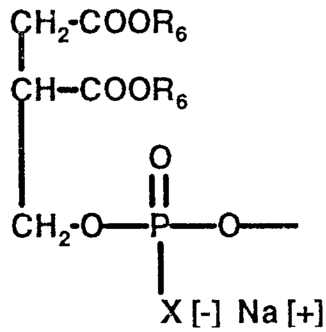

(XVIII)

in welcher $R_6$ eine $C_1$- bis $C_{32}$-Alkyl- oder eine $C_2$- bis $C_{32}$-Alkenyl- bzw. Alkapolyengruppe und X Sauerstoff oder Schwefel bedeuten, und wobei $R_3$ in den Formeln (XIII) bis (XV) für eine $C_4$- bis $C_{32}$-Alkylgruppe oder eine $C_4$- bis $C_{32}$- Alkenyl- bzw. Alkapolyengruppe steht
0 bis 40 Gewichts-% Isopropylmyristat, Isopropylpalmitat oder Neutralöl
und 22,5 bis 40 Gewichts-% des tert. Octylphenylpolyoxyäthylenäthers mit
9 bis 10 Oxyäthylengruppen, sowie
22,5 bis 40 Gewichts-% eines Mischemulgators bestehend aus je 50 % der beiden Verbindungen der Formeln

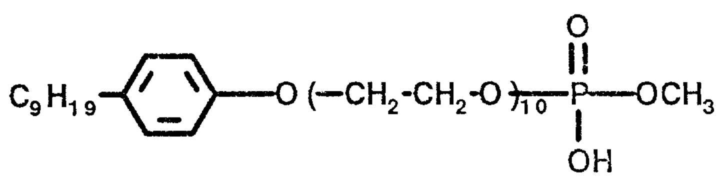

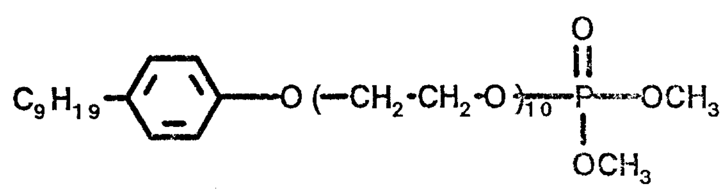

oder eines Emulgators der Formel

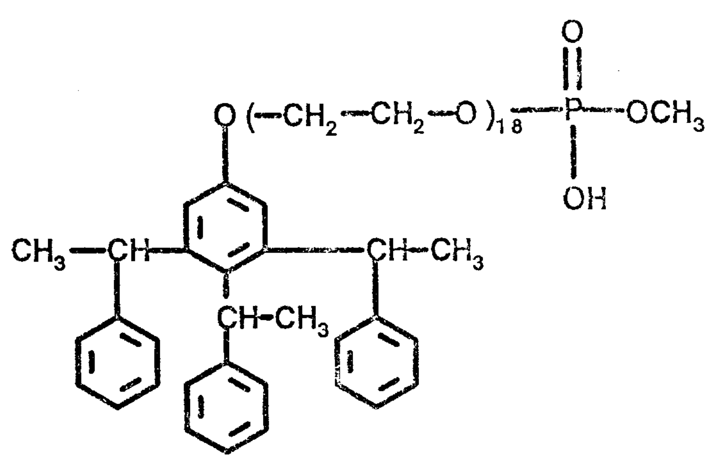

0 bis 10 Gewichts-% eines Vitamins oder Provitamins,
0 bis 10 Gewichts-% einer freien Fettsäure und gegebenenfalls übliche Trägerstoffe und/oder Verdünnungsmittel.

Die erfindungsgemäss einzusetzenden Tenside oder Tensidgemische können anionaktiv, kationaktiv, amphoter oder nichtionogen sein. Am besten sind sie nicht-ionogen und haben einen HLB-Wert (d.h. eine "hydrophilic-lipophilic-balance") zwischen 2 - 18; bevorzugt liegt er zwischen 2 - 6 einerseits und 10 - 15 anderseits. HLB-Werte geben Auskunft über die lipophilen und hydrophilen Eigenschaften eines Emulgators. Vgl. dazu "Hydrophile - Lipophile Balance: History and recent Developments" von Paul Becher im Journal of Dispersion Science and Technology. 5 (1), 81-96 (1984).

Geeignete anionische Tenside können sowohl sog. wasserlösliche Seifen als auch wasserlösliche synthetische Verbindungen sein.

Als Seifen eignen sich die Alkali-, Erdalkali- oder gegebenenfalls substituierten Ammoniumsalze von höheren Fettsäuren ($C_{10}$ bis $C_{22}$), wie z.B. die natürlichen Na- oder K-Salze der Oel- oder Stearinsäure, oder von natürlichen Fett-säuregemischen, welche sich u.a. aus Kokosnuss- oder Talgöl gewinnen lassen. Ferner sind als Tenside auch die Fettsäure-Methyltaurinsalze, sowie modifizierte und nicht-modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate und -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst. Beispiele hiefür sind das Na- oder Ca-Salz der Ligninsulfosäure, des Dodecylschwefelsäure-esters und Sulfonsäuren von Fettalkohol-Aethylenoxyd-Addukten. Die sulfonierten Benzimidazol-Derivate enthalten vorzugsweise zwei Sulfonsäuregruppen und einen Fettsäurerest mit etwa 8 - 22 C-Atomen. Alkylaryl-Sulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Als nichtionische Tenside stehen in erster Linie zur Wahl die Polyglykolätherderivate von aliphatischen oder cycloaliphatischen Alkoholen, gesättigten oder ungesättigten Fettsäuren und Alkylphenolen, welche 3 bis 30 Glykoläthergruppen und 8 bis 20 C-Atome im (aliphatischen) Kohlenwasserstoffrest und 6 bis 18 C-Atome im Alkylrest enthalten können. Weiterhin kommen als nichtionische Tenside in Frage die wasserlöslichen, 20 bis 250 Aethylenglykoläthergruppen und 10 bis 100 Propylenäthergruppen enthaltenden Polyäthylenoxydaddukte an Polypropylenglykol und Alkylpolypropylenglykol mit 1 bis 10 C-Atomen in der Alkylkette. Die genannten Verbindungen enthalten üblicherweise pro Propylenglykol-Einheit 1 bis 5 Aethylenglykoleinheiten.

Als Beispiele nicht-ionischer Tenside seien erwähnt: Nonylphenolpolyäthoxyäthanole, Ricinusölpolyglykoläther, Polypropylen-Polyäthylenoxyd-Addukte, Tributylphenoxy-polyäthoxyäthanol, Polyäthylenglykol und Octylphenoxypolyäthoxyäthanol. Ueberdies kommen auch Fettsäureester von Polyoxyäthylensorbitan, wie das Polyoxyäthylensorbitantrioleat, in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorab als Halogenide, Methylsulfate oder Aethylsulfate vor, z.B. das Stearyltrimethylammoniumchlorid oder das Benzyl-di-(2-chloräthyl)-äthylammoniumbromid.

In hohem Masse bevorzugt zur Herstellung von erfindungsgemässen, spontan dispergierbaren Konzentraten sind Phosphorsäureestertenside, wie z.B. :

Tristyrylphenolpolyoxyäthylen-18-mono/dimethyl-Phosphorsäureester (Soprophor® FL, Rhône-Poulenc);

Nonylphenol-10-polyoxyäthylen-mono/dimethylphosphorsäureester (Diphasol® 3873, CIBA-GEIGY);

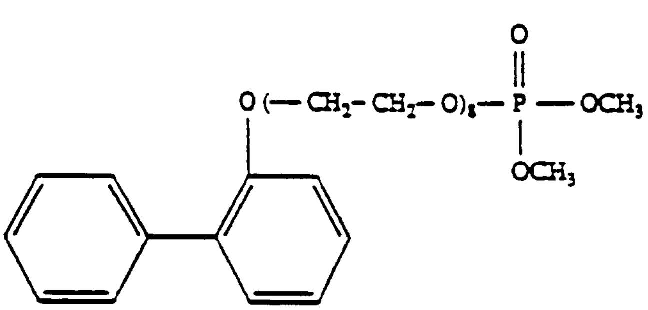

(Tensid 508, CIBA-GEIGY);
Tinovetin® JU (CIBA-GEIGY), ein Hydroxybiphenyl-10-Aethoxy-Phosphorsäureester;
Butyl-mono-4-Aethoxy-Phosphorsäureester (Zerostat® AT, CIBA-GEIGY), bzw.

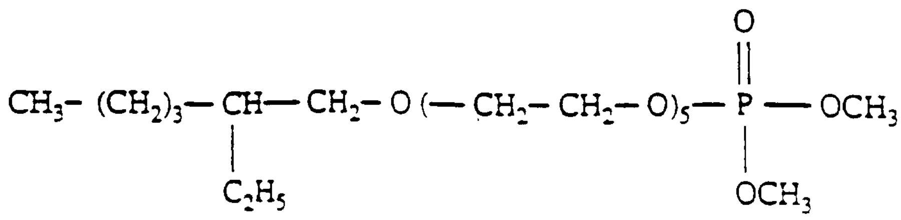

(Zerostat AN® , CIBA-GEIGY)
Als Hydrotrop, bzw. Co-Emulgator dienende, pharmaverträgliche Lösungsmittel lassen sich einsetzen, z.B.: Ester eines aliphatischen Alkohols ($C_3$ - $C_{18}$) mit einer aliphatischen Carbonsäure ($C_{10}$ - $C_{22}$), wie etwa Isopropyllaurat, Hexyllaurat, Decyllaurat, Isopropylmyristat, Isopropylpalmitat und Laurylmyristat; Kohlenwasserstoffe mit einer geraden Kohlenstoffkette ($C_{12}$ - $_{32}$), welche mit 6 - 16 Methylgruppen substituiert ist und bis 6 Doppelbindungen aufweisen kann, wofür Terpene wie Polymethylbutane und Polymethylbutene als Beispiele dienen mögen.

Mono-Ester aus Aethylenglykol oder Propylenglykol mit einer aliphatischen Carbonsäure ($C_6$ - $C_{22}$), wie etwa Propylenglykolmonolaurat und Propylenglykolmonomyristat.

Ester aus einem aliphatischen Alkohol ($C_{12}$ - $C_{22}$) mit Milchsäure, wie z.B. Myristyl- oder vorzugsweise Lauryl-Lactat; Mono-, Di- oder Triester des Glycerins mit einer aliphatischen Carbonsäure ($C_6$ - $C_{22}$), wie z.B. Glyceryl-Caprylat, oder Miglyol® 812 Neutralöl (Oleum neutrale).

Ester aus einem Poly(2 - 7)äthylenglykolglyzerinäther mit mindestens einer freien Hydroxylgruppe mit einer aliphatischen Carbonsäure ($C_6$ - $C_{22}$), wie z.B. aliphatische Alkohole ($C_{12}$ - $_{22}$), somit u.a. Dodecanol, Tetradecanol, Oleylalkohol, 2-Hexyldecanol und 2-Octyl-decanol.
Ester mit mindestens einer freien Hydroxylgruppe, aus Poly(2-10)glykol mit einer aliphatischen Carbonsäure ($C_6$ - $C_{22}$),
Monoäther aus einem Polyäthylenglykol mit einem aliphatischen Alkohol ($C_{12}$ - $_{18}$), wie z.B. Polyoxyäthylen-($C_{10}$)octyläther.

Heterocyclische Verbindungen, wie z.B. 1-Methyl-2-Pyrrolidon.

Alle technischen Tenside wurden vor dem Eintrag in die spontan dispergierbaren Konzentrate mittels Filtration, bzw. Chromatographie über neutralem Aluminiumoxyd mit einem inerten Lösungsmittel wie z.B. Tetrahydrofuran, Aethylalkohol oder Methylenchlorid gereinigt.

Als Zusätze in die erfindungsgemässen spontan dispergierbaren Konzentrate eignen sich Vitamine und Provitamine (wie z.B. Vitamin A, Retinol, Carotine, Tocopherole), sowie auch freie Fettsäuren wie etwa: Valeriansäure, Isovaleriansäure, Sorbinsäure, Isocapronsäure, Laurinsäure, Myristinsäure, Palmitinsäure, Stearinsäure, Arachidsäure, Behensäure, Hexacosansäure, Octacosansäure, Pentadecansäure, Decenylsäure, Undecenylsäure, Dodecenylsäure, Oleinsäure, Linolsäure, Linolensäure, Arachidonsäure, Erucasäure, etc.

Die zur pharmazeutischen Anwendung erforderliche Tagesdosis beträgt 0,001 bis 25 mg/kg Körpergewicht, wenn möglich verteilt auf 2 - 3 Einzeldosen. Hiefür lassen sich die Fettsäureester von Sterolen oder die spontan dispergierbaren Konzentrate mit diesen Estern in die gängigen pharmazeutischen Zubereitungen und Darreichungsformen wie Dragées, Tabletten, Kapseln, Pulver, Granulat, Pellets, Lösungen, Ampullen, Emulsionen, Crèmes oder Zäpfchen zusammen mit den üblichen Trägerstoffen und/oder Verdünnungs- und Stabilisierungsmitteln einarbeiten.

Die Gegenstand der Erfindung bildenden Wirkstoffe oder Wirkstoffmischungen, sowie die spontan dispergierbaren Konzentrate, welche diese Wirkstoffe oder Wirkstoffmischungen enthalten, können dem Menschen oral, durch Injektion (intravenös, subkutan oder intramuskulär) oder in anderer Weise verabreicht werden. Wenn sie als feste Darreichungsformen für die orale Verwendung aufbereitet werden, kann dies in Form von Tabletten, Granulaten, Pellets, Pulvern oder Kapseln, usw. geschehen. Die Aufbereitungen können Zusatzstoffe enthalten, z.B. einen Arzneimittelträger wie eine Saccharid- oder Cellulose-Grundlage, ein Bindemittel wie Stärkepaste oder Methyl-Cellulose, ein Füllmittel oder ein Desintegriermittel, usw. - wobei Zusatzstoffe eingesetzt werden, wie sie üblicherweise bei der Herstellung medizinischer oder paramedizinischer Formulierungen verwendet werden. Wenn die erfindungs-konformen Wirkstoffe oder Wirkstoffmischungen als flüssige Darreichungsformen zu oraler Verabreichung gelangen, so können sie

irgend eine aus wässrigen Zubereitungen für innere Verwendung, aus Suspensionen, Emulsionen und Sirups usw. ausgewählte Form haben, und sie können ausserdem in der Form getrockneter Präparate vorliegen, welche vor ihrer Verwendung in Lösung oder Emulsion gebracht werden.

Wenn die erfindungsgemässen Wirkstoffe oder Wirkstoffmischungen in der Form wässriger Lösungen, Suspensionen oder öliger, bzw. wässriger Emulsionen, vorzugsweise als Mikroemulsionen aus den erfindungskonformen, spontan dispergierbaren Konzentraten aufbereitet sind, können sie auch injiziert werden. Die Injektionslösungen werden jedoch üblicherweise kurz vor der Anwendung hergestellt, indem man die Extrakte oder Konzentrate in wässrigen, flüssigen Medien wie sterilem Wasser, Glucoselösung oder physiologischer Kochsalzlösung auflöst oder suspendiert.

Falls erforderlich, können zu einem Injektionspräparat üblicherweise verwendete Lösungsmittel, Stabilisierungsmittel, Konservierungsmittel und Zusätze für die Herstellung isotonischer Lösungen hinzugegeben werden. Die auf diese Weise erhaltenen Injektions-Präparate werden intravenös, intramuskulär, subkutan oder in einer anderen geeigneten Weise verabreicht.

Die vorliegende Erfindung betrifft ebenfalls pharmazeutische Präparate, welche die Wirkstoffe, bzw. Wirkstoffgemische, und/oder die beschriebenen, spontan dispergierbaren Konzentrate zur Bekämpfung des Wachstums von Tumorzellen enthalten. Bei den der Erfindung entsprechenden pharmazeutischen Präparaten handelt es sich um solche zur enteralen (wie oralen oder rektalen), sowie zur parenteralen oder topischen Verabreichung an Warmblüter, welche das spontan dispergierbare Konzentrat allein oder zusammen mit einem pharmazeutisch anwendbaren Trägermaterial enthalten.

Die Dosierung der erfindungsgemässen Konzentrate hangt von der Warmblüterspezies, dem Alter und dem individuellen Zustand, sowie von der Verabreichungsart ab. So werden z.B. zur Erzielung eines Abtötungseffektes von Tumorzellen an Warmblütern mit geringem Körpergewicht, wie z.B. Mäusen, Ratten und Hamstern, bei sukutaner Verabreichung Dosen im Bereich von ca. 0,1 - 50 mg/kg Körpergewicht, und bei intraperitonealer Verabreichung Dosen im Bereich von 0,05 - 5 mg/kg Körpergewicht angewandt.

Die oralen und rektalen Formen der neuen pharmazeutischen Präparate enthalten zwischen 1 - 95 %, vorzugsweise zwischen 10 - 95 %, insbesondere zwischen 20 - 95 % des erfindungsgemässen, spontan dispergierbaren Konzentrates. Sie können z.B. in Dosis-Einheitsform vorliegen, also als Dragées, Micropellets, Tabletten, Suppositorien oder Ampullen und vor allem als Kapseln.

Geeignete pharmazeutisch anwendbare Trägerstoffe für die oralen Formen sind hauptsächlich Füllstoffe, wie Zucker (z.B. Lactose, Saccharose, Mannit oder Sorbit), Cellulosepräparate und/oder Calciumphosphate (z.B. Tricalcium- oder Calciumhydrogenphosphat), ferner Bindemittel wie Stärkekleister unter Verwendung von u.a. Mais-, Weizen-, Reis- oder Kartoffelstärke, Gelatine, Traganth, Methylcellulose, Hydroxyl-Methylcellulose, Natriumcarboxymethylcellulose und/oder Polyvinylpyrrolidon und/oder Sprengmittel (wenn erwünscht), wie die obgenannten Stärken, ferner Carboxy-methylstärke, quervernetztes Polyvinylpyrrolidon, Agar, Alginsäure oder ein Salz davon, wie z.B. Natriumalginat.

Als Fliessreguliermittel sind z.B. die Polyäthylenglykole Nr. 200 - 600 und höher geeignet.

Die beim Menschen als Darreichungsform bevorzugten Gelatinekapseln werden mit geeigneten Ueberzügen versehen, wobei man u.a. konzentrierte Zuckerlösungen - welche gegebenenfalls arabischen Gummi, Talk, Polyvinylpyrrolidon, Polyäthylenglykol und/oder Titandioxid enthalten - Lacklösungen (wässrige oder solche, die unter Verwendung organischer Lösungsmittel aufbereitet worden sind), oder magensaft-resistente Ueberzüge aus Lösungen von geeigneten Cellulose-präparaten, wie mikrokristalliner Cellulose (Avicel), Acetylcellulosephthalat, Hydroxymethylcellulose-phthalat oder einem Co-polymerisat wie Eudragit® L 30 D verwendet.

Als erfindungsgemäss besonders geeignete, oral anwendbare pharmazeutische Darreichungsform eignen sich Steck-Kapseln aus Gelatine und einem Weichmacher, wie Glyzerin oder Sorbitol. Die Weich- bzw. Hartgelatine-Kapseln können das erfindungsgemässe, spontan dispergierbare Konzentrat im Gemisch mit Füllstoffen, wie Laktose, Bindemitteln wie Stärke und/ oder Gleitmitteln wie Talk oder Magnesium-Stearat und gegebenenfalls mit Stabilisatoren und Anti-oxydantien wie z.B. $\alpha$-Tocopherol enthalten. Der Einsatz von geeigneten Flüssigkeiten wie flüssigen Polyäthylenglykolen No. 200 - 600 als Verdünnungsmittel kann sich empfehlen, wobei sich ebenfalls Stabilisatoren und Antioxydantien zufügen lassen.

Zur parenteralen Verabreichung werden die erfindungskonformen Konzentrate mit destilliertem Wasser versetzt. Der entstehenden wässrigen Injektions-Mikroemulsion können viskositätserhöhende Stoffe, wie z.B. Na-Carboxymethylcellulose, Sorbit, Mannit und/oder Dextran, und gegebenenfalls auch Stabilisatoren und Antioxydantien zugefügt werden.

Die pharmazeutischen Präparate für die parenterale Anwendung enthalten vorzugsweise zwischen 0,1 bis 60 %, vorab zwischen 1 bis 40 % des erfindungsgemässen, spontan dispergierbaren Konzentrates.

Als topisch anwendbare Präparate, welche sich vornehmlich zur Prophylaxe von Hautkrebsarten eignen, kommen z.B. Crèmen, Salben, Pasten, Schäume, Tinkturen und Lösungen in Betracht, welche zwischen

0,01 bis 70 % des erfindungsgemässen Konzentrates enthalten.

Für Crèmen und Oel-in-Wasser-Emulsionen, welche mehr als 50 % Wasser aufweisen, verwendet man als oelige Grundlage in erster Linie Fettalkohole, z.B. Lauryl-, Cetyl- oder Stearylalkohol, flüssige bis feste Wachse, z.B. Isopropylmyristat, Woll- oder Bienenwachs und/oder Kohlenwasserstoffe wie z.B. Vaseline (Petrolatum) oder Paraffinoel. Zur Emulgierung dieser öligen Grundlagen eignen sich in erster Linie oberflächenaktive, pharmaverträgliche Substanzen mit vorwiegend hydrophilen Eigenschaften, wie z.B. nicht-ionogene Emulgatoren, vorab Fettsäureester von Polyalkoholen oder Aethylen-oxydaddukten (etwa Polyglycerinfettsäureester oder Polyäthylensorbitan-Fettsäureester) mit einem HLB-Wert von unter 8. Zusätze zur Wasserphase sind u.a. Mittel, die die Austrocknung der Crèmen vermindern, z.B. Polyalkohole wie Glyzerin, Sorbit, Propylenglykol und/oder Polyäthylenglykole No. 200 - 600, ferner Konservierungsmittel, Riechstoffe, etc.

Salben sind Wasser-in-Oel Emulsionen, die bis zu 70 %, vorzugsweise jedoch zwischen 20 und 50 % Wasser oder wässrige Phasen enthalten.

Als Fettphase kommen in erster Linie Kohlenwasserstoffe, z.B. Vaselin, Paraffinoel und/oder Hartparaffine in Frage, welche zur Verbesserung des Wasserbindungsvermögens geeignete Hydroxydverbindungen, wie z.B. Fettalkohole oder Ester, davon etwa Cetylalkohol oder Wollwachsalkohole, enthalten.

Fallweise werden noch Emulgatoren mit einem HLB-Wert von 8 bis 16, wie z.B. Sorbitan-Fettsäureester (etwa Sorbitanisostearol) zugesetzt. Zusätze zur Wasserphase sind u.a. Feuchthaltungsmittel, wie Polyalkohole (Glycerin, Propylenglykol, Sorbit und/oder Polyäthylenglykole No. 200, 400, 600); ferner Konservierungsmittel, Riechstoffe, etc.

Fettsalben sind wasserfrei und enthalten als Grundlage vornehmlich Kohlenwasserstoffe, z.B. Paraffin, Vaselin und/oder flüssige Paraffine; überdies natürliche oder partial synthetische Fette wie z.B. Kokosfettsäuretriglycerid, ferner: Fettsäurepartialester des Glycerins, wie z.B. die im Zusammenhang mit den Salben erwähnten, die Wasseraufnahmefähigkeit steigernden Fettalkohole, Emulgatoren und/oder Zusätze.

Pasten sind Crèmen und Salben mit sekretabsorbierenden Puderbestandteilen, wie beispielsweise Metalloxide (etwa Titanoxid oder Zinkoxid), ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende Oel-in-Wasser Emulsionen der erfindungsgemässen, spontan dispergierbaren Konzentrate, wobei halogenierte Kohlenwasserstoffe (wie z.B. Chlorfluorniederalkane: etwa Dichlordifluormethan und Dichlortetrafluoräthan) als Treibmittel verwendet werden. Dazu kommen gegebenenfalls die üblichen Zusätze wie Konservierungsmittel, usw.

## Verfahrensbeispiele zur Herstellung der Sterolester und Sterolphosphatide

### 1. Herstellung von Ergosta-5,7,22-trien-3-ol-dodecenoat *

2 g Dodecenylsäure und 1,8 g Thionylchlorid werden in 300 ml Tetrahydrofuran während 2 Stunden am Rückfluss gekocht. Anschliessend werden 150 ml Tetrahydrofuran abdestilliert und 3,97 g Ergosterol (= Ergosta-5,7,22,trien-3-ol) und 0,5 g Dimethylformamid zugesetzt.
Nach zweistündiger Erhitzung bei 70 °C wird das Lösungsmittel durch Vakuumdestillation entfernt. Der Rückstand wird in Acetonitril/Aceton (50/50) umkristallisiert.
Man erhält das reine Ergosteroldodecenoat mit einem Schmelzpunkt von 86,6 - 91,1 °C.

Auf gleichartige Weise werden auch folgende Verbindungen hergestellt:

* diese Verbindungen sind nicht beansprucht

| | |
|---|---|
| Ergosterolcrotonat | Smp: 150 - 151,6 °C |
| Ergosterolcapryloat * | Smp: 97,9 °C |
| Ergosterolundecenoat | Smp: 75,8 - 76,2 °C |
| Ergosterollaurat * | Smp: 86,5 °C |
| Ergosterolpalmitat * | Smp: 99,5 °C |
| Ergosterololeat * | Smp: 86,3 - 95,7 °C |
| Ergosterollinoleat * | Smp: 89,8 - 91,2 °C |
| | UV: 253,0 nm |

* diese Verbindungen sind nicht beansprucht

BI (Brechungsindex) = RI (Refractory Index), gemessen am DUR-Refractometer Schmidt + Haensch, Berlin.

2. Herstellung von Stigmasterol-all-trans-Retinat

Zu 600 mg all-trans-Retinsäure und 50 mg Dimethylformamid in 70 ml Toluol werden bei 5 °C 360 mg Oxalylchlorid in 30 ml Toluol zugetropft. Nach 4 Std. bei 20 °C wird die Hälfte des Lösungsmittels am Vakuum abdestilliert.

Zur Restlösung werden 650 mg Stigmasterol und 50 mg p-Dimethylaminopyridin in 30 ml Toluol zugegeben. Die Reaktionslösung wird während 2 Std. am Rückfluss bei 100 - 110 °C erwärmt. Anschliessend wird das Lösungsmittel durch Vakuumdestillation entfernt. Der Rückstand wird auf einer Silicagelsäule mit Hexan/Essigester (9:1) chromatographiert.

Man erhält das reine Stigmasterol-all-trans-Retinat mit einem Schmelzpunkt von 89 °C, UV-Absorption 358,5 nm.

Auf gleichartige Weise werden auch folgende Verbindungen hergestellt, welche die Eigenschaften von Fettkristallen haben, d.h. dass der Schmelzbereich meist unbestimmbar ist:

| | |
|---|---|
| Ergosterol-all-trans-Retinat | UV: 354,0/264,0 nm |
| β-Sitosterol-all-trans-Retinat | UV: 344,5 nm |
| Cholesterol-all-trans-Retinat * | UV: 354,5 nm |
| Stigmasterol-13-cis-Retinat | UV: 348,0 nm |
| Ergosterol-13-cis-Retinat | UV: 346,0 nm |
| β-Sitosterol-13-cis-Retinat | UV: 368,5 nm |
| Cholesterol-13-cis-Retinat | UV: 345,5 nm |
| Ergosterol-Linolenat * | UV: 252,2 nm |
| Stigmasterol-Arachidonat * | UV: 251,8 nm |
| | BI: 1.51320 / 20 °C |

* diese Verbindungen sind nicht beansprucht

NMR-analytische Daten zu Ergosterol-all trans-Retinat gibt Zeichnung 1/3 wieder.

3. Herstellung von Stigmasterol-Azafrinat

Zu 80 mg Azafrin [Verbindung wie Formel XXI; Herstellung vgl. Helv.Chim.Acta 58 (1975) 1722-1727 und Helv.Chim.Acta 65 (1982) 353-354] in 50 ml Chloroform werden 65 mg N,N'-Carbonyldiimidazol zugesetzt. Man lässt die Reaktionslösung während 12 Std. bei 20 °C stehen und setzt dann 40 mg Stigmasterol zu.

Nach weiteren 12 Std. bei 30 °C wird das Lösungsmittel abdestilliert und der Rückstand in 50 ml Essigester aufgenommen. Diese Lösung schüttelt man einmal mit 1/10 N Salzsäure und einmal mit 1/10 N Natronlauge aus. Nach dem Abdestillieren des Lösungsmittels wird der Rückstand auf einer Silicagelsäule mit Hexan/Essigester (9:1) als Eluiermittel chromatographiert. Man erhält das Stigmasterol-Azafrinat, mit einer UV-Absorption bei 429,5 nm.

Auf gleichartige Weise werden auch folgende Verbindungen hergestellt, charakterisiert durch den Brechungsindex $n_D$ 20 °C (BI), bzw. die UV-Absorptions-Maxima :

| | |
|---|---|
| Ergosterol-Azafrinat | UV: 425,6 nm |
| β-Oestradiol-3,17-Diundecenoat | BI 1,4816<br>UV 232,8/276,0 nm |
| β-Oestradiol-3,17-Dioleat | BI 1,5058<br>UV 232,0/280,0 nm |
| β-Oestradiol-3,17-Diretinat | UV 354,5/371,0 nm |
| β-Oestradiol-3-Benzoat-17-Undecenoat | UV 247,6 nm |
| β-Oestradiol-3-Benzoat-17-Retinat | UV 354,5 nm |

4. Herstellung von Stigmasterol-1,2-dipalmitoyl-glycerophosphat und -thiophosphat

1,2-Dipalmitoyl-glycero-3-H-phosphonat-Triaethylammoniumsalz wird genau nach der Vorschrift von I. Lindth und J. Stawinski in J.Org.Chem. 54, 1338-1342 (1989) ["Synthesis of Glycerophospholipids and their Analogues via H-Phosphonate Intermediates"] hergestellt. Auf die chromatographische Zwischenreinigung des Reaktionsproduktes wird verzichtet; das Rohprodukt wird demnach unmittelbar in der nächsten Stufe eingesetzt.

600 mg Rohprodukt und 600 mg Stigmasterol werden in 15 ml Pyridin gelöst und im Vakuum zur Trockene gebracht. Der Rückstand wird erneut in 15 ml Pyridin gelöst, und unter Rühren werden 0,2 ml Pivaloylchlorid zugegeben. Die Reaktionslösung wird unter Feuchtigkeitsausschluss bei Raumtemperatur 30 Min. gerührt.

Dann werden noch 0,1 ml Pivaloylchlorid zugefügt, und anschliessend wird nochmals 30 Min. gerührt.

Die Reaktionslösung wird in zwei Teile geteilt:
Zur einen Hälfte der Lösung werden unter Rühren 150 mg Jod in 2 ml Pyridin-Wasser 98:2 gegeben. Nach 30 Min. Rühren bei 20 °C wird das Reaktionsgemisch in 50 ml Chloroform aufgenommen. Die Lösung wird mit 20 ml 5% Na-Bisulfitlösung und zweimal mit 20 ml Wasser gewaschen. Die wässrigen Phasen werden mit 50 ml Chloroform zurückgewaschen und die vereinigten Chloroform-phasen im Vakuum zur Trockene eingedampft.

Zur anderen Hälfte der Lösung werden 150 mg Schwefel in 2 ml Pyridin-Toluol 1:1 gegeben. Die Lösung wird 3 Std. bei 20 °C gerührt, in 50 ml Chloroform gelöst, zweimal mit 20 ml Wasser gewaschen und im Vakuum zur Trockene eingedampft.

Die beiden Rohprodukte werden an je 15 g Silicagel mit Chloroform/Hexan 2:1 und 8:1 chromatographiert. Es werden Fraktionen zu 20 ml aufgefangen und im D.C. (Laufmittel Chloroform) untersucht. Die Fraktionen, welche im wesentlichen reines Produkt enthalten, werden im Vakuum zur Trockene gebracht.

Man erhält:
das Stigmasterol-1,2-dipalmitoyl-glycerphosphatid mit einem Schmelzpunkt von 171,6 °C und
das Stigmasterol-1,2-dipalmitoyl-glycero-thiophosphatid mit einem Schmelzpunkt von 168 °C.

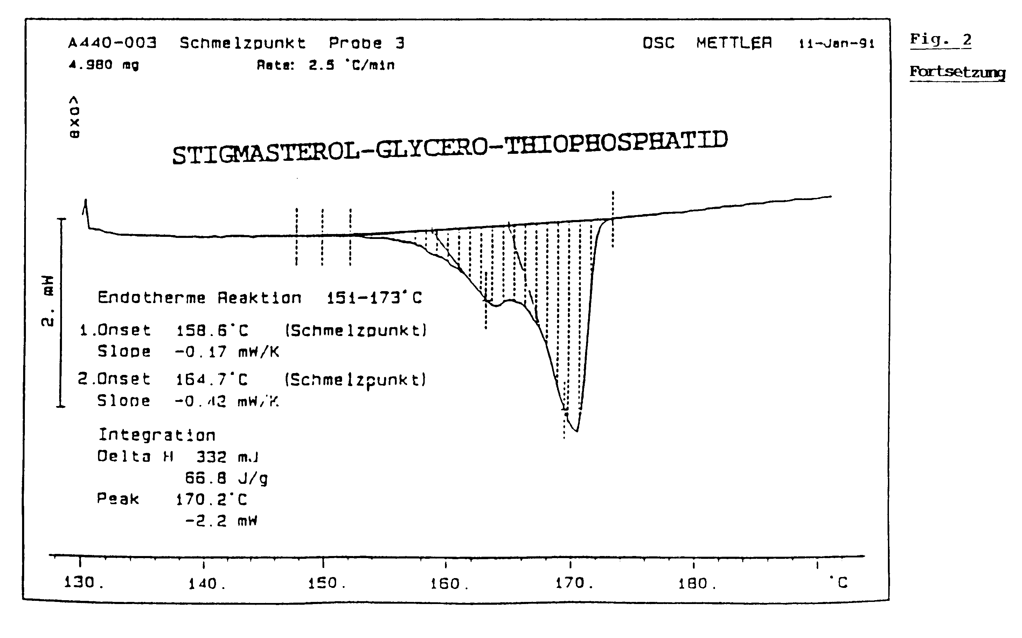

Die Zeichnung 2/2 zeigt den Schmelzbereich für die beiden Verbindungen auf. Vgl. die technische Beilage.

5. Verfahren zur Herstellung von Ergosterol-3-Retinolphosphonat

a) Verfahren zur Herstellung von Ergosterol-3-Hydrogenphosphonat
Eine Lösung von 3,6 g Imidazol in 100 ml wasserfreiem Acetonitril wird unter Feuchtigkeitsabschluss im Eiswasserbad gekühlt. Unter Rühren werden innert 10 Min. 1,4 ml Phosphortrichlorid und anschliessend innert 15 Min. 7,8 ml Triäthylamin zugetropft. Das Gemisch wird während 15 Min. gerührt; dann gibt man unter weiterer Eiskühlung über 30 Min. eine Lösung von 1,5 g Ergosterol in 100 ml Acetonitril zu.
Das Eisbad wird entfernt und das Gemisch 4 Std. lang bei Zimmertemperatur gerührt. Dann werden 25 ml Wasser zugesetzt und noch 30 Min. gerührt.
Das Reaktionsgemisch wird im Vakuum zur Trockene eingedampft; zum Rückstand fügt man 100 ml Pyridin-Triäthylamin 4:1 zu und dampft weiter ein.
Der Rückstand wird in 100 ml Chloroform gelöst, die Lösung 3 Mal mit 50 ml Wasser gewaschen, im Vakuum zur Trockene eingedampft und im Vakuumtrockenschrank bei 50 °C bis zur Gewichtskonstanz getrocknet. Das Produkt: 3,0 g, wird ohne weitere Reinigung für die folgenden Reaktionsschritte verwendet.
b) Ergosterol-3-Retinol-Phosphonat.
1,2 g rohes Ergosterol-Hydrogenphosphonat und 0,8 g Retinol (Vitamin-A-Alkohol) werden in 15 ml

Pyridin gelöst. Unter Rühren werden 0,3 ml Pivaloylchlorid zugetropft, 5 Min. gerührt, nochmals 0,3 ml Pivaloylchlorid zugefügt und weitere 10 Min gerührt.
Zum Reaktionsgemisch werden 25 ml Wasser und 5 ml gesättigte Na-Bicarbonatlösung zugefügt und das Ganze mit 50 ml Chloroform extrahiert.
Die Chloroformphase wird abgetrennt, 2 Mal mit je 25 ml Wasser gewaschen und im Vakuum zur Trockene gebracht. Man erhält das ERGOSTEROL-3-RETINOL-PHOSPHONAT.
Rf.-Wert in Hexan/Essigester 80:20 : 0,97.
Auf gleiche Weise lassen sich herstellen:
ERGOSTEROL-3-GERANYL-PHOSPHONAT
ERGOSTEROL-3-FARNESYL-PHOSPHONAT

6. Herstellung von Ergosterol-3-Geranylphosphothioat.

1,2 g rohes Ergosterol-Hydrogenphosphonat (vgl. 5 a) und 0,5 ml Geraniol werden in 15 ml Pyridin gelöst. Unter Rühren werden 0,3 ml Pivaloylchlorid zugetropft, 5 Min. gerührt, nochmals 0,3 ml Pivaloylchlorid zugetropft und weitere 10 Min. gerührt. Zum Reaktionsgemisch werden 0,1 g Schwefel in 2 ml Toluol-Pyridin 1:1 gefügt und das Ganze 2 Std. bei Raumtemperatur gerührt.
Das Reaktionsgemisch wird in 50 ml Chloroform gelöst, 2 Mal mit je 25 ml Wasser gewaschen und im Vakuum zur Trockene eingedampft. Der Rückstand wird an 50 g Silicagel mit Hexan-Essigester 9:1, 8:2 und 5:5 chromatographiert (je 200 ml). Man fängt Fraktionen zu 25 ml auf, die mittels Dünnschicht-Chromatographie (Laufmittel Hexan/Essigester 8:2) analysiert werden.
Jene Fraktionen, welche im Wesentlichen das reine Produkt enthielten, wurden vereinigt, im Vakuum zur Trockene eingedampft und im Vakuumtrockenschrank bei 50 °C zur Gewichtskonstanz getrocknet.
Man erhält 0,45 g ERGOSTEROL-3-GERANYL-PHOSPHOTHIOAT
Rf.-Wert Hexan/Essigester 80:20 : 0.98
Auf gleiche Weise lässt sich das
ERGOSTEROL-3-FARNESYL-PHOSPHOTHIOAT
herstellen.

Rf.-Werte von erfindungsgemässen Verbindungen, welche nach den Verfahrensbeispielen 1 bis 4 hergestellt worden sind:

1 %-Lösung in $CH_2Cl_2$, bandförmig aufgetragen 2 cm/2µl
Linomat III CAMAC 10cm run
UV 366 nach 1:1 $H_2SO_4$/MeOH 2 min. 120 °C

| SYSTEM / VERBINDUNG | 1 | 2 | 3 | 4 | 5 | 6 | 7 |
|---|---|---|---|---|---|---|---|
| C 8:0 ERGO | -- | 0,17 | 0,32 | 0,77 | -- | 0,81 | -- |
| C 12:0 ERGO | 0,06 | 0,14 | 0,29 | 0,73 | 0,89 | 0,74 | 0,57 |
| C 16:0 ERGO | 0,07 | 0,16 | 0,32 | 0,75 | 0,92 | 0,82 | 0,58 |
| C 18:1 ERGO | 0,08 | 0,18 | 0,35 | 0,80 | 0,92 | -- | 0,63 |
| ERGO-RETINAT | 0,07 | 0,17 | 0,38 | 0,00 | 0,91 | 0,87 | 0,65 |
| | 0,11 | 0,23 | 0,42 | -- | -- | -- | 0,73 |
| C 11:1 β-Oestradiol-doppelester | 0,00 | 0,00 | 0,04 | 0,18 | 0,71 | 0,29 | 0,07 |
| | -- | -- | -- | 0,56 | 0,81 | -- | 0,41 |

Erklärung:

System 1 Platte Merck Art. 5715 Petrolaether/Diethylaether 98:2
System 2 Platte Merck Art. 5715 Petrolaether/Diethylaether 97:3
System 3 Platte Merck Art. 5715 Cyclohexan/Ethylacetat 97:3
System 4 Platte Macherey Nagel RP 18 Art. 811'071 Petrolaether/Diethylaether 95:5
System 5 Platte n Hexan/t.Butylmethylaether/Aceton 90:5:5
System 6 Platte Petrolaether/Cyclohexan/Ethylacetat/$H_2O$ 48:48:3:1
System 7 Platte Petrolaether/Diethylaether 97:3

Rf.-**WERTE** FORTSETZUNG

| System / Verbindung | 1 | 2 | 3 | 4 | 5 |
|---|---|---|---|---|---|
| C 18:2 ERGO | 0,76<br>0,59 | 0,39<br>0,25-0,50 | 0,60<br>0,50 | 0,92 | 0,79<br>0,60-0,61 |
| C 18:3 ERGO | 0,78<br>0,59-0,61 | 0,40<br>0,25 | 0,65<br>0,50-0,55 | 0,92 | 0,80<br>0,60-0,65 |
| β-Oestradiol-3,17-DI-UNDECENOAT | 0,38 | 0,10 | 0,15 | 0,75 | 0,30 |
| β-Oestradiol-3.17-DI-OLEAT | 0,42 | 0,22 | 0,20 | 0,83 | 0,38 |

ERKLÄRUNG:

SYSTEM 1 Platte RP 18 MN Petrolaether/Cyclohexan/EtAc/$H_2O$ 48:48:3:1
SYSTEM 2 Platte SM Cyclohexan/Ethylacetat 97:3
SYSTEM 3 Platte SM Petrolaether/Diaethyleather 97:3
SYSTEM 4 Platte RP 18w MN Hexan/t.BME/Acetonitril 90:5:5
SYSTEM 5 Platte RP 18w MN Petrolaether/Diaethylaether 95:5
N-B.: SM Platte Merck Art. No. 5715
RP18 MN(w) Art. Macherey Nagel Nr. 811'071

Rf.-Werte mit Normalchromatographie Hexan/Essigester 90:10 :

| | |
|---|---|
| β-Oestradiol-Diundecenoat | 0,37 |
| β-Oestradiol-Dioleat | 0,48 |
| Ergosterol-all trans-Retinat | 0,88 |
| Ergosterol-Linoleat | 0,64 / 0,82 |
| Ergosterol-Linolenat | 0,64 / 0,96 |
| Stigmasterol-Glycero-Phosphatid | 0,88 |
| Stigmasterol-Glycero-Thiophosphatid | 0,94 |

Platte Merck Art. No. 5715

Zusammensetzungsbeispiele von erfindungsgemässen, spontan dispergierbaren Mitteln, welche als antitumorale Wirkstoffe Sterolester und/oder Sterolphosphorverbindungen gemäss den Formeln I bis XXX enthalten.

a) 7,5 bis 25 Gewichts-% eines oder mehrerer Sterolester und/oder einer Sterolphosphorverbindung der Formeln I bis XV
0 bis 40 Gewichts-% Isopropylmyristat, Isopropylpalmitat oder Miglyol® 812 (Dynamit-Nobel)
22,5 bis 40 Gewichts-% Diphasol® 3873 (CIBA-GEIGY)
22,5 bis 40 Gewichts-% Invadin® JFC 800 % (CIBA-GEIGY)
b) 7,5 bis 25 Gewichts-% eines oder mehrerer Sterolester und/oder einer Sterolphosphorverbindung der Formeln I bis XV
0 bis 40 Gewichts-% Isopropylmyristat, Isopropylpalmitat oder Miglyol® 812 (Dynamit Nobel)
22,5 bis 40 Gewichts-% Invadin® JFC 800% (CIBA-GEIGY)
22,5 bis 40 Gewichts-% Soprophor® FL (Rhône-Poulenc)

Miglyol® 812-Neutralöl ist ein Neutralöl (Oleum neutrale) der Firma Dynamit-Nobel, das einem gemischtsäurigen Triglycerid der fraktionierten Kokosfettsäuren $C_8$ bis $C_{10}$ entspricht.Diphasol® 3873 ist ein Mischemulgator, bestehend aus je 50 % der beiden Verbindungen mit den Formeln:

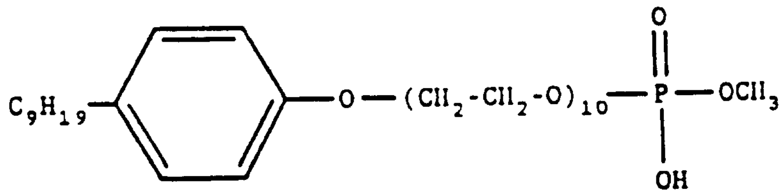

und

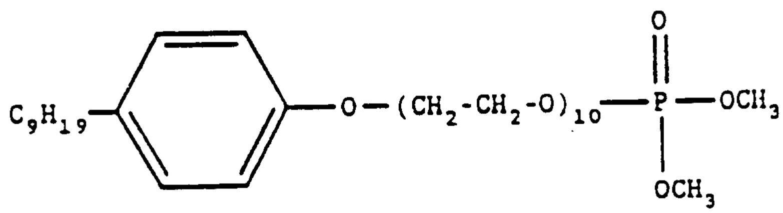

Invadin® JFC 800% (CIBA-GEIGY) ist ein tert. Octylphenylpolyoxyäthylenaether mit 9 bis 10 Oxyaethylengruppen.

Soprophor® FL (Rhône-Poulenc) ist ein Tristyrylphenol-polyoxyäthylen-18-mono/dimethyl-Phosphorsäureester.

Zeichnungen:

1/3 : NMR-Messdaten für ERGOSTEROL-all trans-RETINAT
2/3 : DSC-Messdaten für die Stigmasterol-Phosphatide.
3/3 : DSC-Messdaten für die Stigmasterol-Phosphatide.

Biologische Prüfungen.

Die antitumorale Wirkung von spontan dispergierbaren Konzentraten mit Wirkstoffen gemäss den Aufarbeitungsbeispielen No. 1 - 6 wird anhand folgender Prüfungsergebnisse bestätigt:

In vitro-Tests mit geeigneten Tumorzell-Linien

Es wurde ein biologisches Assay-System entwickelt, das mit Mikrotiterplatten und Verdünnungsreihen arbeitet. Angesetzt werden je $10^4$/ml Tumorzellen in Kulturmedium RPMI 1640 mit 10 % fötalem Kalbserum inaktiviert (GIBCO); sie werden so undicht ausgesät, dass sie während des Assays wachsen können, in sog. nichtkonfluenten Monolayers. Die Probenzugabe erfolgt nach 6 - 24 Stunden, mit 100 µl pro Reihe, die man im 1. Loch mit 100 µl Medium versetzt. Davon wird die Hälfte entnommen und in das folgende Loch eingebracht, wieder mit 100 µl Medium versetzt, usf. Es entsteht eine geometrische Verdünnungsreihe $n\frac{1}{2}$.

Die Proben werden im Plaque Assay während 3-5 Tagen bei 37° C mit $3\frac{1}{2}$ % $CO_2$ inkubiert. Anschliessend färben/fixieren mit 0,1 % Kristallviolett (Fluka, Buchs) in einer Lösung von 70 % Methanol, 1 % Formaldehyd, 29 % Wasser. Die Auswertung wird am Mikroskop vorgenommen, Vergrösserung 300-fach. Man bestimmt die grösste cytotoxische Verdünnung. Die quantitative Auswer- tung lässt sich auch mittels Scanning und Absorptionsmessung am Spektrophotometer vornehmen.

AUSWERTUNG DER ERGEBNISSE

| Tumorlinie / Präparat | TSA (Murines Adenocarcinom) In Verdünnung wirksam bis 1 : | |
|---|---|---|
| | 16 h | 40 h |
| C 18:2 ERGOSTEROL | 2'000'000 | 16'000'000 |
| STIGMA-AZAFRINAT | 28'600'000 | 28'600'000 |
| ERGOSTEROL-all trans-RETINAT | 800'000 | 1'600'000 |
| STIGMASTEROL-GLYCERO-PHOSPHATID | 400'000 | 1'600'000 |
| STIGMASTEROL-GLYCERO-THIOPHOSPHATID | 10'000'000 | 20'000'000 |
| β-OESTRADIOL-3,17-OLEAT | 20'000'000 | 40'000'000 |

T S A : murines Adenocarcinom (spontanes Mamacarcinom)
Prof. Guido Forni, Istituto di Microbiologia,
Università di Torino, Scuola di Medicina

Die Ueberprüfung der Wirkung dieser spontan emulgierbaren Konzentrate mit einem Gehalt von 1000 ppm C 18:2-ERGOSTEROL-Wirkstoff an humanen Leucocyten ergab folgende Werte:

| Verdünnung | 24 h | 4 Tage | 5 Tage |
|---|---|---|---|
| 1 : 20 | alle Zellen tot | alle Z. lysiert | -- |
| 1 : 80 | alle Zellen tot | alle Z. lysiert | -- |
| 1 : 200 | RBC lysiert Leucocyten i.O. | RBC lysiert Leucocyten lysiert | -- |
| 1 : 400 | RBC lysiert Leucocyten i.O. | alle Zellen lysiert | -- |
| 1: 1'000 | alle Z. i.O. | Leucocyten i.O. | Leucocyten i.O. |
| 1: 5'000 | alle Z. i.O. | Leucocyten i.O. | Leucocyten i.O. |
| 1:20'000 | alle Z. i.O. | Leucocyten i.O. | Leucocyten i.O. |
| N.B.: RBC steht für "Red blood cells", Erythrocyten, rote Blutkörperchen. | | | |

Leberschranke

Die Prüfung wurde an der perfundierten, in situ belassenen Rattenleber vorgenommen. Eingesetzt wurden die beiden Präparate Ergosterol-Undecenoat und Ergosterol-all trans-Retinat und zwar in Form von 2%-Konzentraten, verdünnt 1:10, 1:100 und 1:1000. Die Arbeiten wurden in verdankenswerter Weise vom Institut für klinische Pharmakologie der Universität Bern (Prof. Dr. R. Preisig) durchgeführt. Die analytische Auswertung geschah mittels Kapillar-Electrophorese (20 kV konstant) mit einem Gerät von Beckman Instruments (P/ACE System 2000 Version 1.50).

Ergebnis: Die Wirksubstanzen passieren, auch in der höchsten geprüften Konzentration, innerhalb von je 20 Minuten die hepatische Barrière vollständig.

## Patentansprüche

1. Spontan dispergierbares Konzentrat, das mit Wasser versetzt Mikroemulsionen ergibt, enthaltend als Antitumorkomponente
7,5 bis 25 Gewichts-% eines Sterolesters, bzw. Sterolphosphatids der Formeln (I) bis (XV), bzw. einer Kombination solcher Komponenten

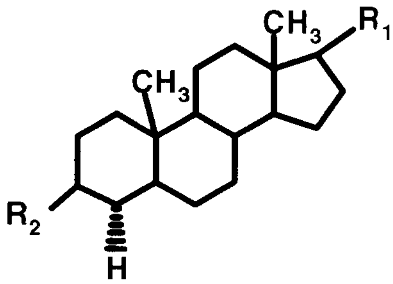

(I)

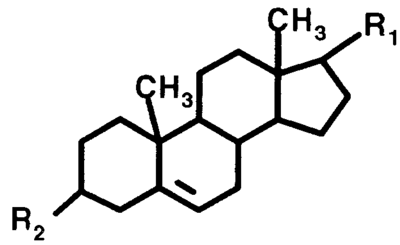

(II)

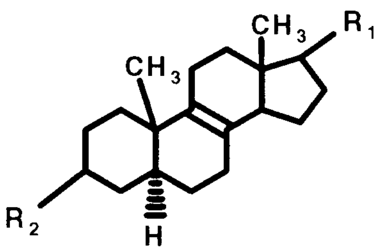

(III)

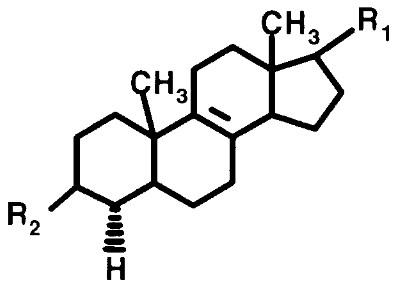

(IV)

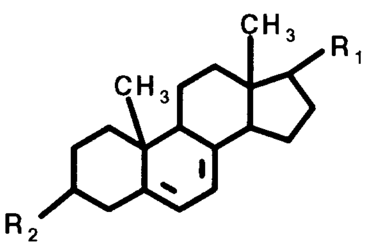

(V)

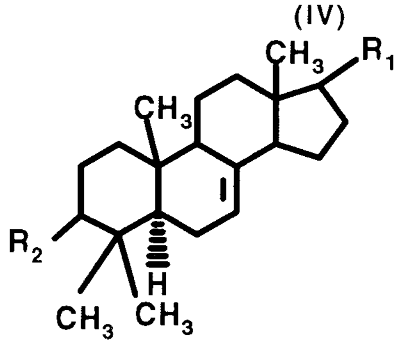

(VI)

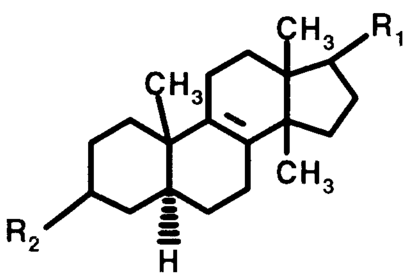

(VII)

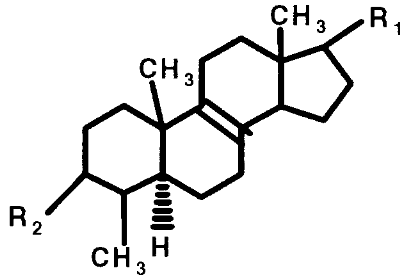

(VIII)

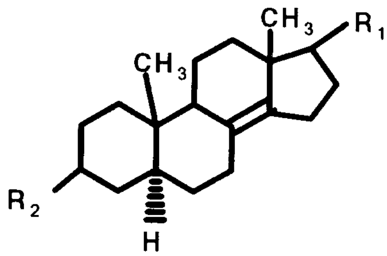

(IX)

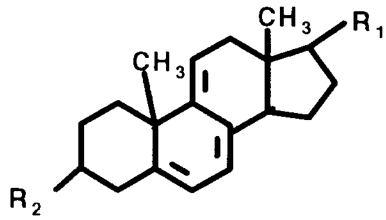

(X)

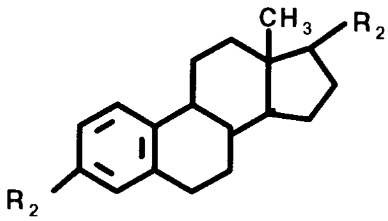

(XI)

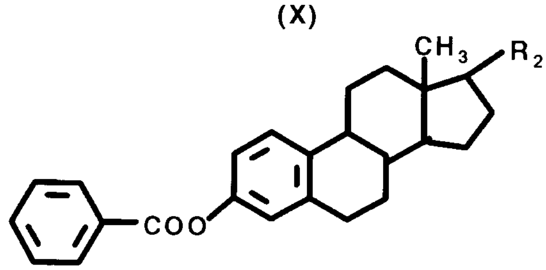

(XII)

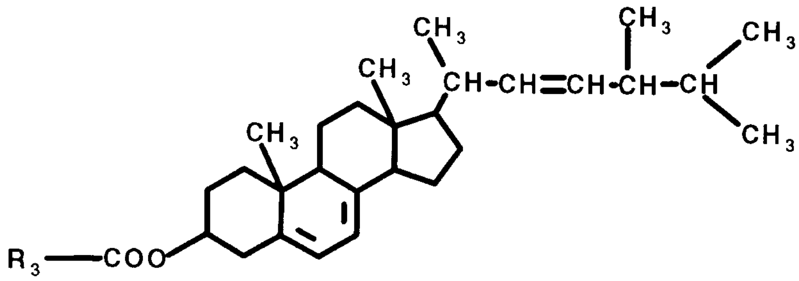

(XIII)

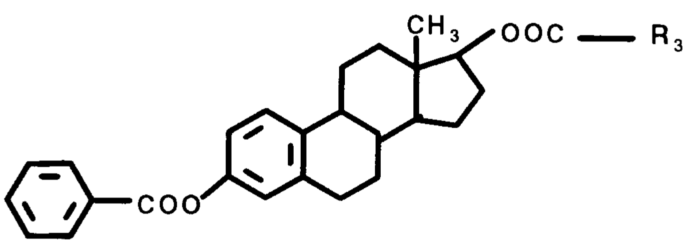

(XIV)

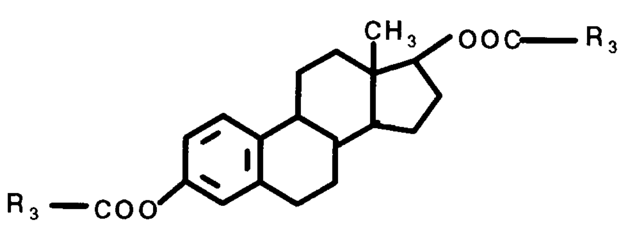

(XV)

wobei in den Formeln (I) bis (X) das Radikal $R_1$ eine $C_1$- bis $C_{10}$-Alkyl- oder eine $C_2$- bis $C_{10}$-Alkenylgruppe und das Radikal $R_2$ in den Formeln (I) bis (XII) eine Gruppe der Formeln (XVI) oder (XVII) ist

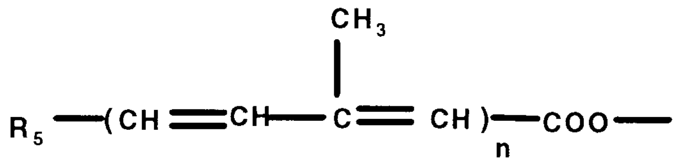

(XVI)

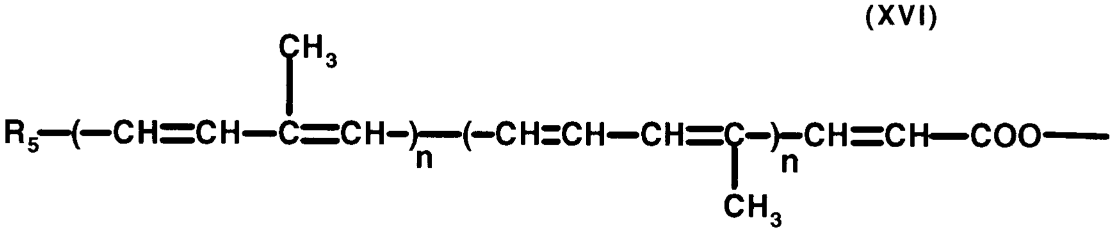

(XVII)

worin n die Zahlen 1, 2, 3, 4 oder 5 bezeichnet und $R_5$ für ein Radikal der Formeln:

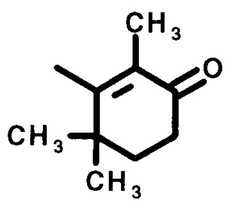
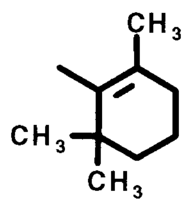
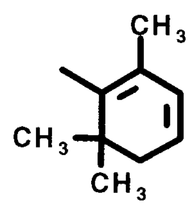
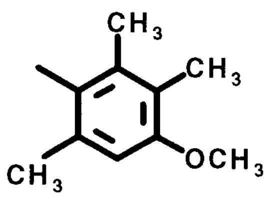
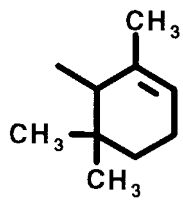
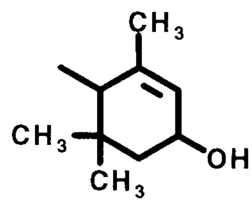
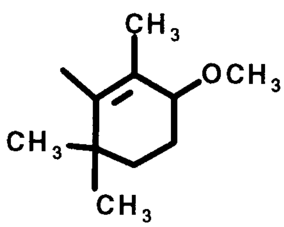
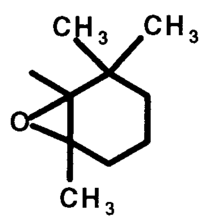
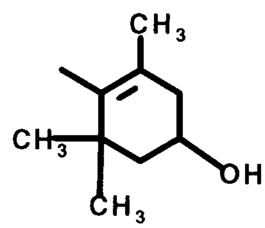
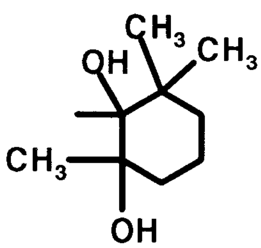
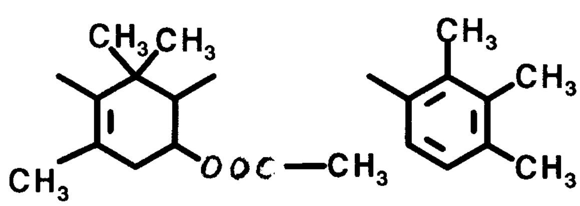

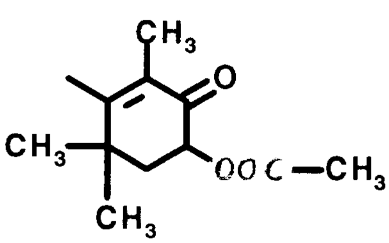 oder 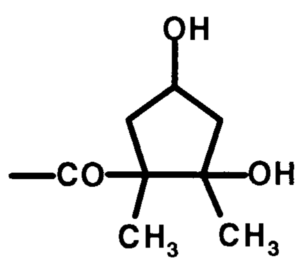

steht oder aber die Gruppe gemäss der Formel (XVIII) darstellt

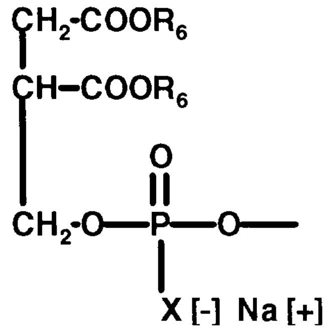

(XVIII)

in welcher $R_6$ eine $C_1$- bis $C_{32}$-Alkyl- oder eine $C_2$- bis $C_{32}$-Alkenyl- bzw. Alkapolyengruppe und X Sauerstoff oder Schwefel bedeuten, und wobei $R_3$ in den Formeln (XIII) bis (XV) für eine $C_4$- bis $C_{32}$-Alkylgruppe oder eine $C_4$- bis $C_{32}$- Alkenyl- bzw. Alkapolyengruppe steht
0 bis 40 Gewichts-% Isopropylmyristat, Isopropylpalmitat oder Neutralöl
und 22,5 bis 40 Gewichts-% des tert. Octylphenylpolyoxyäthylenäthers mit
9 bis 10 Oxyäthylengruppen, sowie
22,5 bis 40 Gewichts-% eines Mischemulgators bestehend aus je 50 % der beiden Verbindungen der Formeln

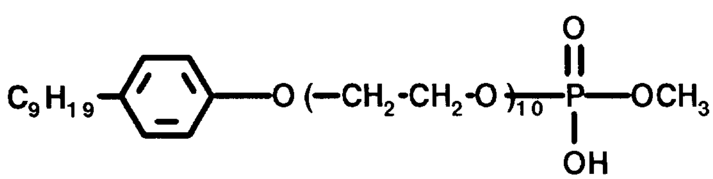

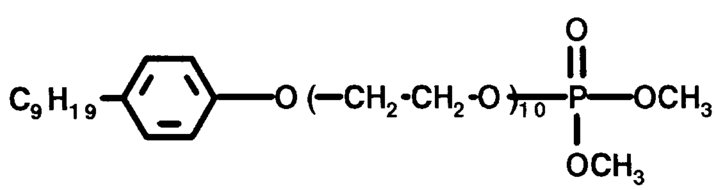

oder eines Emulgators der Formel

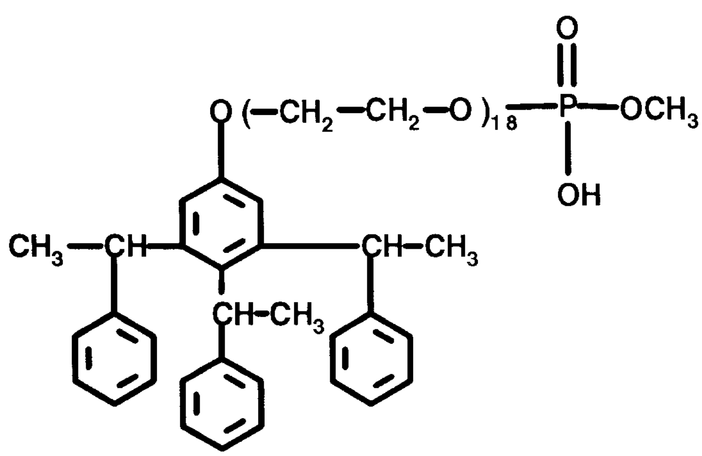

0 bis 10 Gewichts-% eines Vitamins oder Provitamins,
0 bis 10 Gewichts-% einer freien Fettsäure und gegebenenfalls übliche Trägerstoffe und/oder Verdünnungsmittel enthält.

**2.** Therapeutisches Systempräparat, welches 1 bis 95 Gewichts-% des spontan dispergierbaren Konzentrates gemäss Anspruch 1) enthält, und welches in Dosis-Einheitsform als Micropellets, Granulat, Dragées, Suppositorien, Ampullen oder als Kapseln vorliegt.

**3.** Spontan dispergierbares Konzentrat, enthaltend Sterolester- und/oder Sterolphosphorverbindungen der Formeln (I) bis (XV) gemäss Anspruch 1, zur Verwendung als Arzneimittel mit antitumoraler Wirksamkeit.

**4.** Sterolester der Formeln (I) bis (XV),

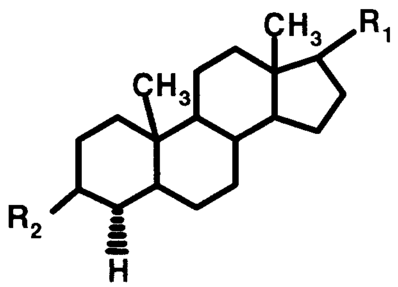

(I)

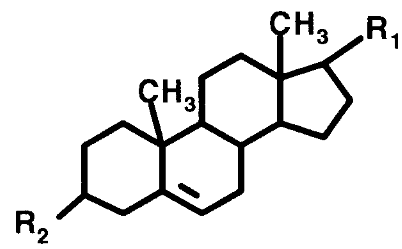

(II)

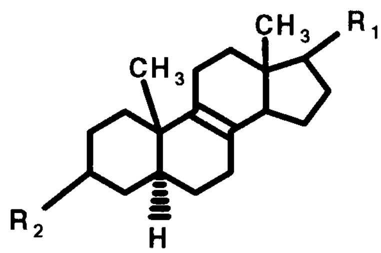

(III)

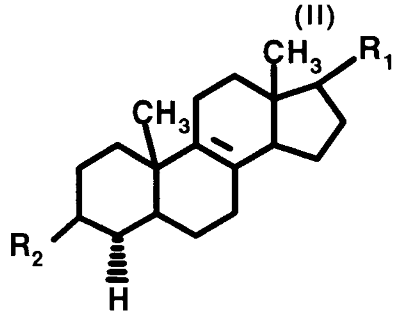

(IV)

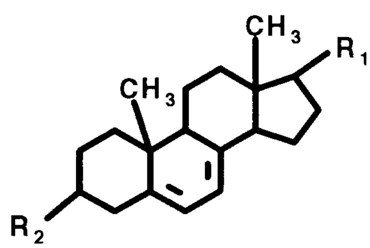

(V)

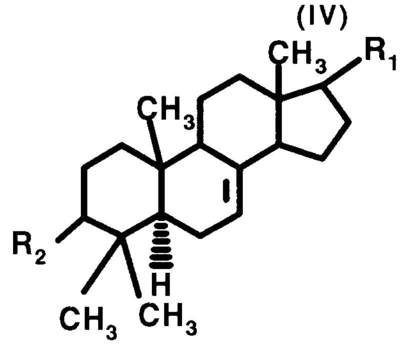

(VI)

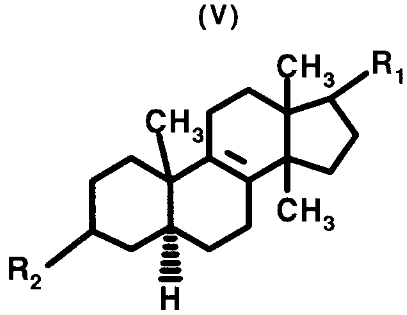

(VII)

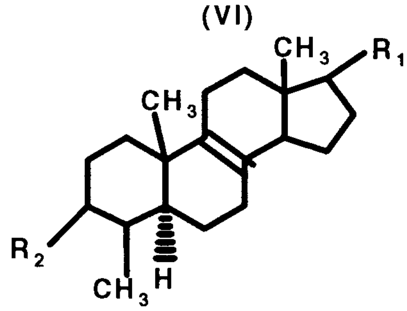

(VIII)

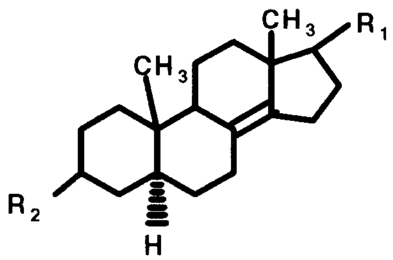

(IX)

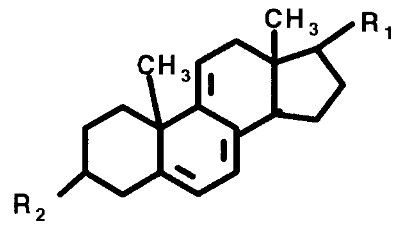

(X)

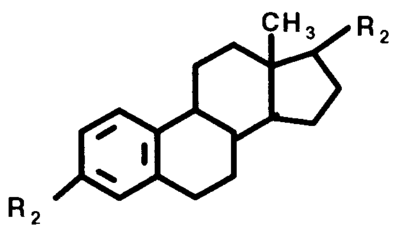

(XI)

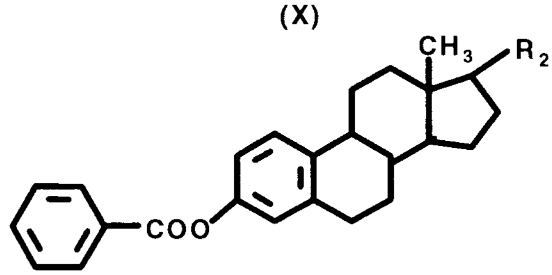

(XII)

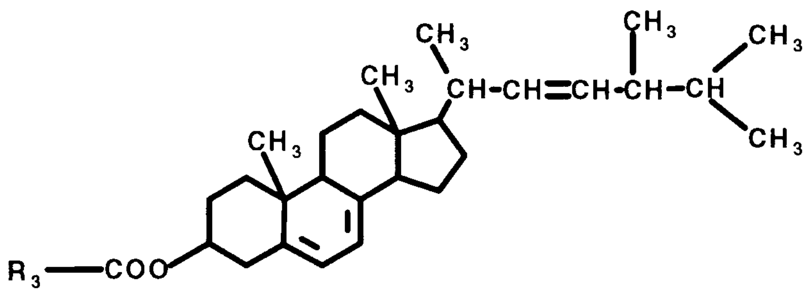

(XIII)

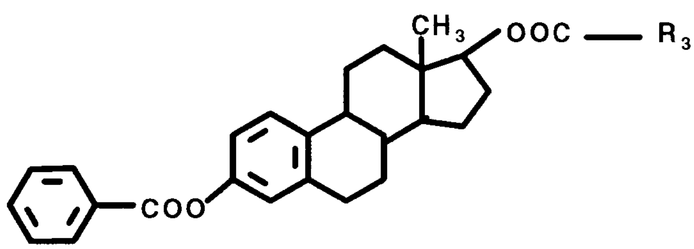

(XIV)

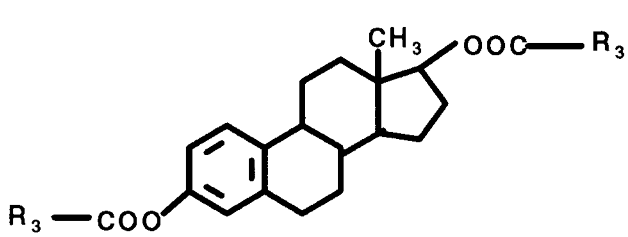

(XV)

wobei in den Formeln (I) bis (X) das Radikal $R_1$ eine $C_1$ bis $C_{10}$-Alkyl- oder eine $C_2$- bis $C_{10}$-Alkenylgruppe und das Radikal $R_2$ in den Formeln (I) bis (XII) eine Gruppe der Formeln (XVI) oder (XVII) ist

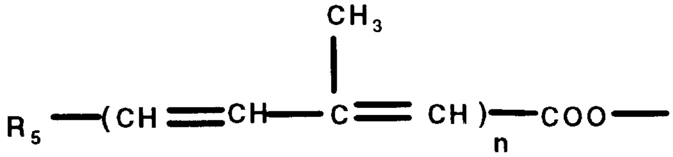

(XVI)

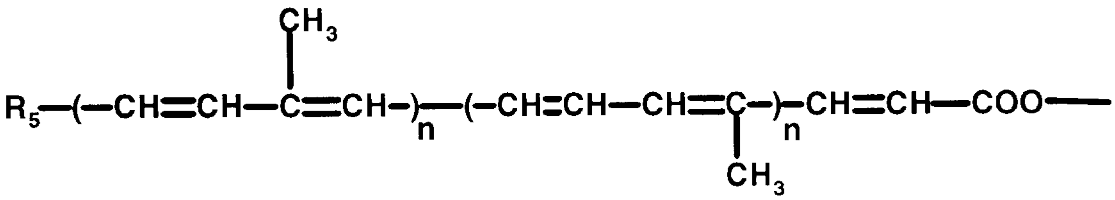

(XVII)

worin n die Zahlen 1, 2, 3, 4 oder 5 bezeichnet und $R_5$ für ein Radikal der Formeln:

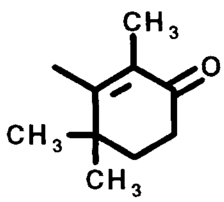

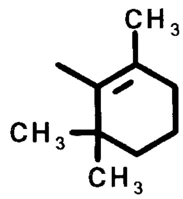

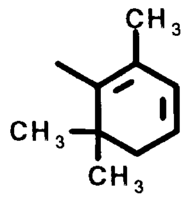

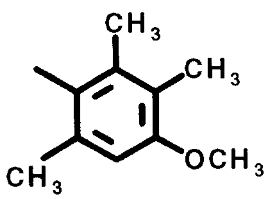

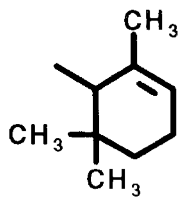

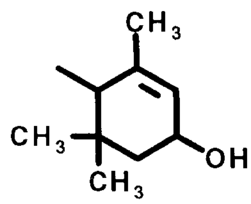

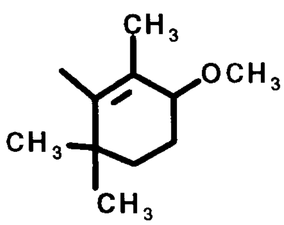

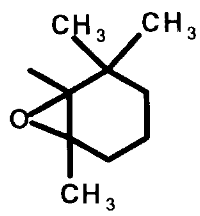

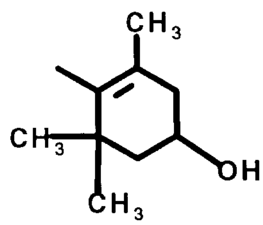

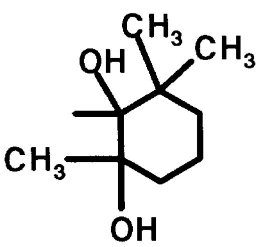

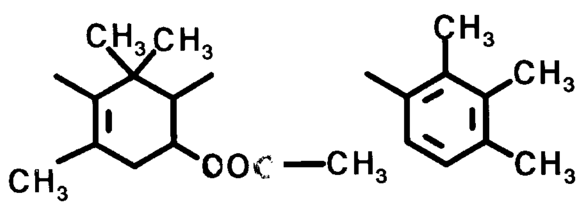

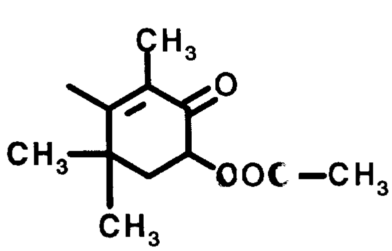

oder

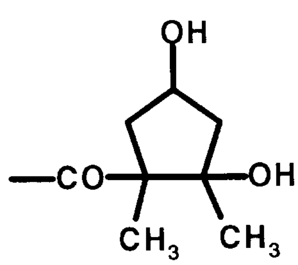

steht,
insbesondere steht $R_2$ für

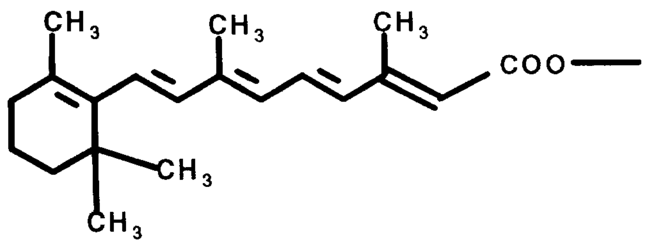

(XX)

und $R_3$ bezeichnet in den Verbindungen (XIII) bis (XV) eine $C_4$- bis $C_{32}$-Alkylgruppe oder eine $C_4$- bis $C_{32}$-Alkenylgruppe ;
mit der Massgabe, dass die Formel (II) nicht für Cholest-5-en-3-all trans-Retinat fungieren kann und dass die folgenden Estradioldiester der Formel (XV) ausgenommen sind: Estradioldiundecenoat, Estradioldipalmitat, Estradioldistearat und Estradioldioleat, und dass ferner im Falle der Verbindungen der Formel (XIII) die Ester der folgenden Säuren ausgeschlossen sind: Butter-, Isobutter-, Valerian-, Isovalerian-, Capron-Capryl-, Pelargon- Caprin- Laurin-, Tridecan-, Myristin-, Palmitin- Stearin-, Arachin- und Behensäure; ferner Palmitolein- Öl-, Linol- und Linolensäure.

**5.** Die Verbindungen
Stigmasteryl-all trans-retinat
Stigmasteryl-13-cis-retinat
Ergosteryl-all trans-retinat
Ergosteryl-13-cis-retinat
Ergosterylcrotonat
Ergosteryl-10-undecenoat
Ergosteryl-trans-2-dodecenoat

**6.** Verfahren zur Herstellung der Sterolester gemäss den Formeln (I) bis (XII ) darin bestehend, dass man eine Verbindung der Formel

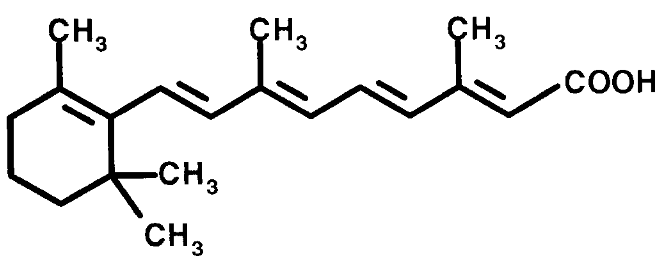

mit N,N'-Carbonyldiimidazol bei 25 bis 70 °C unter Zusatz einer katalytischen Menge eines Alkoholates in einem indifferenten Lösungsmittel umsetzt und anschliessend das entstandene Imidazolat mit einem Sterol der folgenden Formeln (XXV) bis (XXXVI) reagieren lässt

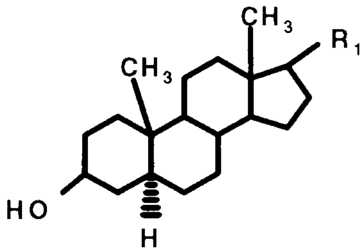

(XXV)

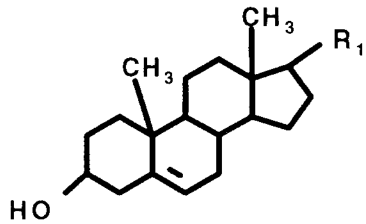

(XXVI)

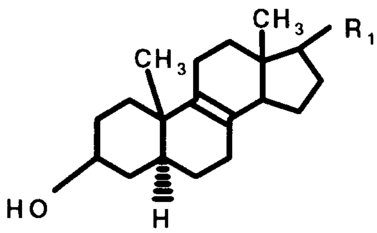

(XXVII)

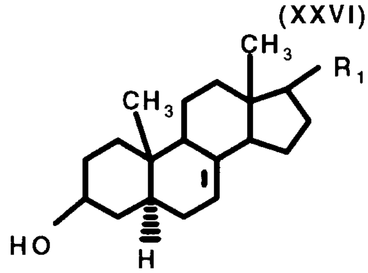

(XXVIII)

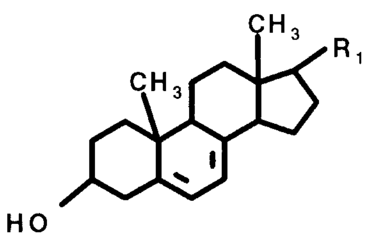

(XXIX)

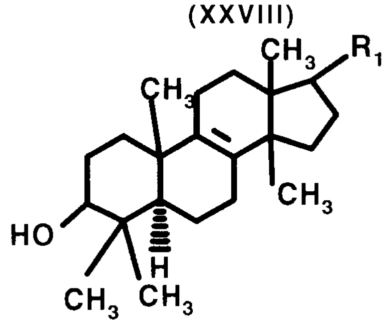

(XXX)

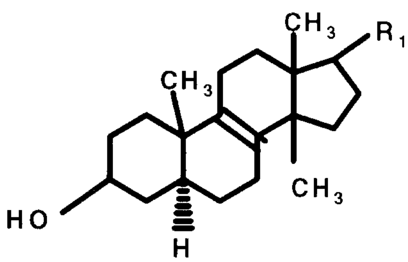

(XXXI)

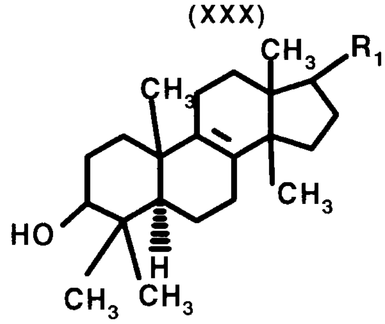

(XXXII)

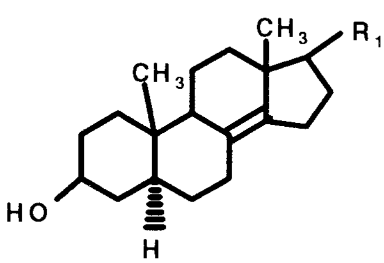

(XXXIII)

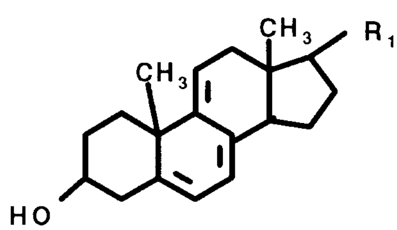

(XXXIV)

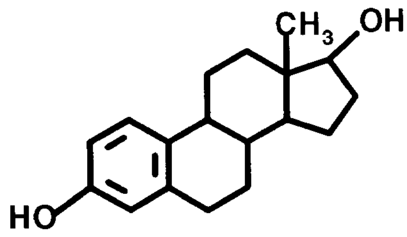

(XXXV)

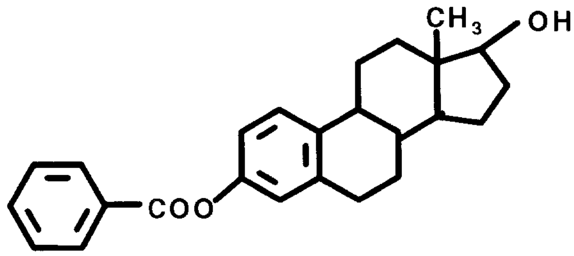

(XXXVI)

wobei in den Formeln (XXV) bis (XXXIV) das Radikal $R_1$ eine $C_1$- bis $C_{10}$-Alkyl- oder eine $C_2$- bis $C_{10}$-Alkenylgruppe ist.

7. Verfahren zur Herstellung von Sterolestern der Formeln (I) bis (XII), gemäss Anspruch 4), dadurch gekennzeichnet, dass man all trans-Retinsäure mit einem Chlorierungs-, bzw. Bromierungsmittel in das Säurechlorid, bzw. in das Säurebromid überführt und diese dann mit einem Sterol der Formeln (XXV) bis (XXXVI) bei einer Temperatur von 40 bis 120 °C in einem indifferenten Lösungsmittel und in Gegenwart eines Katalysator umsetzt.

8. Verfahren zur Herstellung von Sterolestern der Formel (XIII), laut Anspruch 4), dadurch gekennzeichnet, dass man eine Verbindung der Formel (XXXVII)

$R_7$ — COOH (XXXVII)

worin $R_7$ eine $C_4$- bis $C_{32}$-Alkyl- oder eine $C_4$- bis $C_{32}$-Alkenyl-, bzw. Alkapolyengruppe bedeutet, mit einem Chlorierungs-, bzw. Bromierungsmittel in das Säurechlorid, bzw. in das Säurebromid überführt und diese dann mit einem der Sterole der Formel (XXXVIII)

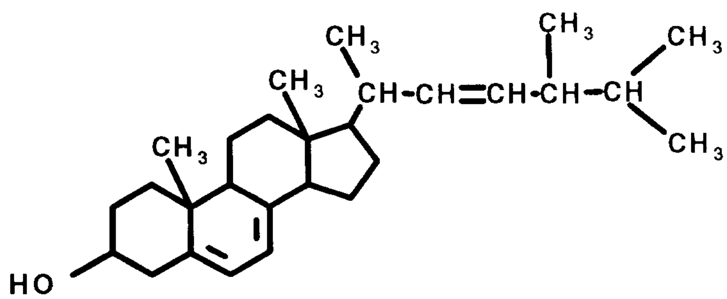

(XXXVIII)

bei einer Temperatur von 40 bis 120 °C in einem inerten Lösungsmittel und in Gegenwart eines Katalysators umsetzt.

9. Verfahren zur Herstellung von Sterolphosphorverbindungen der Formeln (I) bis (XII) gemäss Anspruch 1), dadurch gekennzeichnet, dass man eine Verbindung der Formel (XXIX)

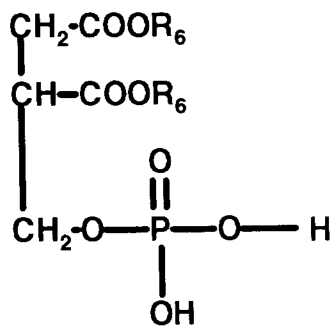

(XXIX)

worin $R_6$ eine $C_1$- bis $C_{32}$-Alkyl- oder eine $C_2$- bis $C_{32}$-Alkenyl-, bzw. Alkapolyengruppe bedeutet, in einem inerten Lösungsmittel mit Pivaloylchlorid umsetzt und dann mit einem Sterol der Formeln (XXV) bis (XXXVI) gemäss Anspruch 6) reagieren lässt.

**Claims**

1. Spontaneously dispersible concentrate, which if diluted with water produces microemulsions, comprising as the antitumor component 7,5 to 25 % by weight of a sterolester or sterol phosphatide according to the formulae (I) to (XV), or a combination of such components

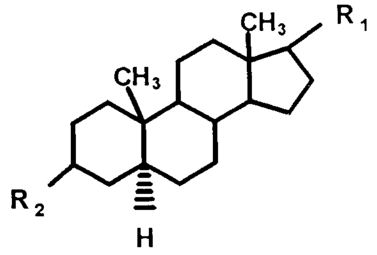

(I)

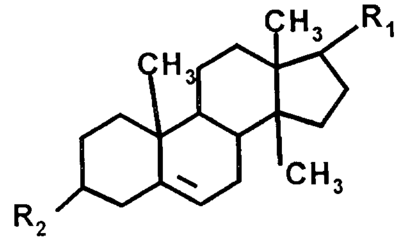

(II)

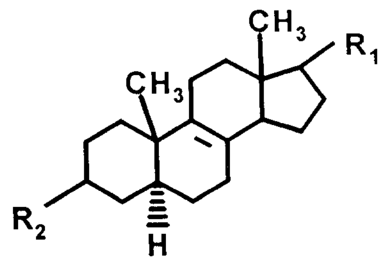

(III)

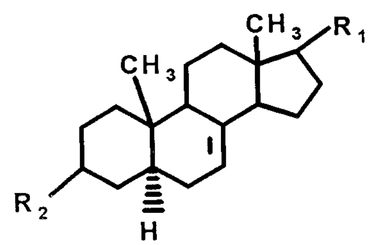

(IV)

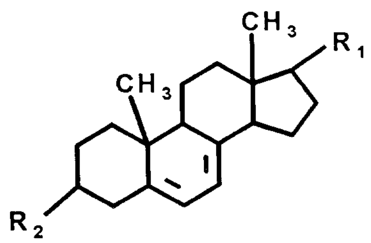

(V)

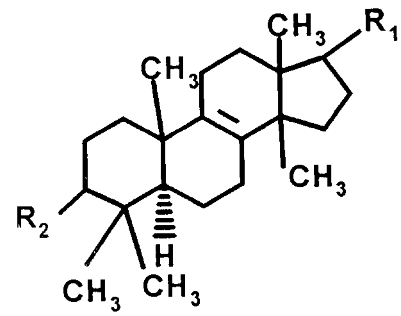

(VI)

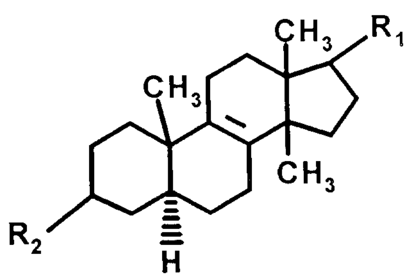

(VII)

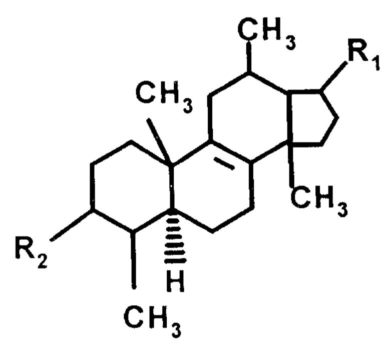

(VIII)

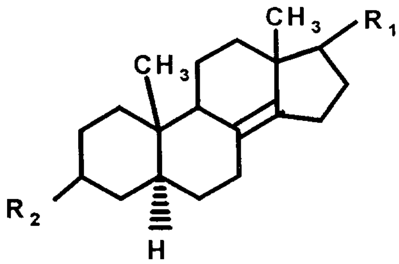

(IX)

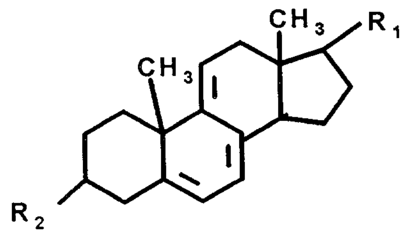

(X)

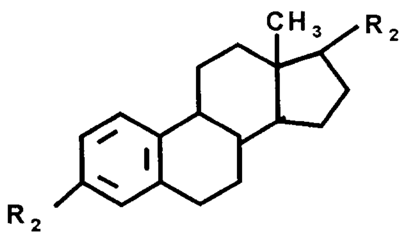

(XI)

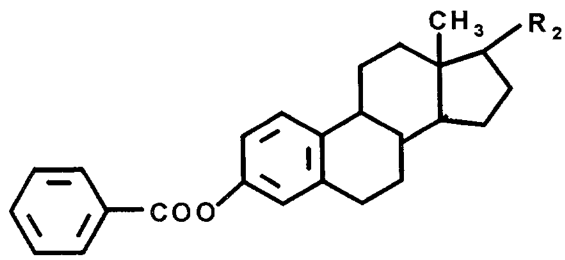

(XII)

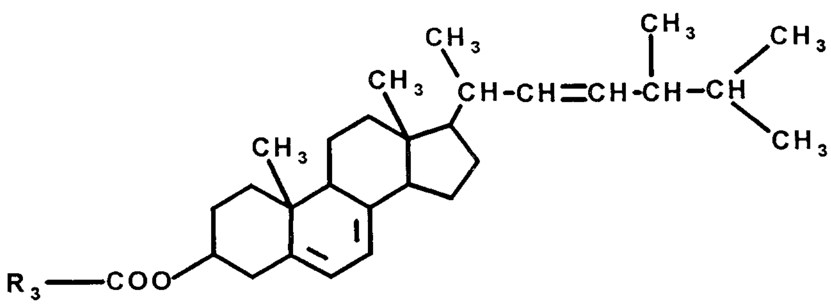

(XIII)

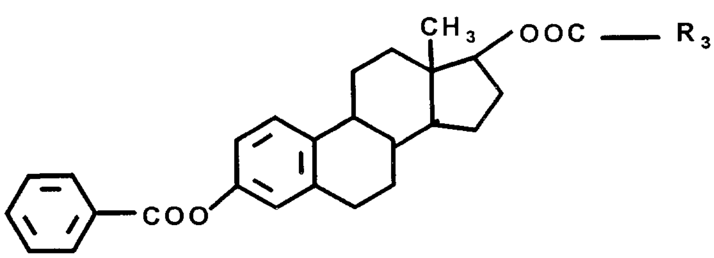

(XIV)

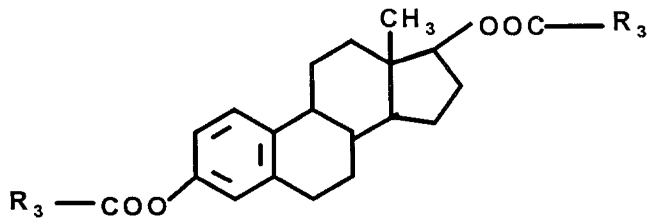

(XV)

whereby in the formulae (I) to (X) the radical $R_1$ is a $C_1$ to $C_{10}$ alkyl or a $C_2$ to $C_{10}$ alkenyl group and the radical $R_2$ in the formulae (I) to (XII) is a group of formulae (XVI) or (XVII)

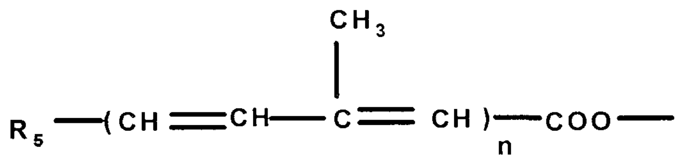

(XVI)

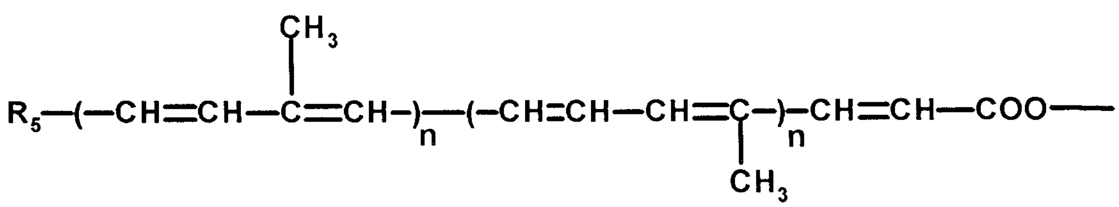

(XVII)

in which n means the numbers 1, 2, 3, 4 or 5 and $R_5$ stands for one of the radicals of formulae:

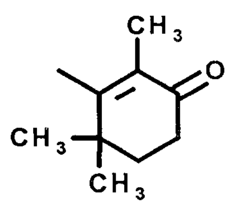
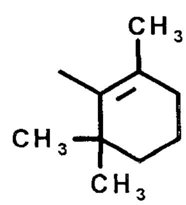
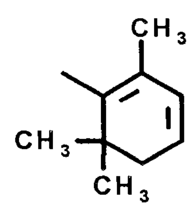
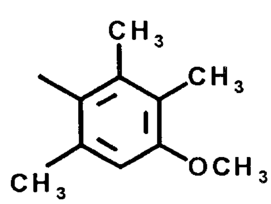
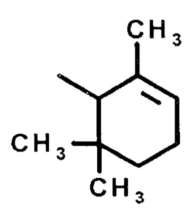
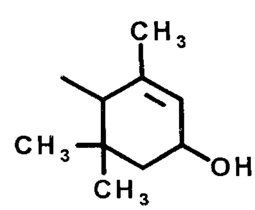
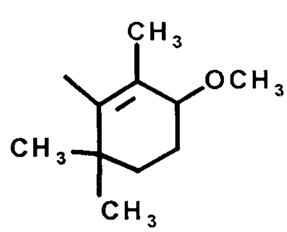
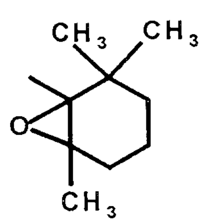
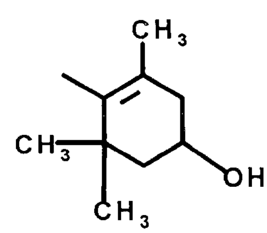

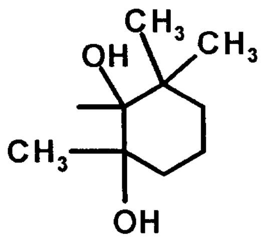

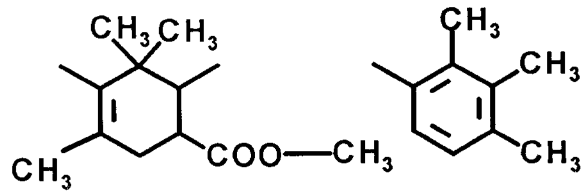

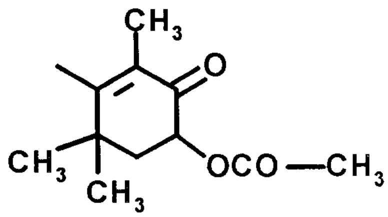

or

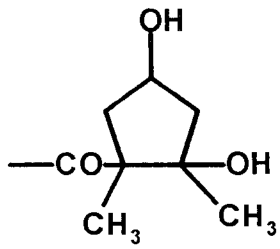

or represents the group according to formula (XVIII)

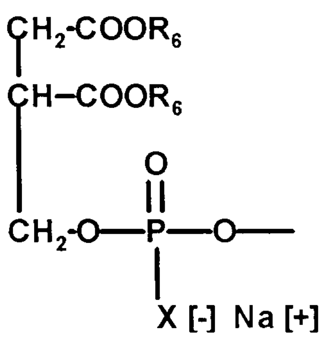

(XVIII)

wherein $R_6$ is a $C_1$ to $C_{32}$ alkyl or a $C_2$ to $C_{32}$ alkenyl or alkapolyene group and X stands for hydrogen or sulfur, and the radical $R_3$ in the formulae (XIII) to (XV) designates a $C_4$ to $C_{32}$ alkyl group and a $C_4$ to $C_{32}$ alkenyl or alkapolyene group respectively,

0 to 40 % by weight of isopropylmyristate, isopropylpalmitate or neutral oil

22,5 to 40 % by weight of the tert. octylphenylpolyoxyethylene ether having 9 to 10 oxyethylene groups, as well as

22,5 to 40 % by weight of a surfactant mixture consisting of 50 % each of the following components with formulae

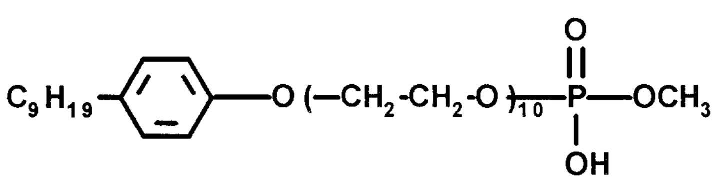

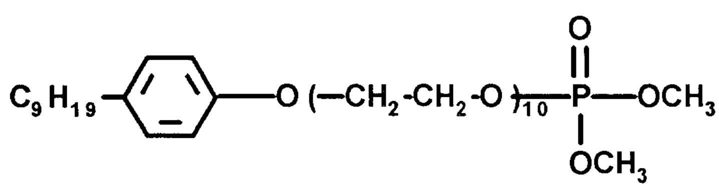

or of a phosphoric acid tenside of formula

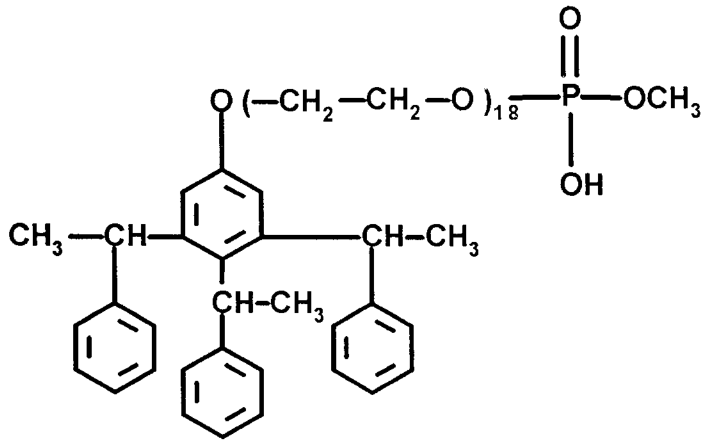

0 to 10 % by weight of a vitamin or provitamin
up to 10 % by weight of a free fatty acid and/or auxiliaries or solvents.

**2.** Therapeutic system's preparation which comprises 1 to 95 % by weight of the spontaneously dispersible concentrate according to claim 1) and which is present in unit-dosage form as micropellets, granules, dragées, suppositories, ampuls or as capsules.

**3.** Spontaneously dispersible concentrate, comprising sterolester and/or sterol phosphorous compounds as defined by the formulae (I) to (XV) according to claim 1), for use as medicaments having antitumor activity.

**4.** Sterol ester of the formulae (I) to (XV)

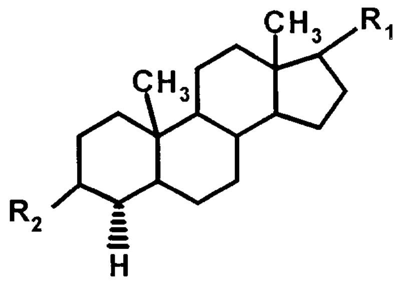

(I)

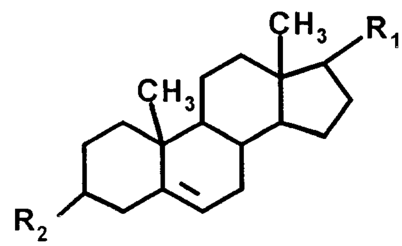

(II)

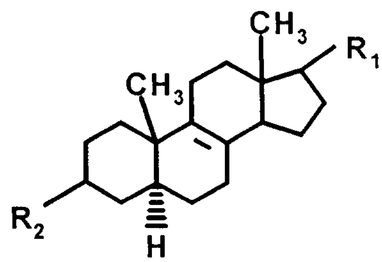

(III)

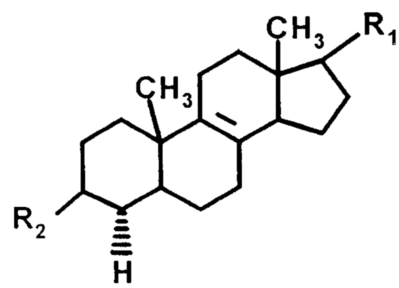

(IV)

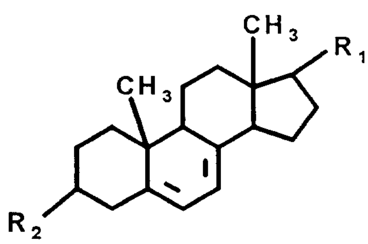

(V)

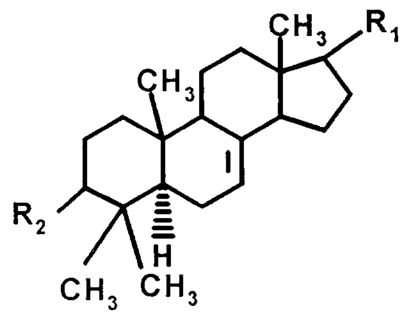

(VI)

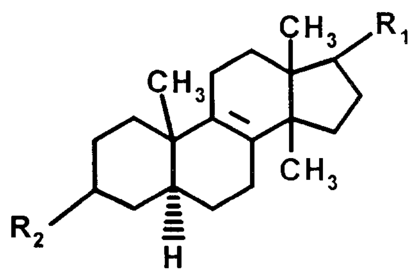

(VII)

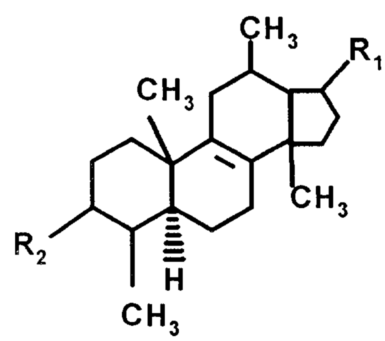

(VIII)

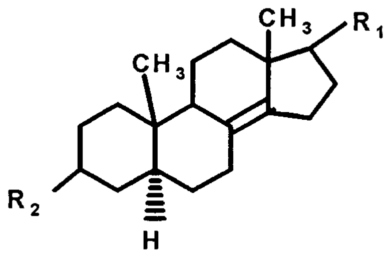

(IX)

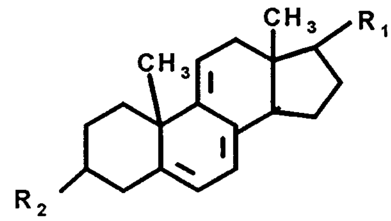

(X)

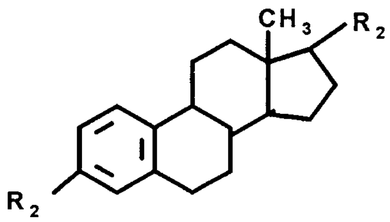

(XI)

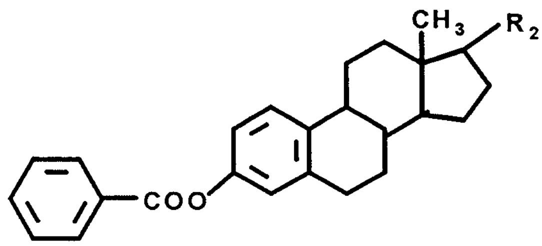

(XII)

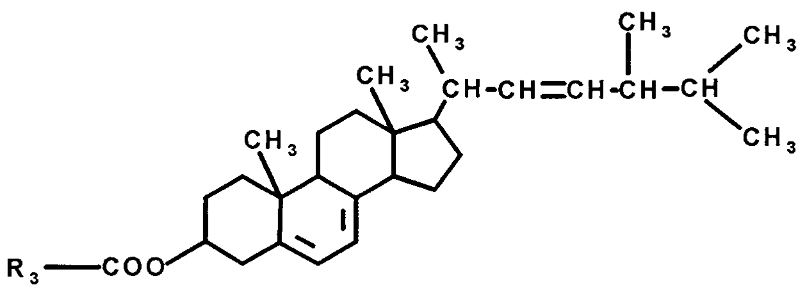

(XIII)

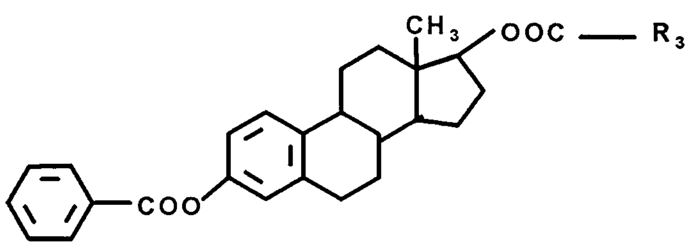

(XIV)

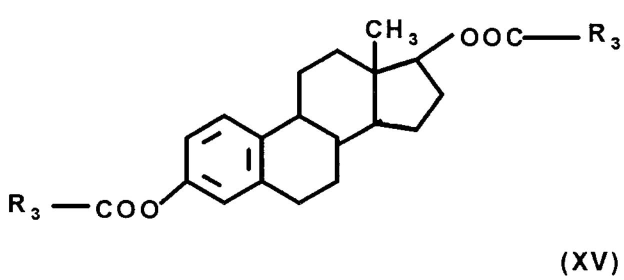

(XV)

whereby in the formulae (I) to (X) the radical $R_1$ is a $C_1$ to $C_{10}$ alkyl or a $C_2$ to $C_{10}$ alkenyl group and the radical $R_2$ in the formulae (I) to (XII) is a group of formulae (XVI) or (XVII)

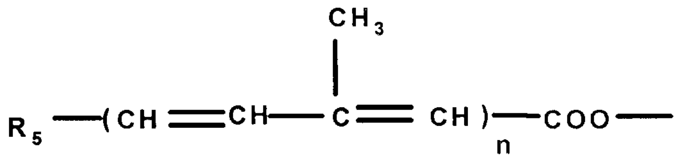

(XVI)

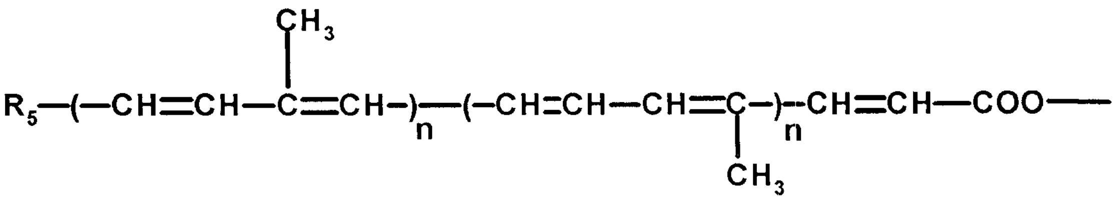

(XVII)

in which n means the numbers 1, 2, 3, 4 or 5 and $R_5$ stands for one of the radicals of formulae:

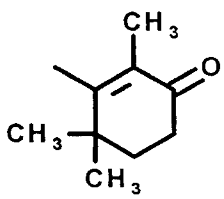
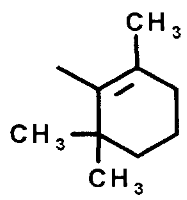
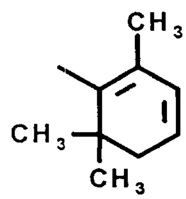
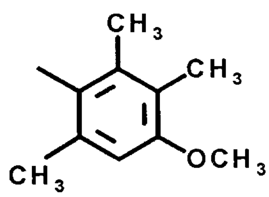
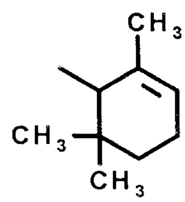
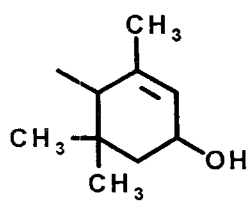
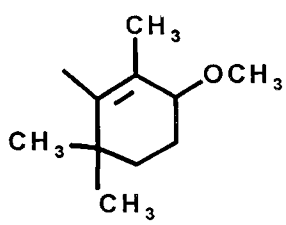
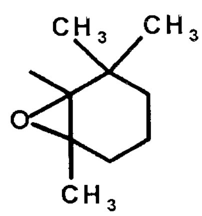
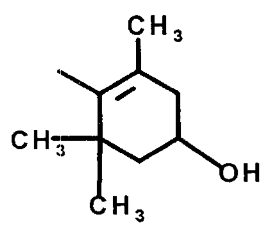
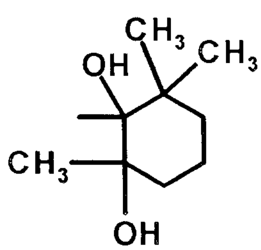
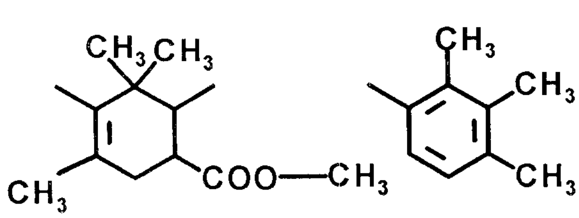

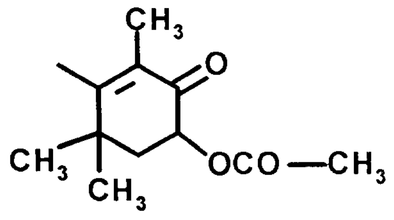

or

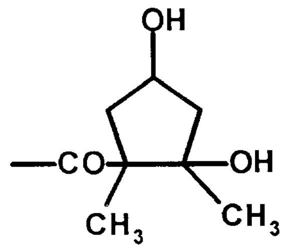

and $R_2$ stands in particular for a compound of formula

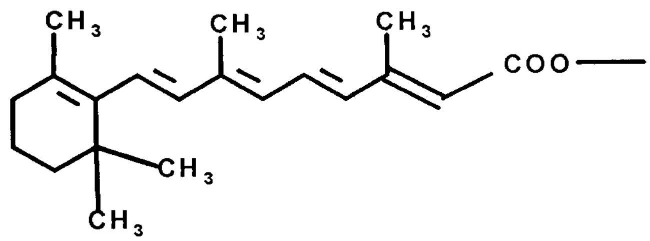

(XX)

and $R_3$ in the compounds (XII) to (XV) represents a $C_4$ to $C_{32}$ alkyl or a $C_4$ to $C_{32}$ alkenyl group, with the proviso that formula (II) cannot stand for the compound cholest-5-en-3-all trans-retinate and that in addition the following estradiol diester compounds according to formula (XV) are excluded: estradiol diundecenoate, estradiol dipalmitate, estradiol distearate and estradiol dioleate, and that, furthermore, in the case of compounds formed according to formula (XIII) the esters with the following acids are also excluded: butyric, isobutyric, valeric, valerianic, isovalerianic, capronic, caprylic, pelargonic, caprinic, lauric, tridecanic, myristic, palmitic, stearic, arachidic and behenic acid; as well as palmitoleic, oleic, linoleic and linolenic acid.

**5.** The compounds
Stigmasteryl-all trans-retinate
Stigmasteryl-13-cis-retinate
Ergosteryl-all trans-retinate
Ergosteryl-13-cis-retinate
Ergosteryl crotonate
Ergosteryl-10-undecenoate
Ergosteryl-trans-2-dodecenoate

**6.** A process for the production of sterol esters according to formulae (I) to (XII), consisting of reacting a compound of formula

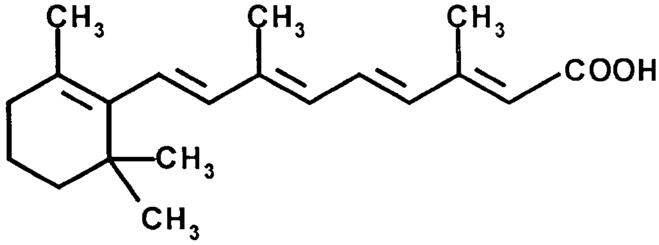

with N,N'-carbonyl diimidazole at a temperature of 25 to 70 °C, under addition of a catalytic amount of an alcoholate in an indifferent solvent and subsequent reaction of the imidazole formed with a sterol of the following formulae (XXV) to (XXXVI) :

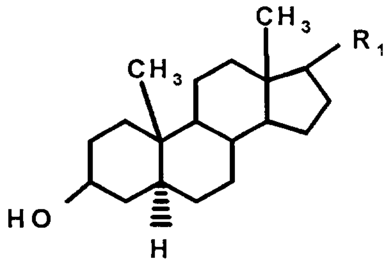

(XXV)

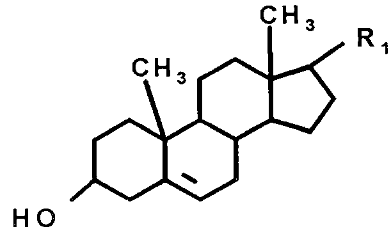

(XXVI)

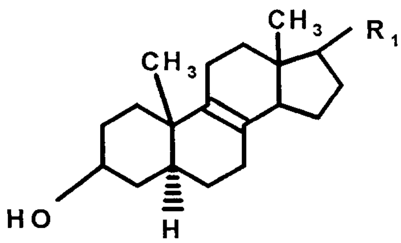

(XXVII)

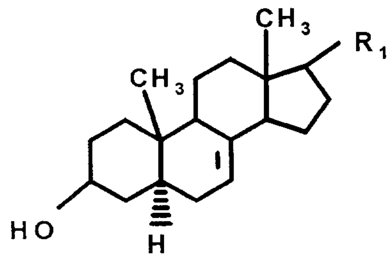

(XXVIII)

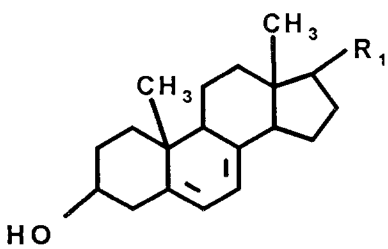

(XXIX)

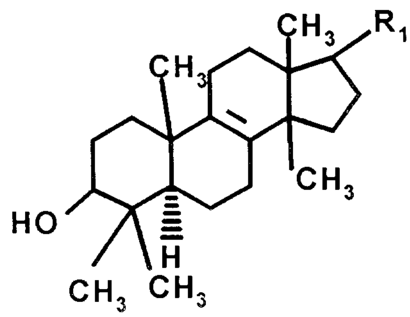

(XXX)

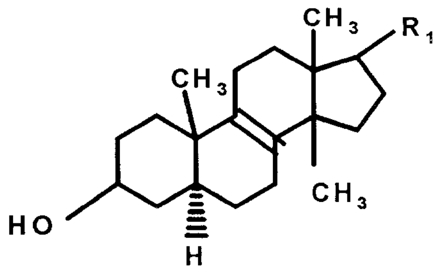

(XXXI)

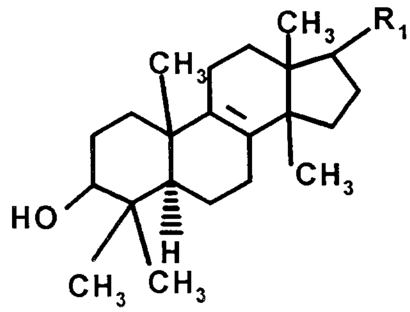

(XXXII)

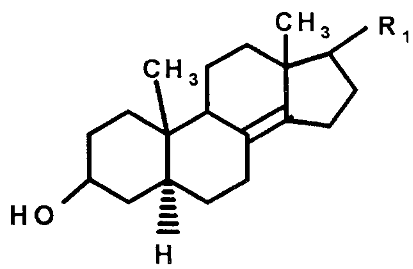

(XXXIII)

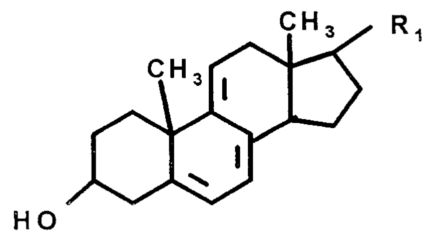

(XXXIV)

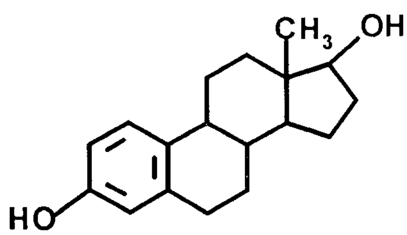

(XXXV)

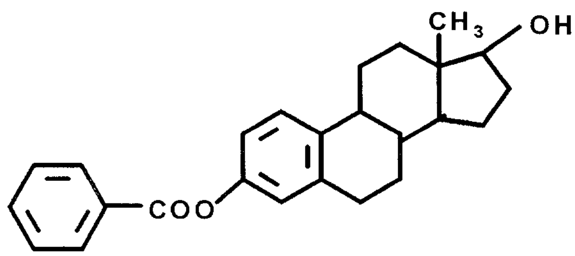

(XXXVI)

whereby in the formulae (I) to (X) the radical $R_1$ is a $C_1$ to $C_{10}$ alkyl or a $C_2$ to $C_{10}$ alkenyl group.

**7.** A process for the production of sterol esters according to formula (I) to (XII) in accordance with claim 1), whereby all trans retinoic acid is activated with a chlorination and bromination agent respectively to give the acid chloride and acid bromide respectively and is subsequently reacted with a sterol according to formulae (XXV) to (XXVI) at a temperature of 40 to 120 °C, in an indifferent solvent and in the presence of a catalyst.

**8.** A procedure for the production of sterol esters according to formulae (XIII) according to claim 3), consisting of reacting a compound of formula (XXXVII)

$R_7 - COOH$ (XXXVII)

in which $R_7$ stands for a $C_4$ to $C_{32}$ alkyl or a $C_4$ to $C_{32}$ alkenyl and alkapolyene group respectively, with a chlorination and bromination agent respectively to give the acid chloride and acid bromide respectively and is subsequently reacted with a sterol according to formula (XXXVIII)

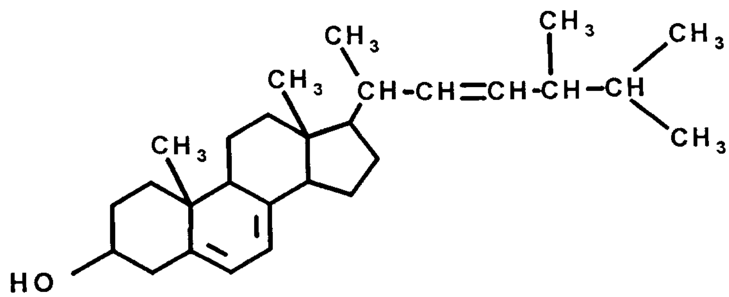

(XXXVIII)

at a temperature of 40 to 120 °C, in an indifferent solvent and in the presence of a catalyst.

**9.** A procedure for the production of sterol phosphorous compounds defined in the formulae (I) to (XII) according to claim 1), consisting of reacting a compound of formula (XXIX)

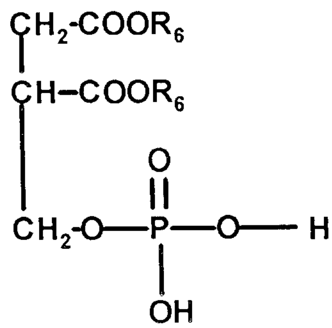

in which $R_6$ is a $C_1$ to $C_{32}$ alkyl or a $C_2$ to $C_{32}$ alkenyl or alkapolyene group, in an inert solvent with pivaloylchloride and subsequently reacting the product formed with a sterol described by the formulae (XXV) to (XXXVI) according to claim 6).

**Revendications**

**1.** Un concentré spontanément dispersible qui avec l'adjonction d'eau forme une microémulsion contenant come composé antitumoral
7,5 à 25 % par poids soit d'un ester formé avec un stérol ou d'un phosphatide de stérol ou d'un phosphonate de stérol, soit d'une combinaison de tels composés, selon formules (I) à (XV)

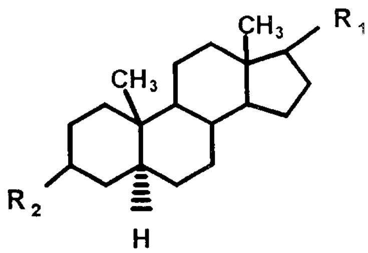

(I)

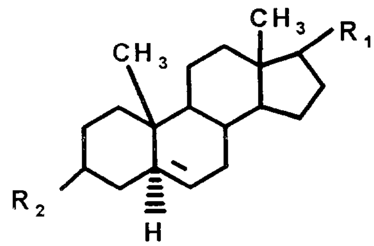

(II)

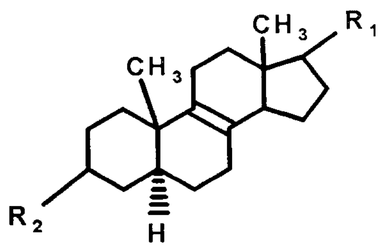

(III)

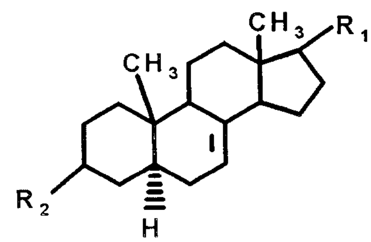

(IV)

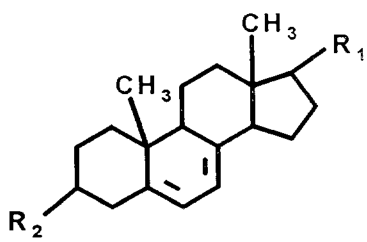

(V)

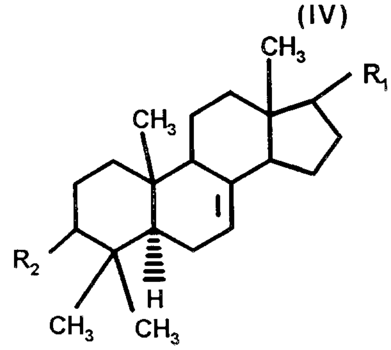

(VI)

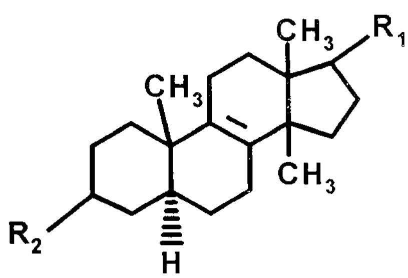

(VII)

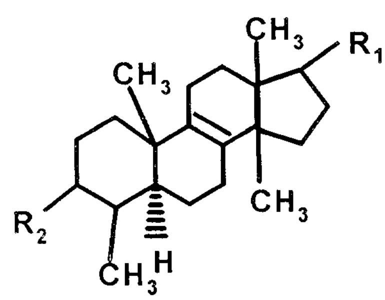

(VIII)

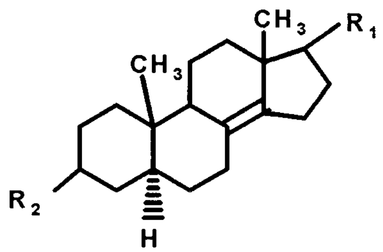

(IX)

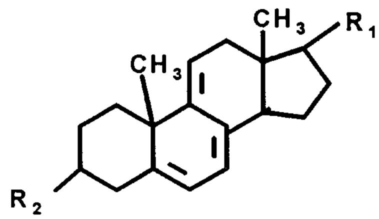

(X)

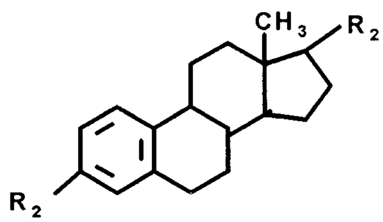

(XI)

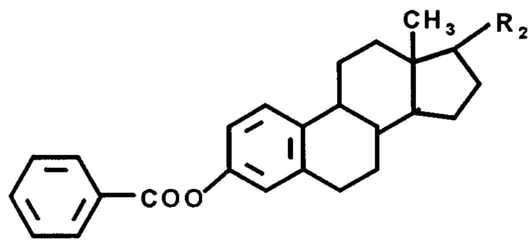

(XII)

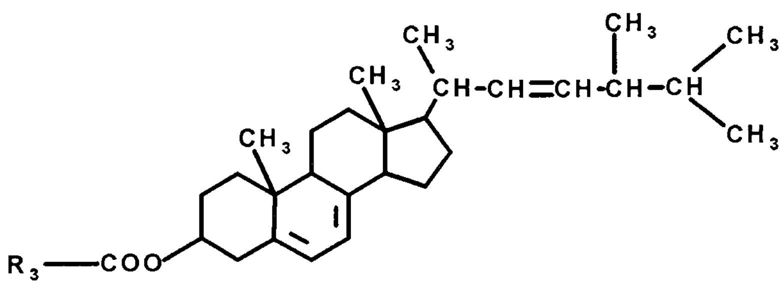

(XIII)

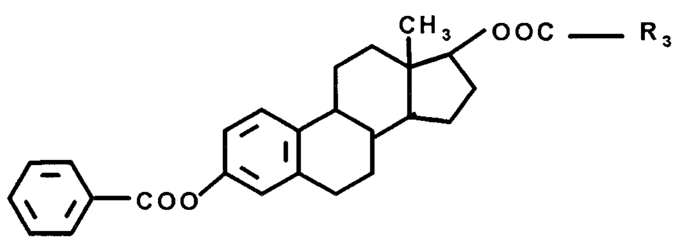

(XIV)

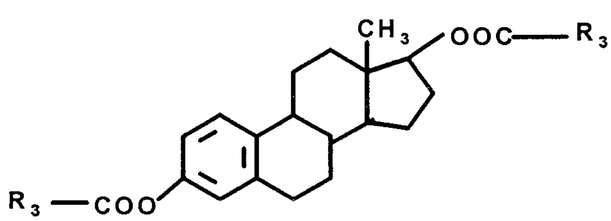

(XV)

où dans les formules (I) à (X) le radical $R_1$ représente un groupe alkyle ($C_1$ à $C_{10}$) ou un groupe alkenyle ($C_2$ à $C_{10}$) et le radical $R_2$ dans les formules (I) à (XII) désigne un groupe correspondant aux formules (XVI) ou (XVII)

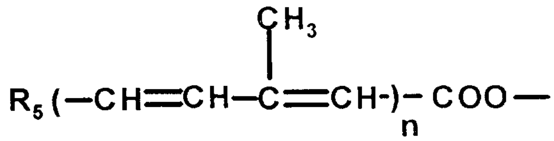

(XVI)

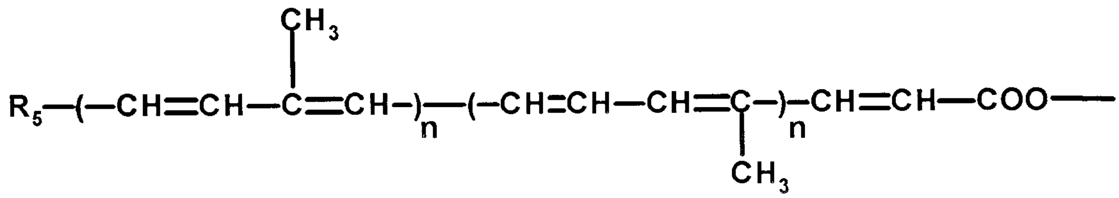

(XVII)

dans lesquelles la lettre n signifie les nombres 1, 2, 3, 4 ou 5 et $R_5$ représente l'un des radicaux selon les formules

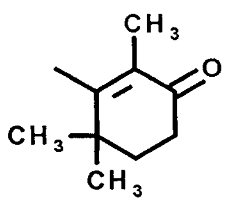
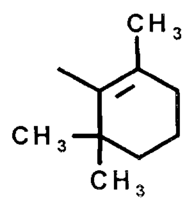
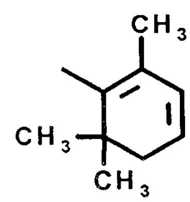
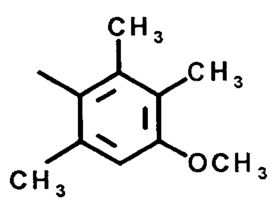
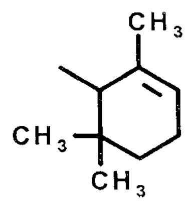
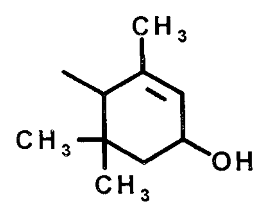
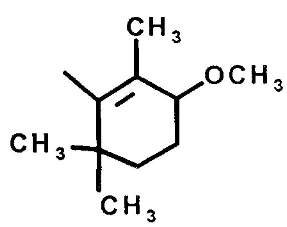
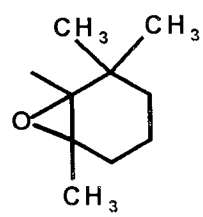
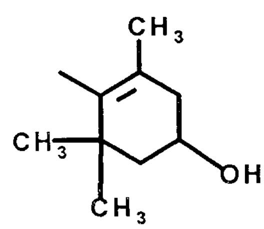
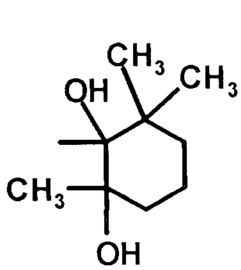
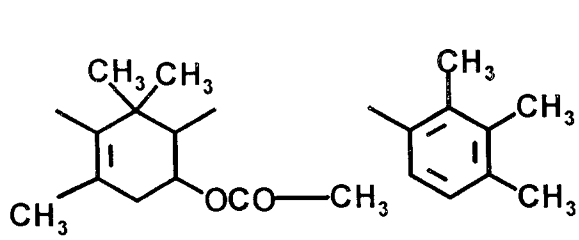

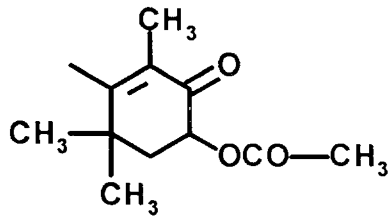

ou

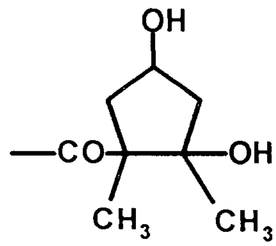

ou symbolise encore le groupe selon formule (XVIII)

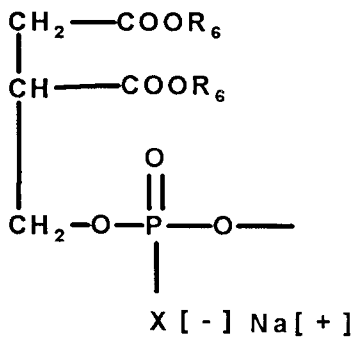

(XVIII)

dans lequel $R_6$ représente un groupe alkyle ($C_1$ à $C_{32}$) ou un groupe alkenyle ($C_2$ à $C_{32}$), et la lettre X se rapporte à oxygène ou soufre, tandis que $R_3$ dans les formules (XIII) à (XV) représente un groupe alkyle ($C_4$ à $C_{32}$) ou un groupe alkenyle ($C_4$ à $C_{32}$), ainsi que

0 à 40 % par poids de myristate d'isopropyle, de palmitate d' isopropyle ou d'une huile neutre

20 à 45 % par poids de surfactant d'éther octylphénylpolyoxyéthylène composé de 9 à 10 groupes oxyéthylène

20 à 45 % par poids d'un mélange 50:50 des surfactants avec formules

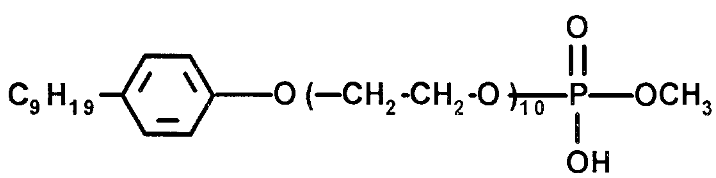

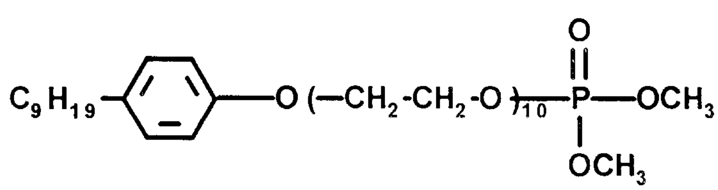

ou du surfactant ester de l'acide phosphorique avec formule

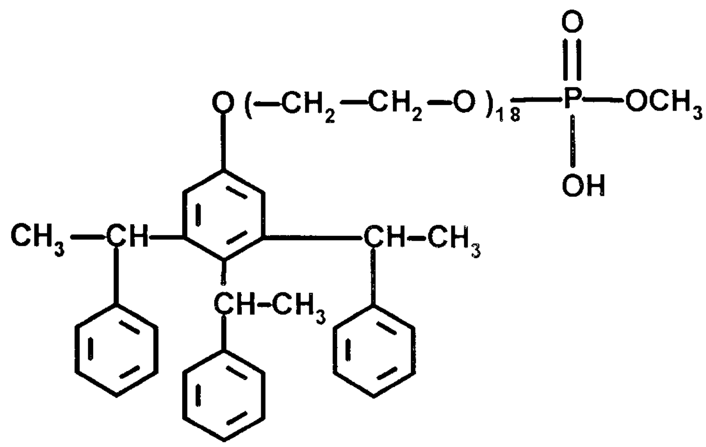

0 à 10 % par poids de divers vitamines ou provitamines
0 à 10 % par poids d'acide gras libres et auxiliaires et/ou diluants usuels.

**2.** Un système thérapeutique contenant de 1 à 95 % par poids de l'un des concentrés spontanément dispersibles définis dans la revendication 1, présent sous forme de "micropellets", granules, dragées, suppositoires, ampoules ou capsules.

**3.** Un concentré spontanément dispersible conforme à la revendication 1, contenant des esters de stérols et/ou des composés à fonction type phosphorique selon les formules (I) à (XV) en accord avec la revendication 1, pour emploi comme médicament à activitées antitumorales.

**4.** Les esters de stérol selon les formules (I) à (XV)

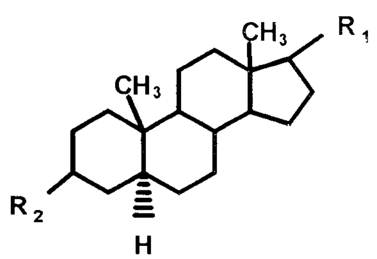

(I)

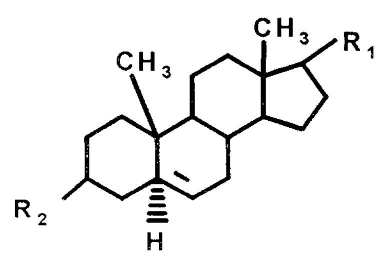

(II)

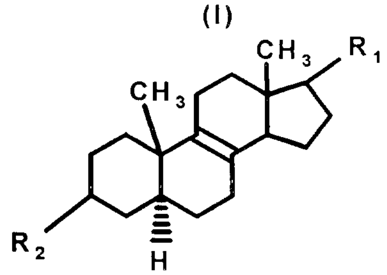

(III)

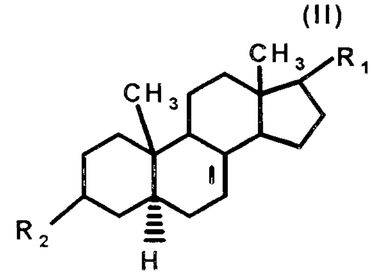

(IV)

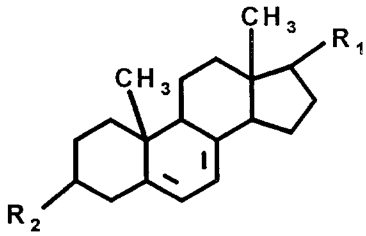

(V)

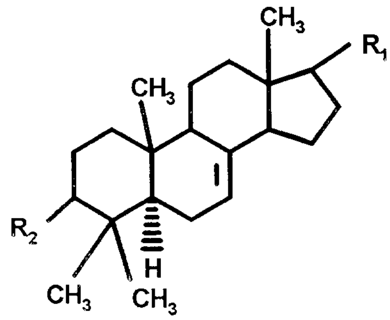

(VI)

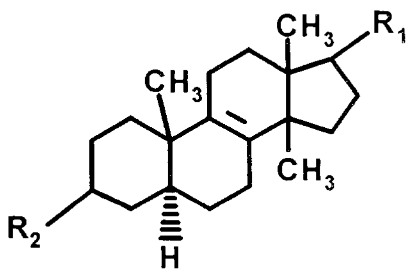

(VII)

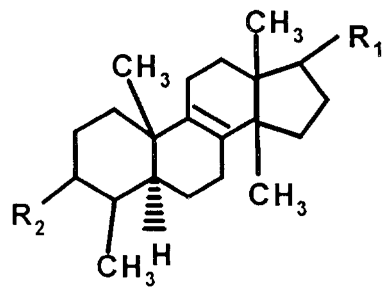

(VIII)

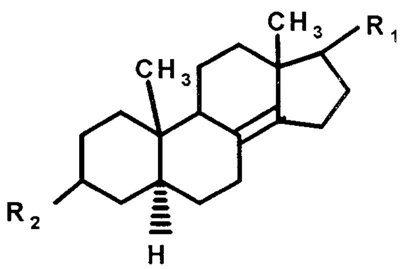

(IX)

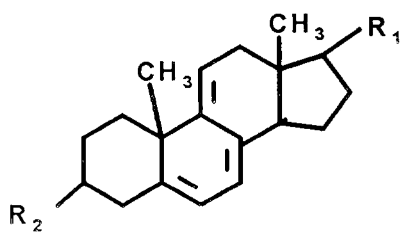

(X)

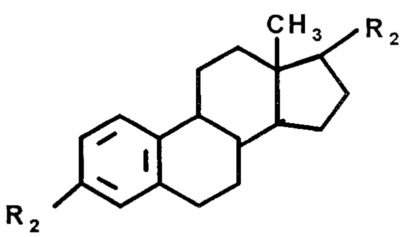

(XI)

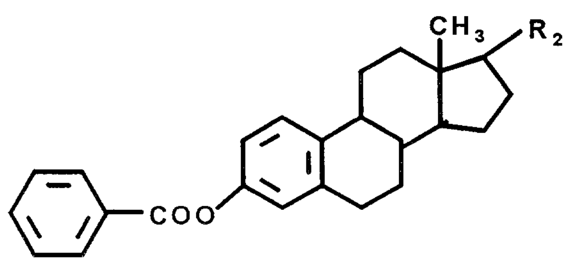

(XII)

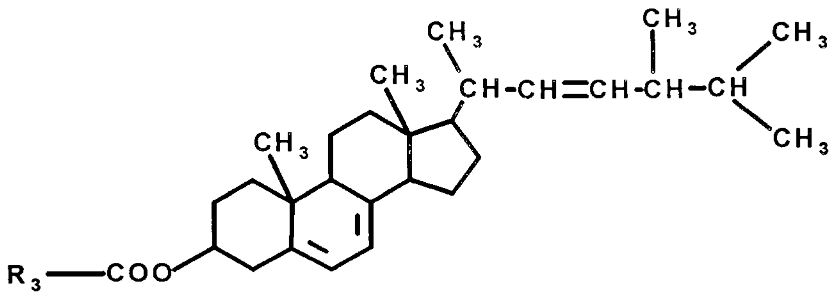

(XIII)

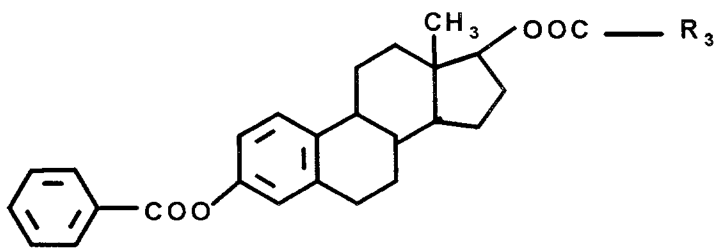

(XIV)

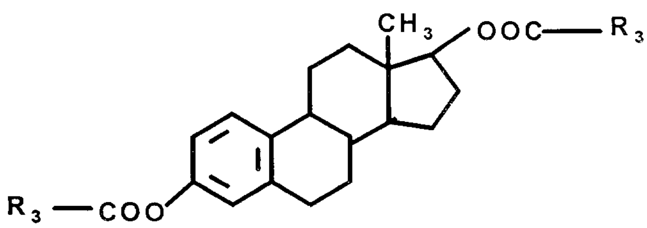

(XV)

où dans les formules (I) à (X) le radical $R_1$ représente un groupe alkyle ($C_1$ à $C_{10}$) ou un groupe alkenyle ($C_2$ à $C_{10}$) et le radical $R_2$ dans les formules (I) à (XII) désigne un groupe correspondant aux formules (XVI) ou (XVII)

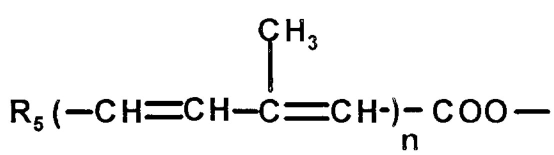

(XVI)

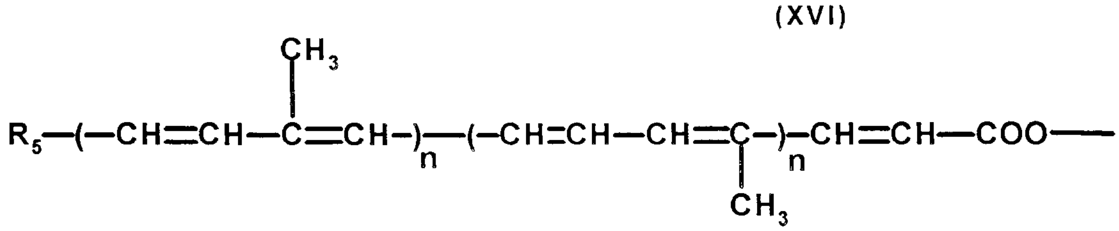

(XVII)

dans lesquelles n signifie les nombres 1, 2, 3, 4 ou 5 et $R_5$ re-présente l'un des radicaux selon les formules

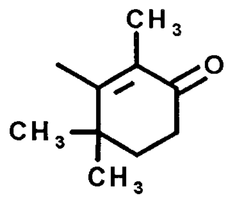
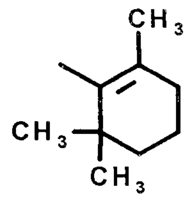
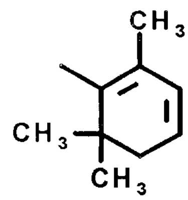
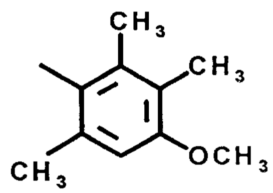
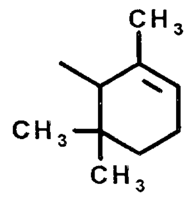
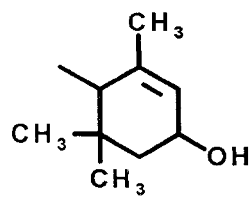
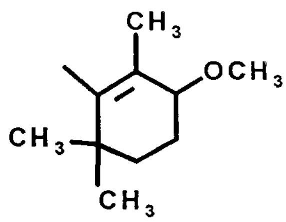
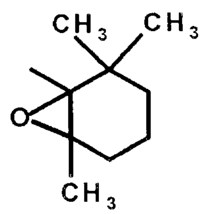
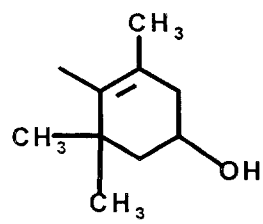
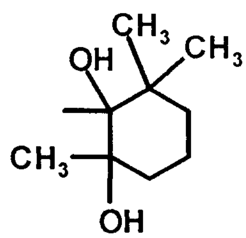
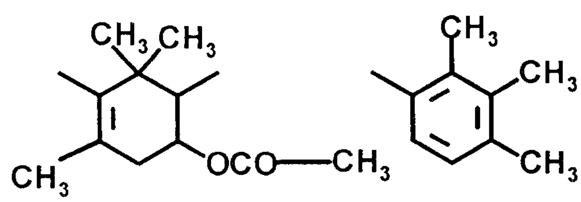
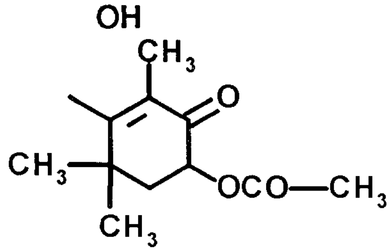

ou

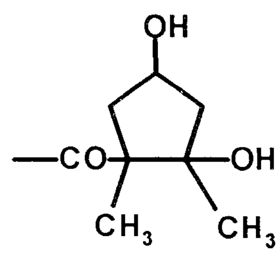

$R_2$ signifie en particulier le composé selon formule (XX)

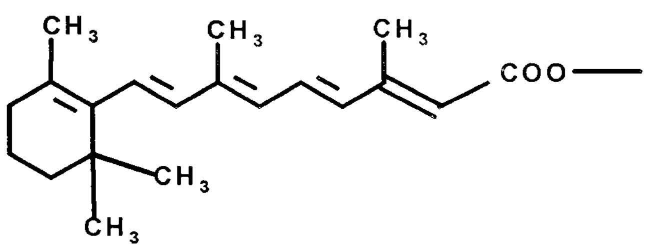

(XX)

et $R_3$ dans les composés (XII) à (XV) représente un groupe alkyle ($C_4$ à $C_{32}$) ou un groupe alkenyle ($C_4$ à $C_{32}$), sous conditions explicites que la formule (II) ne puisse pas signifier le composé cholest-5-en-3-all trans rétinate, que les suivants composés selon la formule (XV) soient exclus: diundecenoate

d'estradiol, dipalmitate d'estradiol, distéarate d'estradiol et dioléate d'estradiol et que les esters formés en accord avec la formule (XIII) avec les acides suivants soient également exclus: acide butyrique, isobutyrique, valérique, valérianique, isovalérianique, capronique, caprylique, pelargonique, caprinique, laurique, tridécanique, myristique, palimitique, stéarique, arachidique et béhénique, ainsi que les acides palmitoléique, oléique, linoléique et linolénique

**5.** Les composés
Stigmasteryl-all trans-rétinate
Stigmasteryl-13-cis-rétinate
Ergosteryl-all trans-rétinate
Ergosteryl-13-cis-rétinate
Ergosteryl crotonate
Ergosteryl-10-undecenoate
Ergosteryl-trans-2-dodécenoate

**6.** Un procédé de préparation d'esters de stérols selon formules (I) à (XII), où un composé de formule

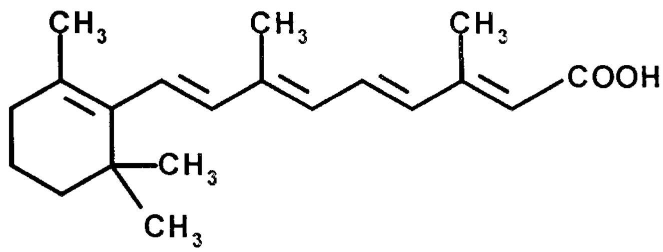

est d'abord réagi avec du N,N'-carbonyl diimidazole à une température de 25 à 70 °C sous addition d'un alcoholat catalyseur dans un solvant indifférent, et est suivi par la réaction de l'imidazolate formé d'acide rétinique, avec un composé de stérol de formules (XXV) à (XXXVI) :

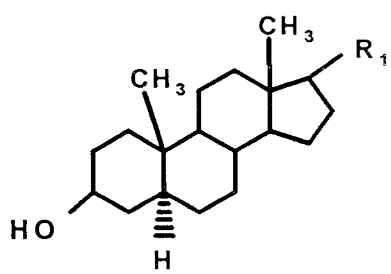

(XXV)

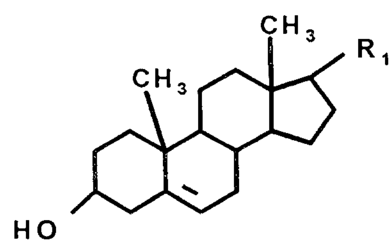

(XXVI)

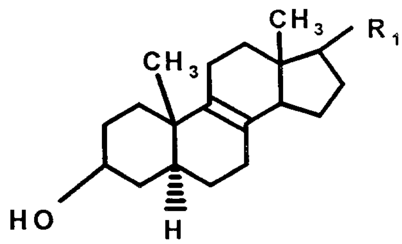

(XXVII)

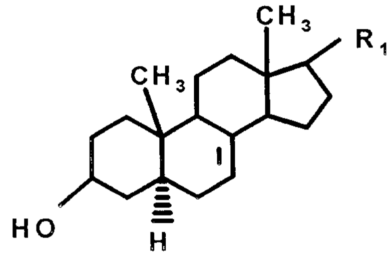

(XXVIII)

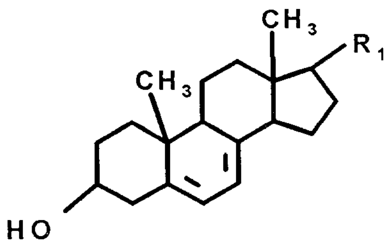

(XXIX)

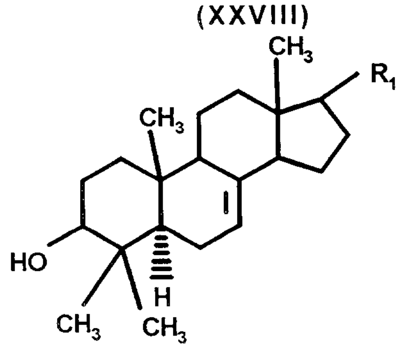

(XXX)

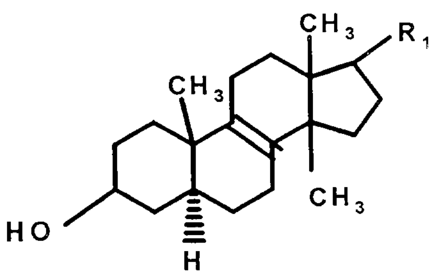

(XXXI)

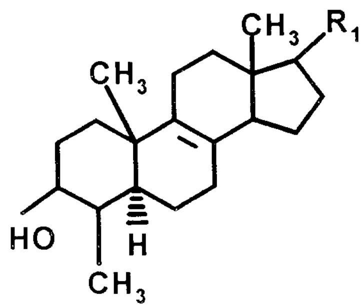

(XXXII)

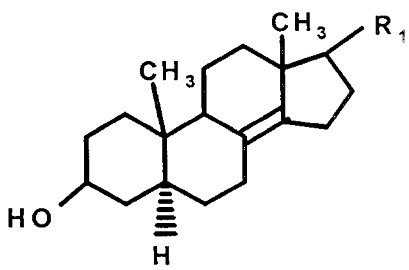

(XXXIII)

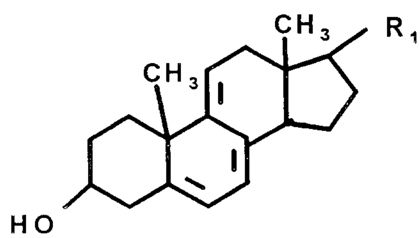

(XXXIV)

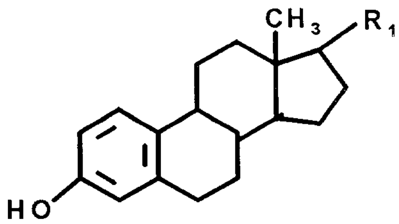

(XXXV)

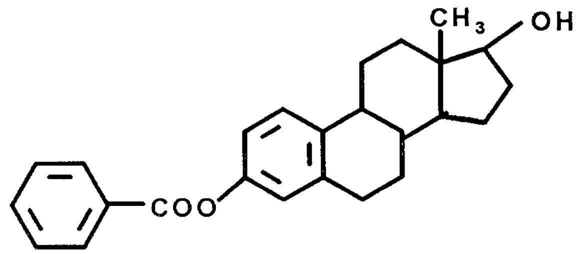

(XXXVI)

ou dans les formules (I) à (X) le radical $R_1$ caractérise un groupe alkyle ($C_1$ à $C_{10}$) ou un groupe alkenyle ($C_2$ à $C_{10}$).

**7.** Une procédure de production d'esters de stérols selon les formules (I) à (X), conformes à la revendication 1, consistant à activer le composé acide all trans rétinique avec un agent de chlorination ou de bromination pour obtenir le chlorure de l'acide ou le bromure de l'acide, suivi par la réaction avec un composé stérol de formule (XXV) à (XXXVI) à une température de 40 à 120 °C dans un solvant indifférent en présence d'un catalyseur.

**8.** Une procédure de production d'esters de stérols selon la formule (XIII) en accord avec la revendication 3, consistant a faire réagir un composé de formule (XXXVII)

$R_7 - COOH$ (XXXVII)

dans laquelle $R_7$ désigne un groupe alkyle ($C_4$ à $C_{32}$) ou un groupe alkenyle ($C_4$ à $C_{32}$) avec un agent de chlorination ou de bromination pour obtenir le chlorure de l'acide ou le bromure de l'acide, suivi par la réaction avec un composé sterol de formule (XXXVII)

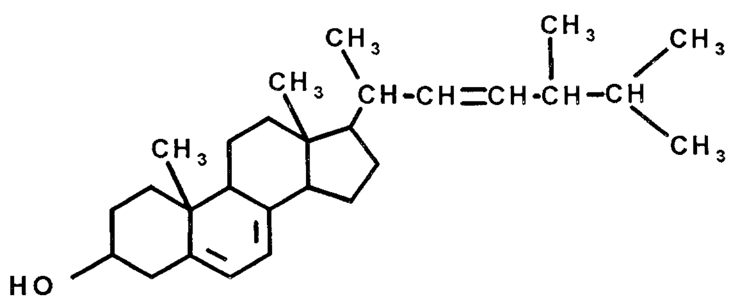

(XXXVII)

à une température de 40 à 120 °C dans un solvant indifférent en présence d'un catalyseur.

**9.** Un procédé pour la production de composés sterols phosphatidiques en accord avec les formules (I) à (XII), conformes à la revendication 1, où un composé selon formule (XXIX) :

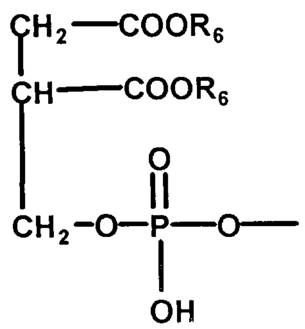

(XXIX)

dans laquelle $R_6$ désigne un groupe alkyle ($C_1$ à $C_{32}$) ou un groupe alkenyle ($C_2$ à $C_{32}$), est réagi dans un solvant indifférent avec le chlorure de l'acide pivalique, suivi par la réaction du produit avec un sterol selon les formules (XXV) à (XXXVI), en accord avec la revendication 6.

ERGOSTEROL-3β-all trans-RETINAT

N M R SPECTRUM (BRUKER AM-360 SPECTROMETER)

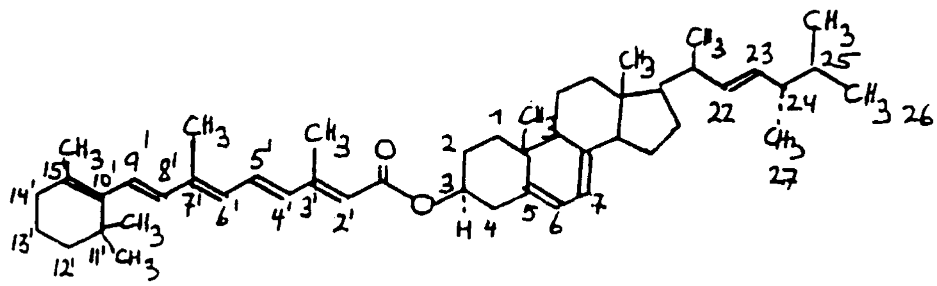

| Chemical shift in ppm vs. TMS*) | Multiplicity **) | Number of protons | Assignments |
|---|---|---|---|
| 7.26 | s | solvent | C<u>H</u>$Cl_3$ |
| 7.0 | d , d | 1 | H-5' |
| 6.27 | m | 2 | H-4' + H-8' |
| 6.15 | d | 1 | H-9' |
| 6.10 | d | 1 | H-6' |
| 5.75 | s | 1 | H-2' |
| 5.57 | m | 1 | H-6 |
| 5.38 | m | 1 | H-7 |
| 5.25 ... 5.13 | m | 2 | H-22 + H-23 |
| 4.75 | m | 1 | H-3α |
| 2.35 | s (m) | 3 | $-CH_3$ (C-3') |
| 2.0 | s (m) | 3 | $-CH_3$ (C-7') |
| 1.70 | s (m) | 3 | $-CH_3$ (C-15') |
| 1.25 + 1.0 | s + s | 6 | $-C<^{CH_3}_{CH_3}$ (C-11') |
| 1.03 + 0.91 | d + d | 6 | sec. $CH_3$ (C-20 + C-24) |
| 0.96 | s | 3 | $-CH_3$-19 |
| 0.83 + o.82 | d + d | 6 | $-CH<^{CH_3\ 26}_{CH_3\ 27}$ |
| 0.63 | s | 3 | $-CH_3$-18 |
| 2.5 ... 0.7 | m | remaining protons | |

*) TMS = Tetramethylsilane
**) s: singlet ; d: doublet ; m: multiplet

Fig. 1

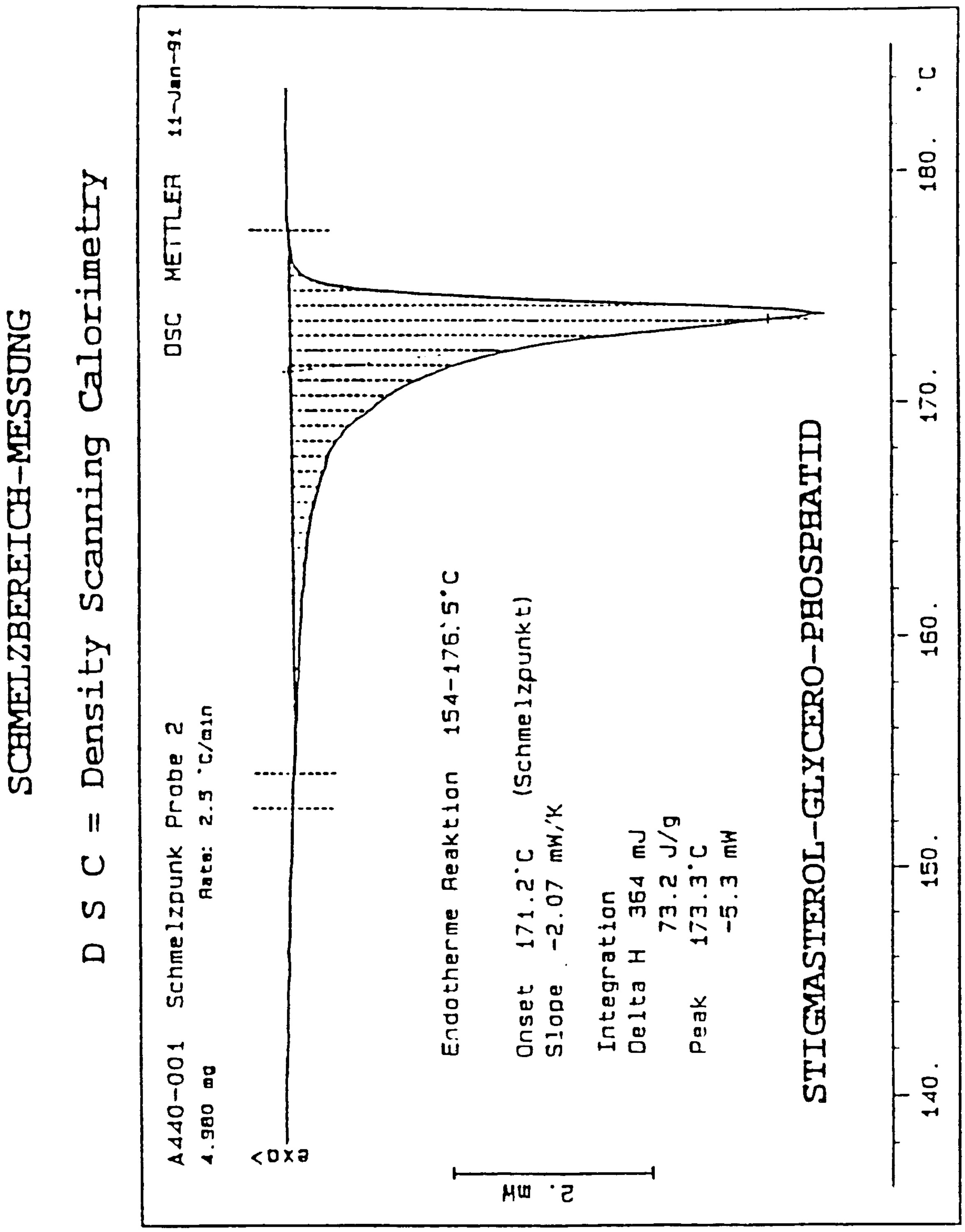

SCHMELZBEREICH-MESSUNG
D S C = Density Scanning Calorimetry
A440-001 Schmelzpunk Probe 2
4.980 mg Rate: 2.5 'C/min
DSC METTLER 11-Jan-91
exo>
2. mW
Endotherme Reaktion 154-176.5'C
Onset 171.2'C (Schmelzpunkt)
Slope -2.07 mW/K
Integration
Delta H 364 mJ
73.2 J/g
Peak 173.3'C
-5.3 mW
STIGMASTEROL-GLYCERO-PHOSPHATID
140.
150.
160.
170.
180.
'C


Fig. 2

Fig. 2

Fortsetzung